# EUROPEAN PATENT SPECIFICATION

(11) **EP 0 697 893 B1**
(45) Date of publication and mention of the grant of the patent: **07.12.2005**
(21) Application number: 94915396.9
(22) Date of filing: 20.04.1994
(51) Int. Cl.: G01N 33/53, A61K 49/00, A61K 31/70, C12Q 1/68, C12N 5/00, C12P 21/02, C07H 15/12

(54) **NEURAL THREAD PROTEIN GENE EXPRESSION AND DETECTION OF ALZHEIMER'S DISEASE**
GENEEXPRESSION DER NEUROFILAMENTPROTEINE UND NACHWEIS DER ALZHEIMESCHEN ERKRANKUNG
EXPRESSION DES GENES DES PROTEINE DES NEUROFILAMENTS ET DETECTION DE LA MALADIE D'ALZHEIMER

(30) Priority: 20.04.1993 US 50559
(43) Date of publication of application: 28.02.1996
(60) Divisional application: 04076254.4
(73) Proprietor: THE GENERAL HOSPITAL CORPORATION, Boston, MA 02114 (US)
(72) Inventor: DE LA MONTE, Suzanne M., Cambridge, MA 02140 (US); WANDS, Jack R., Waban, MA 02168 (US)
(74) Representative: Chapman, Paul William
(86) International application number: PCT/US1994/004321
(87) International publication number: WO 1994/023756

(56) References cited:
- WO-A-90/06993
- J NEUROSCI, JUN 1991, 11 (6) P1515-23, UNITED STATES, XP002015766 CACERES A ET AL: "The effect of tau antisense oligonucleotides on neurite formation of cultured cerebellar macroneurons."
- Nature, Volume 341, issued 21 September 1989, C.L. JOACHIM et al., "Amyloid Beta-Protein Deposits in Tissues Other Than Brain in Alzheimer's Disease", pages 226-230, especially pages 226-227, figure 1.
- Science, Volume 248, issued 18 May 1990, S.A JOHNSON et al., "Relation of Neuronal APP751/APP-695 mRNA Ratio and Neuritic Plaque Density in Alzheimer's Disease", pages 854-857, especially page 856, figure 2.
- Lancet, Volume 339, issued 21 March 1992, A.A. GUTIERREZ et al., "Gene Therapy for Cancer", pages 715-721, especially page 717, col. 1, parag. 2 to col. 2, through parag. 2 and page 719, parag. 2 to page 720, through parag. 2.

## Description

The present invention was made with U.S. government support. Therefore, the U.S. government has certain rights in the invention.

### Cross Reference to Related Applications

This is a continuation-in-part of U.S. Application Serial No. 08/050,559, filed April 20, 1993, the contents of which are fully incorporated by reference herein.

### Field of the Invention

The present invention is in the field of genetic engineering and molecular biology. This invention is directed to recombinant hosts expressing novel proteins associated with Alzheimer's Disease, neuroectodermal tumors, malignant astrocytomas, and glioblastomas. This invention is specifically directed to the recombinant hosts and vectors which contain the genes coding for the neuronal thread proteins. This invention is also directed to substantially pure neural thread proteins, immunodiagnostic and molecular diagnostic methods to detect the presence of neural thread proteins, and the use of nucleic acid sequences which code for neural thread proteins in gene therapy.

### Background of the Invention

### Alzheimer's Disease

Alzheimer's Disease (AD) is the most frequent cause of dementia in the United States, affecting over two million individuals each year. It is a degenerative brain disorder characterized clinically by loss of memory, confusion, and gradual physical deterioration. It is the fourth most common cause of death. The etiology of the disease is virtually unknown but has been attributed to various viruses, toxins, heavy metals, as well as genetic defects. The disease is at present incurable.

Until quite recently, AD was thought to account for relatively few of the cases generally classified as senile dementia. Other factors can lead to such a condition, including repetitious mild strokes, thyroid disorders, alcoholism, and deficiencies of certain vitamins, many of which are potentially treatable. It can be appreciated, then, that a diagnostic test specific for AD would be very useful for the clinical diagnosis and proper clinical treatment of subjects presenting with symptoms common to all of these conditions.

The brains of individuals with AD exhibit characteristic pathological accumulations of congophilic fibrous material which occurs as neurofibrillary tangles within neuronal cell bodies, and neuritic (or senile) plaques. Neurofibrillary tangles may also be found in the walls of certain cerebral blood vessels. The major organized structural components of neurofibrillary tangles are paired helical filaments. Qualitatively indistinguishable amyloid deposits also occur in normal aged brains but in much smaller numbers with restricted topographical distribution.

There has been considerable recent investigative activity regarding the characterization of proteins found in neuritic plaques and neurofibrillary tangles of AD and other neurologic diseases. One of the amyloid proteins initially described by Glenner *et al*. has been cloned and sequenced (Glenner *et al.. Biochem. Biophys. Res. Commun. 120*:1131-1135 (1984): U.S. Patent No. 4,666.829). The A4 amyloid protein found in neuritic plaques and blood vessels has been determined to be a component of a 695 amino acid precursor; a protein postulated to function as a glycosylated cell surface receptor (Masters *et al., Proc. Natl. Acad. Sci. USA 82*:4245-4249 (1985), Kang *et al., Nature* 325:733-736 (1987)). In addition, the amyloid protein has been postulated to function as a cell adhesion molecule and as a calcium ion channel protein (Hooper, *J*. *NIN Res. 4*: 48-54 (1992); Rensberger, *Wayward Protein Molecule May Be Elusive Killer of Brain Cells, The Washington Post,* January 25, 1993, §1, at A3 (1993)). The gene coding for A4 is located on chromosome 21 (Kang *et al*., *ibid.;* Goldgaber *et al., Science 235*:877-880 (1987); Tanzi *et al., Science 235*:880-885 (1987); St. George-Hyslop *et al., Science 235*:885-889 (1987)) but apparently is not linked to the familial form of the disease (Van Broekhoven *et al., Nature 329*:153-155 (1987)). There appears to be little, if any, protein sequence homology between amyloid A4 and β protein, their higher molecular weight precursor, and pancreatic thread protein (PTP) (Gross *et al., J. Clin. Invest. 76*:2115-2126 (1985)).

A number of other proteins thought to be associated with the disease have been described, including Ubiquitin, ALZ-50. microtubular-associated proteins τ and MAP2, and neurofilament protein *(see,* for example, Manetto *et al*., *Proc. Natl. Acad. Sci. USA 85*:4502-4505 (1988): Wolozin *et al., Science 232*:648-651 (1986); Selkoe, *Neurobiol. Aging 7*:425-432 (1986); Perry *et al., in: Alterations of the Neuronal Cytoskeleton in Alzheimer's Disease,* Plenum, New York, pp 137-149 (1987)). More recently, a serine protease inhibitor called α₁-anti-chymotrypsin has been found in AD amyloid deposits (Abraham *et al., Cell 52*:487-501 (1988)).

There is currently no useful diagnostic test for AD being practiced clinically. A definitive diagnosis is possible only postmortem, or during life through a brain biopsy, to reveal the presence of the characteristic plaques, tangles, paired helical filaments, and other cerebrovascular deposits which characterize the disorder. Such an invasive surgical procedure is inherently dangerous and is therefore rarely utilized. As a result, the clinical misdiagnosis of AD is estimated to be approximately 20%-30%.

### Thread Proteins

The prototype thread protein molecule is pancreatic thread protein (PTP) which bears the unusual physical property of forming insoluble fibrils at neutral pH, but is highly soluble at acid or alkaline pH (Gross *et al., supra*). PTP is highly abundant, synthesized by pancreatic acinar cells, and secreted into pancreatic juice in concentrations exceeding 1 mg/ml (*Id*.). An increased thread protein immunoreactivity has been demonstrated in brains with AD lesions, using monoclonal antibodies to PTP (Ozturk *et al*., *Proc. Nail. Acad. Sci. USA 86*:419-423 (1989)). In addition, a highly sensitive forward sandwich immunoradioinetric assay was used to demonstrate that at least three distinct antigenic epitopes were shared between PTP and the related protein in the brain (*Id*.) Despite similarities, the pancreatic and neuronal forms of the thread protein are almost certainly distinct since the mRNA molecules and proteins differ in size, and many of the antigenic epitopes which are present in the pancreatic thread protein are not detectable in brain tissue (de la Monte *et. al., J. Clin. Invest. 86*:1004-1013 (1990); de la Monte *et. al., J. Neurol*. *Sci. 113*:152-164 (1992); de la Monte *et al., Ann*. *Neurol. 32*:733-742 (1992)).

The central nervous system form of the thread protein, designated hereafter as "neural thread protein" (NTP), has been identified in AD and Down's Syndrome brain tissue (Wands *et al*., International Application Publication No. WO 90/06993). NTP has been found in all AD brains studied where characteristic neuropathologic changes of the disease exist (*Id*.). The saline- extractable soluble immunoreactivity shares has a molecular weight of approximately 17 to 20 kD (*Id*.).

Quantitative measurements of NTP immunoreactivity in various regions of AD brains revealed levels varying from 12 to 295 ng/gm tissue (Mean = 116 ng/gm tissue) compared to 1-11 ng/gm tissue (Mean = 5 ng/gm tissue) in comparable ares of control brains (*Id*.).

linmunocytoclieinistry performed with monoclonal antibodies directed against the pancreatic form of PTP demonstrated that NTP is localized within cells, within fine processes within the neuropil, or is extracellular in both AD and Down's Syndrome brains (*Id*.). Two types of cell contain NTP: neurons and astrocytes (*Id*.). The affected neurons are the large pyramidal type which typically contain the neurofibrillary tangles well known in AD brain (*Id*.).

That NTP accumulation within neurons is intrinsically important or integrally related to the evolution of AD lesions is corroborated by the presence of identical patterns of immunolabeling for NTP in Down's Syndrome brains, but not in control brains (*Id*.). It is important to note that the same structural abnormalities of AD occur in brains of all middle-age individuals with Down's syndrome, whether or not they are demented. There is also a higher incidence of AD in family members of Down's Syndrome patients. Moreover, the regional differences in the densities of NTP-containing neurons parallels the density distributions of neurofibrillary tangles in both AD and Down's Syndrome. This provides further evidence that NTP is germane to the pathophysiology of AD. Whether NTP accumulates within neuronal perikarya, as a result of aberrant cellular metabolism or transport is not yet known.

### Summary of the Invention

A need exists for a definitive diagnostic test which can be performed on individuals suspected of having, or being at risk for AD. The present invention satisfies such needs and provides further advantages.

The manner in which these and other objects are realized by the present invention will be apparent from the summary and detailed description set forth below.

Unless defined otherwise, various terms used herein have the same meaning as is well understood in the an to which the invention belongs. All cited publications are incorporated herein by reference.

This invention is directed to recombinant hosts expressing a novel protein associated with Alzheimer's Disease, neuroectodermal tumors, malignant astrocytomas, and glioblastomas. This invention is specifically directed to the recombinant hosts and vectors which contain the gene coding for the neuronal thread protein (NTP) having a molecular weight of about 42 kDa. In the first aspect, the present invention relates to a neural thread protein (NTP) substantially free of any natural impurities and coded for by the AD10-7 DNA molecule present in the *E*. *coli* cells that are on deposit at the American Type Culture Collection, Manassass, Va., under accession no. 69262. This invention is also directed to the substantially pure neural threat proteins, immunodiagnostic and molecular diagnostic methods to detect the presence of neural thread proteins, and the use of nucleic acid sequences which code for neural thread proteins in gene therapy.

In particular, the invention includes an *in vitro* method for detecting the presence of an NTP encoded by AD10-7 DNA molecule, comprising:
(a) contacting a biological sample from a human subject that is suspected of containing NTP encoded by AD10-7 DNA molecule with at least one molecule capable of binding to said NTP; and
(b) detecting the molecule bound to said NTP.

The invention additionally includes the method as above, wherein the binding molecule is selected from the group consisting of:
(a) an antibody substantially free of natural impurities;
(b) a monoclonal antibody; and
(c) a fragment of (a) or (b).

The invention additionally includes the method as above, wherein the detecting molecule is detectably labeled and where a combination of such binding molecules is used.

The invention additionally includes an *in vitro* method for detecting the presence of a genetic sequence coding for an NTP encoded by AD10-7 DNA molecule in a biological sample using a polynucleotide probe derived from a recombinant human NTP of this invention.

The invention additionally includes an *in vitro* method for determining the presence of a condition in a human subject, said condition including, but not limited to, the group consisting of Alzheimer's Disease, the presence of neuroedtodermal tumors, the presence of malignant astrocytomas, and the presence of gliomas.

The invention additionally includes a method of diagnosing the presence of AD in a human subject suspected of having AD which comprises:
(a) incubating a biological sample from said subject suspected of containing an NTP encoded by AD10-7 DNA molecule with a molecule capable of identifying an NTP encoded by AD10-7 DNA molecule; and
(b) detecting the molecule which is bound in the sample, wherein the detection indicates that the subject has AD.

The invention additionally includes a method of diagnosing the presence of neuroectodermal tumors in a human subject suspected of having neuroectodermal tumors which comprises:
(a) incubating a biological sample from said subject suspected of containing an NTP encoded by AD10-7 DNA molecule with a molecule capable of identifying an NTP encoded by AD10-7 DNA molecule; and
(b) detecting the molecule which is bound in the sample, wherein the detection indicates that the subject has neuroectodermal tumors.

The invention additionally includes a method of diagnosing the presence of a malignant astrocytoma in a human subject suspected of having a malignant astrocytoma which comprises:
(a) incubating a biological sample from said subject, which is suspected of containing an NTP encoded by AD10-7 DNA molecule, in the presence of a binding molecule capable of identifying an NTP encoded by AD10-7 DNA molecule; and
(b) detecting molecule which is bound in the sample, wherein the detection indicates that the subject has a malignant astrocytoma.

The invention additionally includes a method of diagnosing the presence of a glioblastoma in a human subject suspected of having a gliobtastoma which comprises:
(a) incubating a biological sample from said subject, which is suspected of containing an NTP encoded by AD10-7 DNA molecule, in the presence of a binding molecule capable of identifying an NTP encoded by AD10-7 DNA molecule; and
(b) detecting molecule which is bound in the sample, wherein the detection indicates that the subject has a glioblastoma.

The invention additionally includes the methods as above, wherein a biological sample is removed a human subject prior to contacting the sample with the molecule.

The invention additionally includes the methods as above, wherein detecting any of the molecules bound to the protein is performed by *in situ* imaging.

The invention additionally includes the methods as above, wherein detecting of any of the molecule bound to the protein is performed by *in vivo* imaging.

The invention additionally includes the methods as above, wherein the biological sample is reacted with the binding molecule in a manner and under such conditions sufficient to determine the presence and the distribution of the protein.

The present invention also particularly relates to the diagnostic methods recited above, wherein the immunoassay comprises two different antibodies bound to a solid phase support combined with a third different detectably labeled antibody in solution.

The invention is also directed to a method of producing an NTP encoded by AD10-7 DNA molecule, said method comprising:
(a) culturing a recombinant host comprising a human gene coding for said NTP; and
(b) isolating said NTP from said host.

Additionally, the invention is directed to a substantially pure NTP encoded by AD10-7 DNA molecule obtained by the such a process.

The invention is also directed to an 15- to 30-mer antisense oligonucleotide which is complementary to a nucleic acid sequence of an NTP encoded by AD10-7 DNA molecule and which is nonhomologous to PTP nucleic acid sequences, as well as pharmaceutical compositions comprising such oligonucleotides and a pharmaceutically acceptable carrier.

The invention is also directed to ribozymes comprising a target sequence which is complementary to a sequence of an NTP encoded by AD10-7 DNA molecule and nonhomologous to PTP nucleic acid sequences, as well as pharmaceutical compositions comprising such ribozymes and a pharmaceutically acceptable carrier.

The invention is also directed to a method of achieving pharmaceutical delivery of NTP encoded by AD10-7 DNA molecules to the brain through acceptable carriers or expression vectors.

The invention is also directed to oligodeoxynucleotides that form triple stranded regions with the gene of the NTP encoded by AD10-7 DNA molecule (nucleic acid sequences) and which are nonhomologous to PTP nucleic acid sequences, as well as pharmaceutical compositions comprising such oligodeoxynucleotides and a pharmaceutically acceptable carrier.

The invention is also directed to methods for the differential diagnosis of sporadic and familial Alzheimer's disease.

### Brief Description of the Drawings

Figures 1A-1J show neural thread protein immunoreactivity in CNS-derived tumors.
Figure 2 depicts a graph showing neural thread protein levels in PNET1, PNET2, A172, C6, and Huh7 hepatocellular carcinoma cells measured by a forward sandwich monoclonal antibody-based immunoradiometric assay (M-IRMA).
Figure 3 shows molecular size of neural thread proteins in SH-Sy5y, A172, and C6 cells demonstrated by immunoprecipitation and Western blot analysis using the Th9 monoclonal antibody.
Figure 4 shows molecular sizes of neural thread proteins in PNET1 cells (a) and C6 glioblastoma cells (b) demonstrated by pulse-chase metabolic labeling with ³⁵S-methionine, and immunoprecipitation with Th9 monoclonal antibody (Figure 4A). The molecular weights are 8, 14, 17, 21, 26 and 42 kDa (arrows).
Figures 5A-5E depict a series of five graphs showing the 21 kDa and 17 kDa neural thread proteins in SH-Sy5y, PNET1, A172, and C6 cells and the absence thereof in Huh7 cells by SDS-PAGE/M-IRMA.
Figure 6 depicts a gel showing that the 21 kDa neural thread protein in C6 glioblastoma cells is phosphorylated.
Figure 7 depicts a bar graph showing altered neural thread protein expression in PNET1 cells with growth phase.
Figure 8 (panels A-F) shows altered phenotype of PNET1 cells with cessation of cell growth and overnight serum starvation.
Figure 9 shows the 1-9a partial cDNA sequence, and Figure 9a shows a partial sequence of the second 5' anchor PCR product corresponding to the 5' region of the 1-9a cDNA (WP5' Sequence).
Figure 10 shows alignment of partial sequences between 1-9a and human PTP and the Reg gene (the nucleic acid sequence corresponding to the genomic clone of human PTP).
Figure 10a shows alignment between 1-9a and Exon 2 of the human Reg gene, and between the first 5' anchor PCR product of 1-9a (WP03-417) and Exon 2 and Reg.
Figure 10b shows alignment between the 1-9a and its second 5' anchor PCR product (WP5') and AD 3-4 and AD 2-2 cDNAs.
Figure 11 shows the partial nucleic acid and deduced amino acid sequences of the HB4 cDNA as well as a protein hydrophilicity window plot.
Figure 11a shows alignment between HB4 and human PTP.
Figure 11b shows alignment between HB4 and human Reg gene.
Figure 12 (panels A-C) shows the expression of mRNA molecules corresponding to the 1-9a CNS neural thread protein cDNA sequence in neuroectodermal tumor cell lines and in rat pancreas.
Figure 13 (panels A and B) shows mRNA transcripts corresponding to the 1-9a CNS neural thread protein cDNA sequence in human brain. This figure also demonstrates higher levels of 1-9a CNS neural thread protein-related mRNAs in AD brains compared with aged-matched controls (panel A). Panel B demonstrates four different transcripts with greater abundance of the lower molecular size mRNAs in AD compared with aged controls.
Figure 14 (panels A-C) shows 1-9a Southern blot analysis of RT/PCR-derived cDNAs in neuroectodermal cell lines. A- and B-PCR amplification of 1-9a mRNA sequences in neuroectodermal cell lines, and using mRNA from newborn rat (NB) brain, AD brain, and aged control brain. Panel A is a longer exposure of Panel B. Panel C shows hybridization of the same blot using the O18 rat PTP probe.

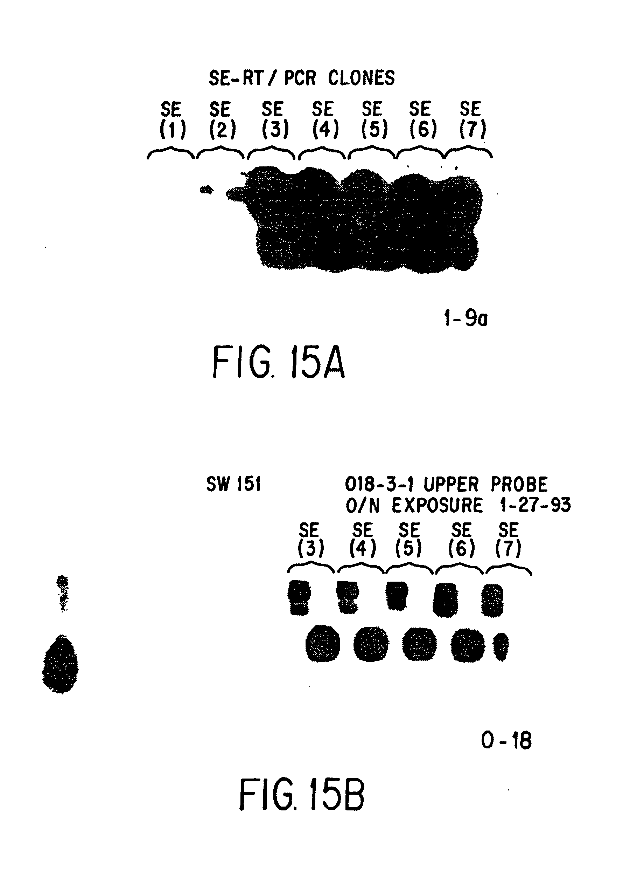

Figure 15 (panels A and B) (SE-RT/PCR) shows hybridization of the 1-9a and O18 probes with several clones isolated from SH-Sy5y cells by reverse transcribing mRNA and amplifying with primers corresponding to the known sequence of the 1-9a partial cDNA.
Figures 16, 16a and 16b show the partial nucleic acid sequences of the AD 2-2 cDNAs isolated from the AD brain library. Figure 16 also shows a hydrophilicity window plot of AD2-2 T7.
Figures 16c, 16d, 16e and 16f show the partial nucleic acid sequences of the AD 3-4 cDNAs isolated from the AD brain library. Figure 16c also shows a hydrophilicity window plot of AD3-4.
Figures 16g, 16h and 16i show the partial nucleic acid sequences of the AD 4-4 cDNAs isolated from the AD brain library.
Figure 16j shows the partial nucleic acid sequences of the AD 16c (also called AD 10-7) cDNAs isolated from the AD brain library. Figure 16j also shows a hydrophilicity window plot of AD16c-T7.
Figure 16k shows the complete nucleotide sequence of the AD10-7 cDNA clone that was isolated from an AD library.
Figure 16l shows the complete nucleotide sequence of the AD 16c cDNA clone that was isolated from the AD brain library.
Figure 17 shows alignment of partial sequences between AD 2-2 and human Reg gene.
Figure 17a shows alignment of partial sequences between AD 2-2 and Exon 1 of Reg and rat PTP.
Figure 17b shows alignment of partial sequences between AD 2-2 and 1-9a.
Figure 17c shows alignment of partial sequences between AD 2-2 and AD 16c.
Figure 18 shows alignment of partial sequences between AD 3-4 (also called AD 5-3) and the Reg gene.
Figure 18a shows alignment of partial sequences between AD 3-4 and the 5' anchor PCR products of the 1-9a mRNA, termed WPO3-5 and 18-4.
Figure 18b shows alignment of partial sequences between AD 3-4 and the G2a-a *Eco*RI/*Pst*I genomic clone.
Figure 19 shows alignment of partial sequences between AD 4-4 and AD 2-2 and 1-9a (also called SE-4 corresponding to the PCR clone which is identical to 1-9a).
Figure 20 shows alignment of partial sequences between AD 16c and Reg gene.
Figure 20a shows alignment of partial sequences between AD 16c and human PTP.
Figure 20b shows alignment of partial sequences between AD 16c and AD 2-2.

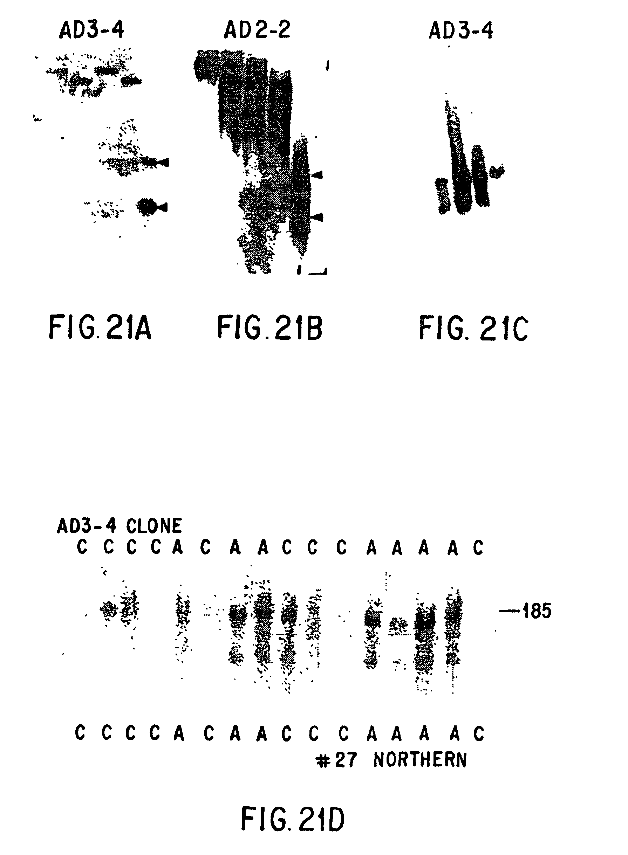

Figure 21 (panel A) shows a genomic Southern blot analysis using the AD 3-4 as a probe; Figure 21 (panel B) shows a similar pattern of hybridization on a genomic Southern using AD 2-2 as a probe. Figure 21 (panel C) shows a Northern blot analysis of neuroectodermal tumor cell lines using AD 3-4 as a probe. The four cell lines that exhibit AD 3-4 transcripts are neuronal in phenotype; C6 glioma cell mRNA did not hybridize with the AD 3-4 probe. Figure 21 (panel D) shows a Northern analysis of human AD and aged control brain temporal lobe tissue using the AD 3-4 probe, and demonstrates over-expression of the corresponding gene in AD (lanes labeled A) compared with aged control brains (lanes labeled C).
Figures 22, 22a, 22b, 22c, 22d, 22e, 22f, 22g and 22h shows partial sequences of four genomic clones (isolated using both the 1-9a cDNA and rat PTP 0-18 cDNA as probes.
Figures 23 and 23a show the alignment of the G2a-2 *Pst*I partial sequence with the Reg gene.
Figure 23b shows alignment of the G2a-2 *Pst*I*-Eco*RI sequence and the Reg gene and the rat PTP.
Figures 23c and 23d show the alignment of the G5d-1 *Pst*I sequence and the Reg gene.
Figure 24 shows neural thread protein expression by the 1-9a cDNA (panel A) and the G2a-2 *Pst*I genomic clone (panel B). Panels C and D show negative expression by the G5d-1 *Eco*RI/*Pst*I genomic clone, and pBluescript which lacks a cloned insert, respectively.
Figure 25 depicts a Northern blot analysis of AD16c mRNA in AD and aged control brains. The data shows elevated.levels of AD16c mRNA expression in 6 of 9 AD compared to 1 of 6 age-matched controls.
Figure 26 depicts a Western blot analysis of AD 10-7 fusion proteins using monoclonal antibodies against the expressed tag protein (T7-tag mouse monoclonal antibodies.
Figure 27 (panels A and B) depicts brightfield and darkfield microscopic analysis of the *in situ* hybridization of sense and antisense cRNA probes to human brain tissue sections of early AD.

### Definitions

In the description that follows, a number of terms used in recombinant DNA technology are utilized extensively. In order to provide a clear and consistent understanding of the specification and claims, including the scope to be given such terms, the following definitions are provided.

***Cloning vector**.* A plasmid or phage DNA or other DNA sequence which is able to replicate autonomously in a host cell, and which is characterized by one or a small number of restriction endonuclease recognition sites at which such DNA sequences may be cut in a determinable fashion without loss of an essential biological function of the vector, and into which a DNA fragment may be spliced in order to bring about its replication and cloning. The cloning vector may further contain a marker suitable for use in the identification of cells transformed with the cloning vector. Markers, for example, provide tetracycline resistance or ampicillin resistance.

***Expression vector**.* A vector similar to a cloning vector but which is capable of enhancing the expression of a gene which has been cloned into it, after transformation into a host. The cloned gene is usually placed under the control of (i.e., operably linked to) certain control sequences such as promoter sequences. Promoter sequences may be either constitutive or inducible.

***Substantially pure.*** As used herein means that the desired purified protein is essentially free from contaminating cellular components, said components being associated with the desired protein in nature, as evidenced by a single band following polyacrylamide-sodium dodecyl sulfate gel electrophoresis. Contaminating cellular components may include, but are not limited to, proteinaceous, carbohydrate, or lipid impurities.

The term "substantially pure" is further meant to describe a molecule which is homogeneous by one or more purity or homogeneity characteristics used by those of skill in the art. For example, a substantially pure NTP will show constant and reproducible characteristics within standard experimental deviations for parameters such as the following: molecular weight, chromatographic migration, amino acid composition, amino acid sequence, blocked or unblocked N-terminus, HPLC elution profile, biological activity, and other such parameters. The term, however, is not meant to exclude artificial or synthetic mixtures of the factor with other compounds. In addition, the term is not meant to exclude NTP fusion proteins isolated from a recombinant host.

***Recombinant Host.*** According to the invention, a recombinant host may be any prokaryotic or eukaryotic cell which contains the desired cloned genes on an expression vector or cloning vector. This term is also meant to include those prokaryotic or eukaryotic cells that have been genetically engineered to contain the desired gene(s) in the chromosome or genome of that organism.

***Recombinant vector.*** Any cloning vector or expression vector which contains the desired cloned gene(s).

***Host.*** Any prokaryotic or eukaryotic cell that is the recipient of a replicable expression vector or cloning vector. A "host," as the term is used herein, also includes prokaryotic or eukaryotic cells that can be genetically engineered by well known techniques to contain desired gene(s) on its chromosome or genome. For examples of such hosts, *see* Sambrook *et al., Molecular Cloning: A Laboratory Manual*, Second Edition, Cold Spring Harbor Laboratory, Cold Spring Harbor, New York (1989).

***Promoter**.* A DNA sequence generally described as the 5' region of a gene, located proximal to the start codon. The transcription of an adjacent gene(s) is initiated at the promoter region. If a promoter is an inducible promoter, then the rate of transcription increases in response to an inducing agent. In contrast, the rate of transcription is not regulated by an inducing agent if the promoter is a constitutive promoter.

***Gene.*** A DNA sequence that contains information needed for expressing a polypeptide or protein.

***Structural gene.*** A DNA sequence that is transcribed into messenger RNA (mRNA) that is then translated into a sequence of amino acids characteristic of a specific polypeptide.

***Antisense RNA gene*****/*****Antisense RNA.*** In eukaryotes, mRNA is transcribed by RNA polymerase II. However, it is also known that one may construct a gene containing a RNA polymerase II template wherein a RNA sequence is transcribed which has a sequence complementary to that of a specific mRNA but is not normally translated. Such a gene construct is herein termed an "antisense RNA gene" and such a RNA transcript is termed an "antisense RNA." Antisense RNAs are not normally translatable due to the presence of translation stop codons in the antisense RNA sequence.

***Antisense oligonucleotide*****.** A DNA or RNA molecule containing a nucleotide sequence which is complementary to that of a specific mRNA. An antisense oligonucleotide binds to the complementary sequence in a specific mRNA and inhibits translation of the mRNA.

***Antisense Therapy.*** A method of treatment wherein antisense oligonucleotides are administered to a patient in order to inhibit the expression of the corresponding protein.

***Complementary DNA (cDNA)*****.** A "complementary DNA," or "cDNA" gene includes recombinant genes synthesized by reverse transcription of mRNA and from which intervening sequences (introns) have been removed.

***Expression*****.** Expression is the process by which a polypeptide is produced from a structural gene. The process involves transcription of the gene into mRNA and the translation of such mRNA into polypeptide(s).

***Homologous*****/*****Nonhomologous*** Two nucleic acid molecules are considered to be "homologous" if their nucleotide sequences share a similarity of greater than 50%, as determined by HASH-coding algorithms (Wilber, W.J. and Lipman, D.J., *Proc. Natl. Acad. Sci. 80*:726-730 (1983)). Two nucleic acid molecules are considered to be "nonhomologous" if their nucleotide sequences share a similarity of less than 50%.

***Ribozyme.*** A ribozyme is an RNA molecule that contains a catalytic center. The term includes RNA enzymes, self-splicing RNAs, and self-cleaving RNAs.

***Ribozyme Therapy.*** A method of treatment wherein ribozyme is administered to a patient in order to inhibit the translation of the target mRNA.

***Fragment.*** A "fragment" of a molecule such as NTP is meant to refer to any polypeptide subset of that molecule.

***Functional Derivative.*** The term "functional derivatives" is intended to include the "variants," "analogues," or "chemical derivatives" of the molecule. A "variant" of a molecule such as NTP is meant to refer to a naturally occurring molecule substantially similar to either the entire molecule, or a fragment thereof. An "analogue" of a molecule such as NTP is meant to refer to a non-natural molecule substantially similar to either the entire molecule or a fragment thereof.

A molecule is said to be "substantially similar" to another molecule if the sequence of amino acids in both molecules is substantially the same, and if both molecules possess a similar biological activity. Thus, provided that two molecules possess a similar activity, they are considered variants as that term is used herein even if one of the molecules contains additional amino acid residues not found in the other, or if the sequence of amino acid residues is not identical.

As used herein, a molecule is said to be a "chemical derivative" of another molecule when it contains additional chemical moieties not normally a part of the molecule. Such moieties may improve the molecule's solubility, absorption, biological half-life, etc. The moieties may alternatively decrease the toxicity of the molecule, eliminate or attenuate any undesirable side effect of the molecule, etc. Examples of moieties capable of mediating such effects are disclosed in *Remington's Pharmaceutical Sciences* (1980) and will be apparent to those of ordinary skill in the art.

NTP. The term "NTP" refers to a neural thread protein with a molecular weight of about 42 kDa, as described herein.

***Immuno-Polymerase Chain Reaction*****.** A method for the detection of antigens using specific antibody-DNA conjugates. According to this method, a linker molecule with bispecific binding affinity for DNA and antibodies is used to attach a DNA molecule specifically to an antigen-antibody complex. As a result, a specific antigen-antibody-DNA conjugate is formed. The attached DNA can be amplified by the polymerase chain reaction (PCR) using appropriate oligonucleotide primers. The presence of specific PCR products demonstrates that DNA molecules are attached specifically to antigen-antibody complexes, thus indicating the presence of antigen. (Sano *et al., Science 258*:120-122 (1992)).

For example, Sano *et al*., *supra*, constructed a streptavidin-protein A chimera that possesses specific binding affinity for biotin and immunoglobulin G. This chimera (i.e., the "linker molecule") was used to attach a biotinylated DNA specifically to antigen-monoclonal antibody complexes that had been immobilized on microtiter plate wells. A segment of the attached DNA was subsequently amplified by PCR.

### Detailed Description of the Invention

This invention is directed to a neural thread protein (NTP) encoded by AD10-7 DNA molecule, genetic sequences coding for the NTP mRNA or antisense mRNA, expression vectors containing the genetic sequences, recombinant hosts transformed therewith, and RNA for the NTP encoded by AD10-7 DNA molecule and antisense RNA produced by such transformed recombinant host expression. This invention further relates to NTP ribozymes, and recombinant DNA molecules which code for ribozymes for the NTP encoded by AD10-7 DNA molecule and antisense oligonucleotides for the NTP encoded by AD10-7 DNA molecule. This invention further relates to antibodies directed against an NTP, as well as the use of NTP encoded by AD10-7 DNA molecule antibodies and nucleic acid sequences of the NTP encoded by AD10-7 DNA molecule for detection of the presence of an NTP encoded by AD10-7 DNA molecule in biological samples. The invention further relates to the use of coding sequences for the NTP encoded by AD10-7 DNA molecule in gene therapy.

### I. Isolation of DNA Sequences Coding for Neuronal Thread Proteins

DNA sequences coding for an NTP are derived from a variety of sources. These sources include genomic DNA, cDNA, synthetic DNA, and combinations thereof.

Human NTP genomic DNA can be extracted and purified from any human cell or tissue, by means well known in the art (for example, *see* Sambrook *et al*., *Molecular Cloning: A Laboratory Manual,* Second Edition, Cold Spring Harbor Laboratory Press, 1989). The NTP genomic DNA of the invention may or may not include naturally occurring introns. Moreover, such genomic DNA may be obtained in association with the 5' promoter region of the NTP gene sequences and/or with the 3' translational termination region. Further, such genomic DNA may be obtained in association with DNA sequences which encode the 5' nontranslated region of the NTP mRNA and/or with the genetic sequences which encode the 3' nontranslated region. To the extent that a host cell can recognize the transcriptional and/or translational regulatory signals associated with the expression of the mRNA and protein, then the 5' and/or 3' nontranscribed regions of the native gene, and/or, the 5' and/or 3' nontranslated regions of the mRNA, may be retained and employed for transcriptional and translational regulation.

Alternatively, an NTP mRNA can be isolated from any cell which expresses an NTP, and used to produce cDNA by means well known in the art (for example, *see* Sambrook *et al*., *supra*). Preferably, the mRNA preparation used will be enriched in mRNA coding for an NTP, either naturally, by isolation from cells which produce large amounts of an NTP, or *in vitro*, by techniques commonly used to enrich mRNA preparations for specific sequences, such as sucrose gradient centrifugation, or both. An NTP mRNA may be obtained from mammalian neuronal tissue, or from cell lines derived therefrom. Preferably, human cDNA libraries are constructed from 17-18 week old fetal brain, 2 year old temporal lobe neocortex, end-stage AD cerebral cortex, or from cell lines derived from human neuronal tissue. Such cell lines may include, but are not limited to, central nervous system primitive neuroectodermal tumor cells (such as PNET1 or PNET2, as described herein), neuroblastoma cells (such as SH-Sy5y, as described herein), or human glioma cells (such as A172; ATCC CRL 1620). Alternatively, a rat cDNA library can be prepared from mRNA isolated from rat glioma cells, for example, C6 rat glioma cells (ATCC CCL107).

For cloning into a vector, suitable DNA preparations (either genomic or cDNA) are randomly sheared or enzymatically cleaved, respectively, and ligated into appropriate vectors to form a recombinant gene (either genomic or cDNA) library. A DNA sequence encoding an NTP may be inserted into a vector in accordance with conventional techniques, including blunt-ending or staggered-ending termini for ligation, restriction enzyme digestion to provide appropriate termini, filling in of cohesive ends as appropriate, alkaline phosphatase treatment to avoid undesirable joining, and ligation with appropriate ligases. Techniques for such manipulation are disclosed by Sambrook *et al., supra,* and are well known in the art.

Libraries containing NTP clones may be screened and the NTP clones identified by any means which specifically selects for NTP DNA such as, for example: 1) by hybridization with an appropriate nucleic acid probe(s) containing a sequence specific for the DNA of this protein; or, 2) by hybridization-selected translational analysis in which native mRNA hybridizes to the clone in question, is translated *in vitro*, and the translation products are further characterized; or, 3) if the cloned DNA sequences are themselves capable of expressing mRNA, by immunoprecipitation of a translated NTP product produced by the host containing the clone.

Oligonucleotide probes specific for an NTP which can be used to identify clones to this protein can be designed from knowledge of the amino acid sequence of the corresponding NTP, or homologous regions of the PTP. Alternatively, oligonucleotide probes can be designed from knowledge of the nucleotide sequence of PTP (de la Monte *et al*., *J. Clin. Invest. 86*:1004-1013 (1990)).

The suitable oligonucleotide, or set of oligonucleotides, which is capable of encoding a fragment of the NTP gene (or which is complementary to such an oligonucleotide, or set of oligonucleotides) may be synthesized by means well known in the art (for example, *see* Sambrook *et al., supra*). Techniques of nucleic acid hybridization and clone identification are disclosed by Sambrook *et al., supra.* Those members of the above-described gene library which are found to be capable of such hybridization are then analyzed to determine the extent and nature of the NTP encoding sequences which they contain.

To facilitate the detection of the desired NTP coding sequence, the above-described DNA probe is labeled with a detectable group. Such detectable group can be any material having a detectable physical or chemical property. Such materials have been well-developed in the field of nucleic acid hybridization and in general most any label useful in such methods can be applied to the present invention. Particularly useful are radioactive labels including ³²P, ³H, ¹⁴C, ¹²⁵I, or the like. Any radioactive label may be employed which provides for an adequate signal and has sufficient half-life. The DNA probe may be labeled, for example, by nick-translation, by T4 DNA polymerase replacement synthesis, or by random priming, among other methods well known in the art *(see* Sambrook *et al. supra*).

Alternatively. DNA probes can be labeled with non-radioactive markers such as biotin, an enzyme, or fluorescent group.

In an alternative method of cloning NTP DNA sequences, NTP cDNAs are obtained by direct cloning of cDNAs from cell lines and brain tissue, using the 3'- and 5'-RACE methods, as described herein. Preferably, a human neuroectodermal tumor cell line or AD brain tissue is used as a source of mRNA.

### II. Expressing the Gene Coding for NTP

The above-discussed methods are, therefore, capable of identifying DNA sequences which are code for an NTP encoded by AD10-7 DNA molecule or fragments thereof. In order to further characterize such DNA sequences, and in order to produce the recombinant protein, it is desirable to express the proteins which the DNA sequences encode.

To express an NTP, transcriptional and translational signals recognizable by an appropriate host are necessary. The cloned NTP DNA sequences, obtained through the methods described above, and preferably in double-stranded form, may be "operably linked" to sequences controlling transcriptional expression in an expression vector, and introduced into a host cell, either prokaryotic or eukaryotic, to produce recombinant NTP. Depending upon which strand of the NTP coding sequence is operably linked to the sequences controlling transcriptional expression, it is also possible to express an NTP antisense RNA.

Expression of the NTP in different hosts may result in different post-translational modifications which may alter the properties of the NTP. Preferably, the present invention encompasses the expression of an NTP in eukaryotic cells, and especially mammalian, insect, and yeast cells. Especially preferred eukaryotic hosts are mammalian cells. Mammalian cells provide post-translational modifications to recombinant NTP which include folding and/or phosphorylation. Most preferably, mammalian host cells include human CNS primitive neuroectodermal tumor cells, human neuroblastoma cells, human glioma cells, or rat glioma cells. Especially preferred primitive neuroectodermal tumor cells include PNET1 and PNET2, especially preferred human glioblastoma cells include Hg16 and Hg17, especially preferred human glioma cells include A172, and especially preferred rat glioma cells include C6 (*see* Example 1).

Alternatively, an NTP may be expressed by prokaryotic host cells. Preferably, a recombinant NTP is expressed by such cells as a fusion protein, as described herein. An especially preferred prokaryotic host is *E. coli*. Preferred strains of *E. coli* include Y1088, Y1089, CSH18, ER1451. and ER1647 (*see*, for example. *Molecular Biology LabFax.* Brown. T.A., Ed., Academic Press, New York (1991)). An alternative preferred host is *Bacillus subtilus,* including such strains as BR 151, YB886, M1119, M1120, and B170 (*see,* for.example, Hardy, "Bacillus Cloning Methods," in *DNA Cloning: A Practical Approach,* IRL Press, Washington, D.C. (1985)).

A nucleic acid molecule, such as DNA, is said to be "capable of expressing" a polypeptide if it contains expression control sequences which in turn contain transcriptional regulatory information and such sequences are "operably linked" to the nucleotide sequence which encodes the protein.

Two sequences of a nucleic acid molecule are said to be operably linked when they are linked to each other in a manner which either permits both sequences to be transcribed onto the same RNA transcript, or permits an RNA transcript, begun in one sequence to be extended into the second sequence. Thus, two sequences, such as a promoter sequence and any other "second" sequence of DNA or RNA are operably linked if transcription commencing in the promoter sequence will produce an RNA transcript of the operably linked second sequence. In order to be operably linked it is not necessary that two sequences be immediately adjacent to one another.

The promoter sequences of the present invention may be either prokaryotic, eukaryotic or viral. Suitable promoters are repressible, constitutive, or inducible. Examples of suitable prokaryotic promoters include promoters capable of recognizing the T4 polymerases (Malik *et al., J. Biol*. *Chem. 263*:1174-1181 (1984); Rosenberg *et al., Gene 59*:191-200 (1987); Shinedling *et al., J. Molec. Biol*. *195*:471-480 (1987); Hu *et al., Gene 42*:21-30 (1986)), T3, Sp6, and T7 (Chamberlin *et al., Nature 228*:227-231 (1970); Bailey *et al., Proc. Natl. Acad. Sci. (U.S.A.) 80*:2814-2818 (1983); Davanloo *et al., Proc. Natl. Acad. Sci*. *(U.S.A.)* 81:2035-2039 (1984)); the P_{R} and P_{L} promoters of bacteriophage lambda (*The Bacteriophage Lambda*, Hershey, A.D., Ed., Cold Spring Harbor Press, Cold Spring Harbor, NY (1973); *Lambda II*, Hendrix, R.W., Ed., Cold Spring Harbor Press, Cold Spring Harbor, NY (1980)); the trp, recA, heat shock, and lacZ promoters of *E. coli*; the α-amylase (Ulmanen *et al., J. Bacteriol. 162*:176-182 (1985)) and the delta-28-specific promoters of *B. subtilis* (Gilman *et al., Gene 32*:11-20 (1984)); the promoters of the bacteriophages of *Bacillus* (Gryczan, In: *The Molecular Biology of the Bacilli,* Academic Press, Inc., NY (1982)); *Streptomyces* promoters (Ward *et al., Mol. Gen. Genet. 203*:468-478 (1986)); the int promoter of bacteriophage lambda; the *bla* promoter of the β-lactamase gene of pBR322, and the CAT promoter of the chloramphenicol acetyl transferase gene of pBR325, etc. Prokaryotic promoters are reviewed by Glick, *J. Ind. Microbiol. 1*:277-282 (1987); Cenatiempo, *Biochimie* 68:505-516 (1986); Watson *et al*., In: *Molecular Biology of the Gene,* Fourth Edition, Benjamin Cummins, Menlo Park, CA (1987); Gottesman, *Ann. Rev. Genet. 18*:415-442 (1984); and Sambrook *et al*., *supra.*

Preferred eukaryotic promoters include the promoter of the mouse metallothionein I gene (Hamer *et al., J. Mol. Appl. Gen. 1*:273-288 (1982)); the TK promoter of Herpes virus (McKnight, *Cell 31*:355-365 (1982)); the SV40 early promoter (Benoist, *et al., Nature (London) 290*:304-310 (1981)); and the yeast *gal4* gene promoter (Johnston, *et al., Proc. Natl. Acad. Sci. (USA) 79*:6971-6975 (1982); Silver, *et al., Proc. Natl. Acad. Sci. (USA) 81*:5951-5955 (1984)). All of the above listed references are incorporated by reference herein.

Strong promoters are the most preferred promoters of the present invention. Examples of such preferred promoters are those which recognize the T3, SP6 and T7 polymerase promoters; the P_{L} promoter of bacteriophage lambda; the *recA* promoter and the promoter of the mouse metallothionein I gene. The most preferred promoter for expression in prokaryotic cells is one which is capable of recognizing the T7 polymerase promoter. The sequences of such polymerase recognition sequences are disclosed by Watson, *et al*. (In: *Molecular Biology of the Gene,* Fourth Edition, Benjamin Cummins, Menlo Park, CA, (1987)). The most preferred promoter for expression in mammalian cells is SV40 (Gorman, "High Efficiency Gene Transfer into Mammalian cells," in *DNA Cloning: A Practical Approach,* Volume II, IRL Press, Washington, D.C., pp. 143-190 (1985)).

### III. Methods of Detecting NTP

This invention is directed towards methods of detecting neurological disease in a human subject, utilizing the nucleic acid probes hybridisable to genes or transcripts of an NTP encoded by AD10-7 DNA molecule, or antibodies specific for an NTP encoded by AD10-7 DNA molecule. By "neurological" is meant Alzheimer's Disease (AD), or other neurodegenerative disorders with the Alzheimer's type pathogenic changes (for example, Parkinson's disease with AD-type neurodegeneration), as well as neuroectodermal tumors, malignant astrocytomas, and glioblastomas. By "human subject" is meant any human being or any developmental form thereof, such as a human embryo or fetus, prior to birth. The diagnostic methods of the present invention do not require invasive removal of neural tissue.

The present invention additionally pertains to assays, both nucleic acid hybridization assays and immunoassays, for detecting the presence of NTP encoded by AD10-7 DNA molecule in cells or in the biological fluids of a human subject using light or electron microscopic histology, imagining, radioactive or enzyme based assays, and the like.

### a. Nucleic Acid Hybridization Assays

In testing a tissue sample for an NTP using a nucleic acid hybridization assay, RNA can be isolated from tissue by sectioning on a cryostat and lysing the sections with a detergent such as SDS and a chelating agent such as EDTA, optionally with overnight digestion with proteinase K (50 µg/ml). Such tissue is obtained by autopsy and biopsy. A preferred quantity of tissue is in the range of 1-10 milligrams. Protein is removed by phenol and chloroform extractions, and nucleic acids are precipitated with ethanol. RNA is isolated by chromatography on an oligo dT column and then eluted therefrom. Further fractionation can also be carried out, according to methods well known to those of ordinary skill in the art.

A number of techniques for molecular hybridization are used for the detection of DNA or RNA sequences in tissues; each has certain advantages and disadvantages. When large amounts of tissue are available, analysis of hybridization kinetics provides the opportunity to accurately quantitate the amount of DNA or RNA present, as well as to distinguish sequences that are closely related but not identical to the probe, and determine the percent homology.

Reactions are run under conditions of hybridization (Tm-25°C) in which the rate of reassociation of the probe is optimal (Wetmur *et al., J. Mol. Biol. 31*:349-370 (1968)). The kinetics of the reaction are second- order when the sequences in the tissue are identical to those of the probe; however, the reaction exhibits complex kinetics when probe sequences have partial homology to those in the tissue (Sharp *et al., J. Mol. Biol. 86*:709-726 (1974)).

The ratio of probe to cell RNA is determined by the sensitivity desired. To detect one transcript per cell would require about 100 pg of probe per µg of total cellular DNA or RNA. The nucleic acids are mixed, denatured, brought to the appropriate salt concentration and temperature, and allowed to hybridize for various periods of time. The rate of reassociation can be determined by quantitating the amount of probe hybridized either by hydroxy apatite chromatography (Britten *et al., Science 161*:529-540 (1968)) or S1 nuclease digestion (Sutton, *Biochim. Biophys. Acta 240*:522-531 (1971)).

A more flexible method of hybridization is the northern blot technique. This technique offers variability in the stringency of the hybridization reaction, as well as determination of the state of the retroviral sequences in the specimen under analysis. Northern analysis can be performed as described herein.

A major consideration associated with hybridization analysis of DNA or RNA sequences is the degree of relatedness the probe has with the sequences present in the specimen under study. This is important with the blotting technique, since a moderate degree of sequence homology under nonstringent conditions of hybridization can yield a strong signal even though the probe and sequences in the sample represent non-homologous genes.

The particular hybridization technique is not essential to the invention, any technique commonly used in the art being within the scope of the present invention. Typical probe technology is described in United States Patent 4,358,535 to Falkow *et al*.. For example, hybridization can be carried out in a solution containing 6 x SSC (10 x SSC: 1.5 M sodium chloride, 0.15 M sodium citrate, pH 7.0), 5 x Denhardt's (1 x Denhardt's: 0.2% bovine serum albumin, 0.2% polyvinylpyrrolidone, 0.02% Ficoll 400), 10 mM EDTA, 0.5% SDS and about 10⁷ cpm of nick-translated DNA for 16 hours at 65°C.

The labeled probes, as described above, provide a general diagnostic method for detection of an NTP in tissue. The method is reasonably rapid, has a simple protocol, has reagents which can be standardized and provided as commercial kits, and allows for rapid screening of large numbers of samples.

In one method for carrying out the procedure, a clinical isolate containing RNA transcripts is fixed to a support. The affixed nucleic acid is contacted with a labeled polynucleotide having a base sequence complementary or homologous to the coding strand of the NTP gene.

The hybridization assays of the present invention are particularly well suited for preparation and commercialization in kit form, the kit comprising a carrier means compartmentalized to receive one or more container means (vial, test tube, etc.) in close confinement, each of said container means comprising one of the separate elements to be used in hybridization assay.

For example, there may be a container means containing NTP cDNA molecules suitable for labeling by "nick translation" *(see,* for example, *Sambrook et al., supra,* for standard methodology), or labeled NTP cDNA or RNA molecules. Further container means may contain standard solutions for nick translation of NTP cDNA comprising DNA polymerase I/DNase I and unlabeled deoxyribonucleotides (i.e., dCTP, dTTP, dGTP, and dATP).

The presence of NTP RNA is determined by the variation in the appearance and/or quantity of probe-related RNA in tested tissue.

The DNA probes of this invention can also be used for differential diagnosis of hereditary or familial AD and non-hereditary or sporadic AD. The familial form of AD often occurs at an earlier age and is associated with Down's syndrome in the family. Thus, a genetic test for familial AD allows for genetic counseling of families. While much effort has been directed toward characterizing a genetic marker for familial AD (Gusella, *FASEB J 3*:2036-2041 (1989); Hooper, *J NIH Res. 4*:48-54 (1992)), genetic linkage analysis only identifies a genetic marker sequence without providing the knowledge of the function of the genomic sequence. In contrast, the cDNA probes described herein and obtained from individuals with sporatic AD encode a known protein of known function which is over-expressed in brain tissue of patients with AD.

Most cases of the AD disorder appear to be the sporadic form, although there are well-documented familial cases (Gusella, *supra; Harrison's Principles of Internal Medicine,* Braunwald *et al.,* Eds., Eleventh Edition, Mc-Graw-Hill Book Company, New York, pp. 2012-2013 (1987)). A patient with familial AD, unlike a patient with sporadic AD, inherited the predisposing mutation through the germ cells. Some of the familial cases have been shown to follow an autosomal dominant pattern of inheritance (*Id*.). Thus, the DNA of a patient with familial AD will contain the inherited genetic alteration which is absent from the DNA of a patient with sporadic AD.

A method of differentiating between sporadic and familial AD in a human subject involves obtaining a biological sample from the human subject who is suspected of having Alzheimer's Disease. Then, DNA is purified from the biological sample. Finally, the DNA is contacted with a NTP DNA probe under conditions of hybridization. Familial AD is indicated by the detection of a hybrid of the probe and the DNA, whereas sporadic AD is indicated by the absence of detection of hybridization.

For example, the biological sample can be a blood sample which is subjected to differential centrifugation to enrich for white blood cells within three days of collection (Park, "PCR in the Diagnosis of Retinoblastoma," in *PCR Protocols,* Innis *et al*., Eds., Academic Press, Inc., New York, pp. 407-415 (1990)). The DNA sample can be prepared using the sodium N-lauroylsarcosine-Proteinase K, phenol, and RNase method (Sambrook *et al., supra).* DNA analysis can be performed by digesting the DNA sample, preferably 5 micrograms, with a restriction endonuclease (such as *Hind*III). Digested DNA is then fractionated using agarose gel electrophoresis, preferably, a 1% horizontal agarose gel, for 18 hours in a buffer preferably containing 89 mM Tris-Hcl (pH 8), 89 mM sodium borate and 2 mM EDTA (Gusella *et al., Nature 306*:234-238 (1983)). Southern analysis can be performed using conventional techniques (Sambrook *et al., supra*), and the labelled AD cDNA probes can be hybridized under conditions described above. The preferred DNA probes for this differential diagnosis method include 1-9a, AD3-4, AD4-4 and G2-2 PstI.

### b. Immunoassays

Antibodies directed against an NTP can be used, as taught by the present invention, to detect and diagnose AD. Various histological staining methods, including immunohistochemical staining methods, may also be used effectively according to the teaching of the invention. Silver stain is but one method of visualizing NTP. Other staining methods useful in the present invention will be obvious to the artisan, the determination of which would not involve undue experimentation (*see generally*, for example, *A Textbook of Histology,* Eds. Bloom and Fawcett, W.B. SaundersCo., Philadelphia (1964)).

One screening method for determining whether a given compound is an NTP functional derivative comprises, for example, immunoassays employing radioimmunoassay (RIA) or enzyme-linked immunosorbant assay (ELISA) methodologies, based on the production of specific antibodies (monoclonal or polyclonal) to an NTP. For these assays, biological samples are obtained by venepuncture (blood), spinal tap (cerebral spinal fluid (CSF)), urine and other body secretions such as sweat and tears. For example, in one form of RIA, the substance under test is mixed with diluted antiserum in the presence of radiolabeled antigen. In this method, the concentration of the test substance will be inversely proportional to the amount of labeled antigen bound to the specific antibody and directly related to the amount of free labeled antigen. Other suitable screening methods will be readily apparent to those of skill in the art.

The present invention also relates to methods of detecting an NTP encoded by AD10-7 DNA molecule or functional derivatives in a sample or subject. For example, antibodies specific for an NTP, or a functional derivative, may be detectably labeled with any appropriate marker, for example, a radioisotope, an enzyme, a fluorescent label, a paramagnetic label, or a free radical.

Alternatively, antibodies specific for an NTP, or a functional derivative, may be detectably labeled with DNA by the technique of immuno-polymerase chain reaction (Sano *et al., Science 258:* 120-122 (1992)). The polymerase chain reaction (PCR) procedure amplifies specific nucleic acid sequences through a series of manipulations including denaturation, annealing of oligonucleotide primers, and extension of the primers with DNA polymerase (*see,* for example. Mullis *et al*., U.S. Patent No. 4,683,202; Mullis *et al*., U.S. Patent No. 4,683,195; Loh *et al*., *Science 243*:217 (1988)). The steps can be repeated many times, resulting in a large amplification of the number of copies of the original specific sequence. As little as a single copy of a DNA sequence can be amplified to produce hundreds of nanograms of product (Li *et al., Nature 335*:414 (1988)). Other known nucleic acid amplification procedures include transcription-based amplification systems (Kwoh *et al., Proc. Natl. Acad. Sci. USA 86*:1173 (1989); Gingeras *et al.,* WO 88/10315), and the "ligase chain reaction" in which two (or more) oligonucleotides are ligated in the presence of a nucleic acid target having the sequence of the resulting "di-oligonucleotide" thereby amplifying the di-oligonucleotide (Wu *et al., Genomics 4*:560 (1989); Backman *et al*., EP 320,308; Wallace, EP 336,731; Orgel, WO 89/09835). For example, the immuno-PCR assay can be carried out by immobilizing various amounts of the test material on the surface of microtiter wells *(see* Sanzo *et al*., *supra,* page 122, footnote 7). The wells are subsequently incubated with an NTP monoclonal antibody, washed, and then incubated with biotinylated NTP DNA molecules which have been conjugated to streptavidin-protein chimera (*Id*.). This chimera binds biotin (via the streptavidin moiety) and the Fc portion of an immunoglobulin G molecule (via the protein A moiety) (*Id*., at 120; Sanzo *et al., Bio*/*Technology 9*:1378 (1991)). The wells are then washed to remove unbound conjugates. Any NTP present in the test material will be bound by the NTP monoclonal antibody, which in turn, is bound by the protein A moiety of the biotinylated NTP DNA - streptavidin-protein A conjugate. Then, the NTP DNA sequences are amplified using PCR. Briefly, the microtiter wells are incubated with deoxyribonucleoside triphosphates, NTP oligonucleotide primers, and Taq DNA polymerase *(see* Sanzo *et al*., *supra,* page 122, footnote 11). An automated thermal cycler (such as the PTC-100-96 Thermal Cycler, MJ Research, Inc.) can be used to perform PCR under standard conditions (*Id*.). The PCR products are then analyzed by agarose gel electrophoresis after staining with ethidium bromide.

Methods of making and detecting such detectably labeled antibodies or their functional derivatives are well known to those of ordinary skill in the art, and are described in more detail below. Standard reference works setting forth the general principles of immunology include the work of Klein (*Immunology: The Science of Self-Nonself Discrimination*, John Wiley & Sons, New York (1982)); Kennett *et al.* (*Monoclonal Antibodies and Hvbridonias: A New Dimension in Biological Analyses*, Plenum Press, New York (1980)); Campbell ("Monoclonal Antibody Technology," In: *Laboratory Techniques in Biochemistry and Molecular Biology*, Volume 13 (Burdon, R., *et al*., eds.). Elsevier, Amsterdam (1984)); and Eisen (In: *Microbiology*, 3rd Ed. (Davis, *et al*., Harper & Row, Philadelphia (1980)).

The term "antibody" refers both to monoclonal antibodies which are a substantially homogeneous population and to polyclonal antibodies which are heterogeneous populations. Polyclonal antibodies are derived from the sera of animals immunized with an antigen. Monoclonal antibodies (mAbs) to specific antigens may be obtained by methods known to those skilled in the art. *See,* for example, Kohler and Milstein, *Nature 256*:495-497 (1975) and U.S. Patent No. 4,376,110. Such antibodies may be of any immunoglobulin class including IgG, IgM, IgE, IgA, IgD and any subclass thereof.

The monoclonal antibodies, particularly mAbs Th7, Th9, and Th10 used in the present invention, may be prepared as previously described (Gross *et al., J. Clin. Invest. 76*:2115-2126 (1985); Ozturk *et al., Proc. Natl*. *Acad. Sci. USA 86*:419-423 (1989); de la Monte *et*. *al*., *J. Clin. Invest. 86*:1004-1013 (1990); de la Monte *et. al., J. Neurol*. *Sci. 113*:152-164 (1992); de la Monte *et al., Ann. Neurol. 32*:733-742 (1992)). The Th monoclonal antibodies were generated against the purified pancreatic form of thread protein (*Id*.). NTP-specific polyclonal and monoclonal antibodies can also be generated against a substantially pure NTP isolated from recombinant hosts (for example, *see* Carroll *et al.,* "Production and Purification of Polyclonal Antibodies to the Foreign Segment of β-Galactosidase Fusion Proteins," in *DNA Cloning: A Practical Approach,* Volume III, IRL Press, Washington, D.C., pp. 89-111 (1987); Mole *et al*., "Production of Monoclonal Antibodies Against Fusion Proteins Produced in *Escherichia coli,"* in *DNA Cloning: A Practical Approach*, Volume III, IRL Press, Washington, D.C., pp. 113-1139 (1987)). Alternatively, NTP-specific polyclonal and monoclonal antibodies can be generated against a substantially pure NTP isolated from biological material such as brain tissue and cell lines, by using well known techniques.

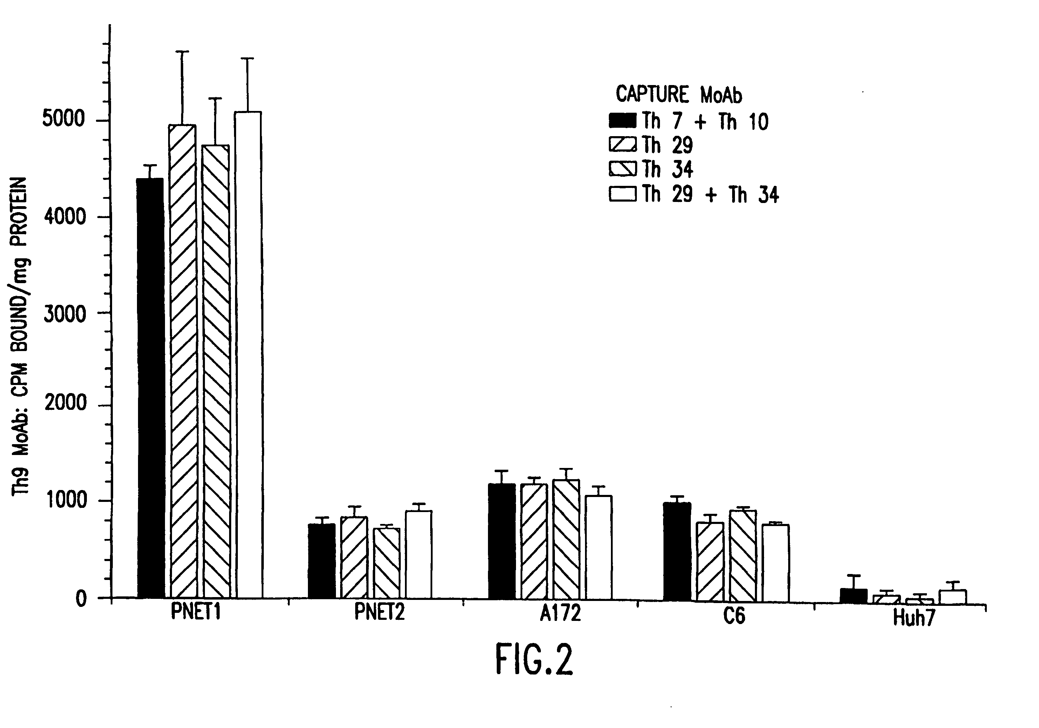

For example, monoclonal antibodies specific for the NTP molecule of approximately, 42 kDa molecular weight encoded by AD10-7 DNA molecule may be prepared from recombinant-derived proteins, which are expressed, isolated and purified from the cDNA (i.e., 1-9a), genomic clones (G2-2 Pstl) and AD-NTP 3-4 cDNA clones. The NTP molecules are derived from the above cDN'A's and genomic clones, inserted and produced in suitable expression vectors (*see* Figures 2A and 2B). Since there are regions of 60-70% homology in the 5' ends of the 1-9a NTP cDNA and PTP, one can obtain monoclonal antibodies that bind specifically to the NTP recombinant protein and not to the pancreatic form by performing routine differential screening (*see*, for example, de la Monte *et al., J. Clin. Invest. 86*: 1004-1013 (1990)). Although there will be monoclonal antibodies that bind to both NTP and PTP, it will be possible to generate NTP-specific monoclonal antibodies because there is a substantial sequence divergence between NTP molecules of various forms (e.g., 8, 14, 17, 21, 26 and 42 kDa) and because an epitope may be defined by as few as 6-8 amino acids.

The term "antibody" is also meant to include both intact molecules as well as fragments thereof, such as, for example, Fab and F(ab')₂, which are capable of binding antigen. Fab and F(ab')₂ fragments lack the Fc fragment of intact antibody, clear more rapidly from the circulation, and may have less non-specific tissue binding than an intact antibody (Wahl *et al., J. Nucl. Med. 24*:316-325 (1983)).

It will be appreciated that Fab and F(ab')₂ and other fragments of the antibodies useful in the present invention may be used for the detection and quantitation of an NTP according to the methods disclosed herein in order to detect and diagnose AD in the same manner as an intact antibody. Such fragments are typically produced by proteolytic cleavage, using enzymes such as papain (to produce Fab fragments) or pepsin (to produce F(ab')₂ fragments).

An antibody is said to be "capable of binding" a molecule if it is capable of specifically reacting with the molecule to thereby bind the molecule to the antibody. The term "epitope" is meant to refer to that portion of any molecule capable of being bound by an antibody which can also be recognized by that antibody. Epitopic determinants usually consist of chemically active surface groupings of molecules such as amino acids or sugar side chains and have specific three dimensional structural characteristics as well as specific charge characteristics.

An "antigen" is a molecule capable of being bound by an antibody which is additionally capable of inducing an animal to produce antibody capable of binding to an epitope of that antigen. An antigen may have one, or more than one epitope. The specific reaction referred to above is meant to indicate that the antigen will react, in a highly selective manner, with its corresponding antibody and not with the multitude of other antibodies which may be evoked by other antigens.

The antibodies, or fragments of antibodies, useful in the present invention may be used to quantitatively or qualitatively detect the presence of cells which contain the NTP antigens. Thus, the antibodies (or fragments thereof) useful in the present invention may be employed histologically to detect or visualize the presence of an NTP.

Such an assay for an NTP typically comprises incubating a biological sample from said subject suspected of having such a condition in the presence of a detectably labeled binding molecule (e.g., antibody) capable of identifying an NTP, and detecting said binding molecule which is bound in a sample.

Thus, in this aspect of the invention, a biological sample may be treated with nitrocellulose, or other solid support which is capable of immobilizing cells, cell particles or soluble proteins. The support may then be washed with suitable buffers followed by treatment with the detectably labeled NTP-specific antibody. The solid phase support may then be washed with the buffer a second time to remove unbound antibody. The amount of bound label on said solid support may then be detected by conventional means.

By "solid phase support" is intended any support capable of binding antigen or antibodies. Well-known supports, or carriers, include glass, polystyrene, polypropylene, polyethylene, dextran, nylon, amylases, natural and modified celluloses, polyacrylamides, agaroses, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will note many other suitable carriers for binding monoclonal antibody or antigen, or will be able to ascertain the same by use of routine experimentation.

One embodiment for carrying out the diagnostic assay of the present invention on a biological sample containing an NTP, comprises:
(a) contacting a detectably labeled antibody specific for an NTP encoded by AD10-7 DNA molecule with a solid support to effect immobilization of said NTP-specific antibody or a fragment thereof;
(b) contacting a sample suspected of containing an NTP encoded by AD10-7 DNA molecule with said solid support;
(c) incubating said detectably labeled antibody specific for an NTP encoded by AD10-7 DNA molecule with said support for a time sufficient to allow the immobilized NTP-specific antibody to bind to the NTP;
(d) separating the solid phase support from the incubation mixture obtained in step (c); and
(e) detecting the bound label and thereby detecting and quantifying NTP.

Alternatively, labeled NTP-specific antibody/NTP complexes in a sample may be separated from a reaction mixture by contacting the complex with an immobilized antibody or protein which is specific for an immunoglobulin, e.g., *Staphylococcus* protein A, *Staphylococcus* protein G, anti-IgM or anti-IgG antibodies. Such anti-immunoglobulin antibodies may be polyclonal, but are preferably monoclonal. The solid support may then be washed with a suitable buffer to give an immobilized NTP/labeled NTP-specific antibody complex. The label may then be detected to give a measure of an NTP.

This aspect of the invention relates to a method for detecting an NTP encoded by AD10-7 DNA molecule or a fragment thereof in a sample comprising:
(a) contacting a sample suspected of containing an NTP with an NTP-specific antibody or fragment thereof which binds to an NTP encoded by AD10-7 DNA molecule; and
(b) detecting whether a complex is formed.

The invention also relates to a method of detecting an NTP encoded by AD10-7 DNA molecule in a sample, further comprising:
(c) contacting mixture obtained in step (a) which an Fc binding molecule, such as an antibody, *Staphylococcus* protein A, or *Staphylococcus* protein G, which is immobilized on a solid phase support and is specific for the NTP encoded by AD10-7 DNA molecule specific antibody to give a NTP/NTP-specific antibody immobilized antibody complex;
(d) washing the solid phase support obtained in step (c) to remove unbound NTP/NTP-specific antibody complex;
(e) and detecting the label bound to said solid support.

Of course, the specific concentrations of detectably labeled antibody and NTP, the temperature and time of incubation, as well as other assay conditions may be varied, depending on various factors including the concentration of an NTP in the sample, the nature of the sample, and the like. The binding activity of a given lot of anti-NTP antibody may be determined according to well known methods. Those skilled in the art will be able to determine operative and optimal assay conditions for each determination by employing routine experimentation.

Other such steps as washing, stirring, shaking, filtering and the like may be added to the assays as is customary or necessary for the particular situation.

One of the ways in which the NTP-specific antibody can be detectably labeled is by linking the same to an enzyme. This enzyme, in turn, when later exposed to its substrate, will react with the substrate in such a manner as to produce a chemical moiety which can be detected, for example, by spectrophotometric, fluorometric or by visual means. Enzymes which can be used to detectably label the NTP-specific antibody include, but are not limited to, malate dehydrogenase, staphylococcal nuclease, delta-V-steroid isomerase, yeast alcohol dehydrogenase, α-glycerophosphate dehydrogenase, triose phosphate isomerase, horseradish peroxidase, alkaline phosphatase, asparaginase, glucose oxidase, β-galactosidase, ribonuclease, urease, catalase, glucose-VI-phosphate dehydrogenase, glucoamylase and acetylcholinesterase.

Detection may be accomplished using any of a variety of immunoassays. For example, by radioactively labeling the NTP-specific antibodies or antibody fragments, it is possible to detect NTP through the use of radioimmune assays. A good description of a radioimmune assay may be found in *Laboratory Techniques and Biochemistry in Molecular Biology,* by Work, *et al*., North Holland Publishing Company, NY (1978), with particular reference to the chapter entitled "An Introduction to Radioimmune Assay and Related Techniques" by Chard.

The radioactive isotope can be detected by such means as the use of a gamma counter or a scintillation counter or by autoradiography. Isotopes which are particularly useful for the purpose of the present invention are: ³H, ¹²⁵I, ¹³¹I, ³⁵S, ¹⁴C, and preferably ¹²⁵I.

It is also possible to label the NTP-specific antibody with a fluorescent compound. When the fluorescently labeled antibody is exposed to light of the proper wave length, its presence can then be detected due to fluorescence. Among the most commonly used fluorescent labelling compounds are fluorescein isothiocyanate, rhodamine, phycoerytherin, phycocyanin, allophycocyanin, *o*-phthaldehyde and fluorescamine.

The NTP-specific antibody can also be detectably labeled using fluorescence emitting metals such as ¹⁵²Eu, or others of the lanthanide series. These metals can be attached to the NTP-specific antibody using such metal chelating groups as diethylenetriaminepentaacetic acid (DTPA) or ethylenediaminetetraacetic acid (EDTA).

The NTP-specific antibody also can be detectably labeled by coupling it to a chemiluminescent compound. The presence of the chemiluminescent-tagged NTP-specific antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

The NTP-specific antibody may also be labeled with biotin and then reacted with avidin. A biotin-labeled DNA fragment will be linked to the NTP-biotinylated monoclonal antibody by an avidin bridge. NTP molecules are then detected by polymerase chain reaction (PCR) amplification of the DNA fragment with specific primers (Sano *et al*., *Science 258:* 120-122 (1992)).

Likewise, a bioluminescent compound may be used to label the NTP-specific antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

Detection of the NTP-specific antibody may be accomplished by a scintillation counter, for example, if the detectable label is a radioactive gamma emitter, or by a fluorometer, for example, if the label is a fluorescent material. In the case of an enzyme label, the detection can be accomplished by colorimetric methods which employ a substrate for the enzyme. Detection may also be accomplished by visual comparison of the extent of enzymatic reaction of a substrate in comparison with similarly prepared standards.

The detection of foci of such detectably labeled antibodies is indicative of a disease or dysfunctional state as previously described. For the purposes of the present invention, the NTP which is detected by this assay may be present in a biological sample. Any sample containing an NTP can be used. However, one of the benefits of the present diagnostic invention is that invasive tissue removal may be avoided. Therefore, preferably, the sample is a biological solution such as, for example, cerebrospinal fluid, amniotic fluid, blood, serum, urine and the like. However, the invention is not limited to assays using only these samples, it being possible for one of ordinary skill in the art to determine suitable conditións which allow the use of other samples.

For example, the three-site monoclonal antibody-based immunoradiometric assays (M-IRMA) may be used to measure NTP levels in a biological fluid, such as CSF. It is possible to obtain, by spinal tap, on a routine basis, CSF from individuals suspected of having AD. Thus, the diagnosis of AD can be established by a simple, non-invasive immunoassay which reveals NTP levels greatly increased over normal levels.

In one embodiment, as described above, this examination for AD is accomplished by removing samples of biological fluid and incubating such samples in the presence of detectably labeled antibodies (or antibody fragments). In a preferred embodiment, this technique is accomplished in a non-invasive manner through the use of magnetic imaging, fluorography, etc.

There are many different labels and methods of labeling known to those of ordinary skill in the art. Examples of the types of labels which can be used in the present invention include radioactive isotopes and paramagnetic isotopes. Those of ordinary skill in the art will know of other suitable labels for binding to the antibodies used in the invention, or will be able to ascertain such, using routine experimentation. Furthermore, the binding of these labels to the antibodies can be done using standard techniques common to those of ordinary skill in the art.

The antibodies (or fragments thereof) useful in the present invention are particularly suited for use in *in vitro* immunoassays to detect the presence of an NTP in body tissue, fluids (such as CSF), or cellular extracts. In such immunoassays, the antibodies (or antibody fragments) may be utilized in liquid phase or, preferably, bound to a solid-phase carrier, as described above.

Those of ordinary skill in the art will know of other suitable labels which may be employed in accordance with the present invention. The binding of these labels to antibodies or fragments thereof can be accomplished using standard techniques commonly known to those of ordinary skill in the art. Typical techniques are described by Kennedy, *et al.* (*Clin. Chim. Acta 70*:1-31 (1976)) and Schurs, *et al.* (*Clin. Chim. Acta 81*:1-40 (1977)). Coupling techniques mentioned in the latter are the glutaraldehyde method, the periodate method, the dimaleimide method, the m-maleimidobenzyl-N-hydroxy-succinimide ester method.

*In situ* detection may be accomplished by removing a histological specimen from a patient, and providing the combination of labeled antibodies of the present invention to such a specimen. The antibody (or fragment) is preferably provided by applying or by overlaying the labeled antibody (or fragment) to a biological sample. Through the use of such a procedure, it is possible to determine not only the presence of an NTP, but also the distribution of an NTP on the examined tissue. Using the present invention, those of ordinary skill will readily perceive that any of a wide variety of histological methods (such as staining procedures) can be modified in order to achieve such *in situ* detection.

The binding molecules of the present invention may be adapted for utilization in an immunometric assay, also known as a "two-site" or "sandwich" assay. In a typical immunometric assay, a quantity of unlabeled antibody (or fragment of antibody) is bound to a solid support that is insoluble in the fluid being tested (i.e., CSF) and a quantity of detectably labeled soluble antibody is added to permit detection and/or quantitation of the ternary complex formed between solid-phase antibody, antigen, and labeled antibody.

Typical, and preferred, immunometric assays include "forward" assays in which the antibody bound to the solid phase is first contacted with the sample being tested to extract the antigen from the sample by formation of a binary solid phase antibody-antigen complex. After a suitable incubation period, the solid support is washed to remove the residue of the fluid sample, including unreacted antigen, if any, and then contacted with the solution containing an unknown quantity of labeled antibody (which functions as a "reporter molecule"). After a second incubation period to permit the labeled antibody to complex with the antigen bound to the solid support through the unlabeled antibody, the solid support is washed a second time to remove the unreacted labeled antibody. This type of forward sandwich assay may be a simple "yes/no" assay to determine whether antigen is present or may be made quantitative by comparing the measure of labeled antibody with that obtained for a standard sample containing known quantities of antigen. Such "two-site" or "sandwich" assays are described by Wide at pages 199-206 of *Radioimmune Assay Method*, edited by Kirkham and Hunter, E. & S. Livingstone, Edinburgh, 1970.

In another type of "sandwich" assay, which may also be useful with the antigens of the present invention, the so-called "simultaneous" and "reverse" assays are used. A simultaneous assay involves a single incubation step as the antibody bound to the solid support and labeled antibody are both added to the sample being tested at the same time. After the incubation is completed, the solid support is washed to remove the residue of fluid sample and uncomplexed labeled antibody. The presence of labeled antibody associated with the solid support is then determined as it would be in a conventional "forward" sandwich assay.

In the "reverse" assay, stepwise addition first of a solution of labeled antibody to the fluid sample followed by the addition of unlabeled antibody bound to a solid support after a suitable incubation period is utilized. After a second incubation, the solid phase is washed in conventional fashion to free it of the residue of the sample being tested and the solution of unreacted labeled antibody. The determination of labeled antibody associated with a solid support is then determined as in the "simultaneous" and "forward" assays.

The above described *in vitro* detection methods may be used in the detection and diagnosis of AD without the necessity of removing tissue. Such detection methods may be used to assist in the determination of the stage of neurological deterioration in AD by evaluating and comparing the concentration of an NTP in the biological sample.

As used herein, an effective amount of a diagnostic reagent (such as an antibody or antibody fragment) is one capable of achieving the desired diagnostic discrimination and will vary depending on such factors as age, condition, sex, the extent of disease of the subject, counterindications, if any, and other variables to be adjusted by the physician. The amount of such materials which are typically used in a diagnostic test are generally between 0.1 to 5 mg, and preferably between 0.1 to 0.5 mg.

The assay of the present invention is also ideally suited for the preparation of a kit. Such a kit may comprise a carrier means being compartmentalized to receive in close confinement therewith one or more container means such as vials, tubes and the like, each of said container means comprising the separate elements of the immunoassay.

For example, there may be a container means containing a first antibody immobilized on a solid phase support, and a further container means containing a second detectably labeled antibody in solution. Further container means may contain standard solutions comprising serial dilutions of the NTP to be detected. The standard solutions of an NTP may be used to prepare a standard curve with the concentration of NTP plotted on the abscissa and the detection signal on the ordinate. The results obtained from a sample containing an NTP may be interpolated from such a plot to give the concentration of the NTP.

### IV. Isolation of NTP

The NTP protein encoded by AD10-7 DNA molecule or fragments of this invention may be obtained by expression from recombinant DNA as described above. Alternatively, the NTP encoded by AD10-7 DNA molecule may be purified from the biological material.

For purposes of the present invention, one method of purification which is illustrative, without being limiting, consists of the following steps.

A first step in the purification of an NTP encoded by AD10-7 DNA molecule includes extraction of the NTP fraction from a biological sample, such as brain tissue or CSF, in buffers, with or without solubilizing agents such as urea, formic acid, detergent, or thiocyanate.

A second step includes subjecting the solubilized material to ion-exchange chromatography on Mono-Q or Mono-S columns (Pharmacia LKB Biotechnology, Inc; Piscataway, NJ). Similarly, the solubilized material may be separated by any other process wherein molecules can be separated according to charge density, charge distribution and molecular size, for example. Elution of the NTP from the ion-exchange resin are monitored by an immunoassay, such as M-IRMA, on each fraction. Immunoreactive peaks would are then dialyzed, lyophilized, and subjected to molecular sieve, or gel chromatography.

Molecular sieve or gel chromatography is a type of partition chromatography in which separation is based on molecular size. Dextran, polyacrylamide, and agarose gels are commonly used for this type of separation. One useful gel for the present invention is Sepharose 12 (Pharmacia LKB Biotechnology, Inc.). However, other methods, known to those of skill in the an may be used to effectively separate molecules based on size.

A fourth step in a purification protocol for an NTP includes analyzing the immunoreactive peaks by sodium dodecyl sulfate-polyacrylamide gel electrophoresis (SDS-PAGE), a further gel chromatographic purification step, and staining, such as, for example, silver staining.

A fifth step in a purification method includes subjecting the NTP obtained after SDS-PAGE to affinity chromatography, or any other procedure based upon affinity between a substance to be isolated and a molecule to which it can specifically bind. For further purification of an NTP, affinity chromatography on Sepharose conjugated to anti-NTP mAbs (such as Th9, or specific mABs generated against substantially pure NTP) can be used. Alternative methods, such as reverse-phase HPLC, or any other method characterized by rapid separation with good peak resolution are useful.

Another method to purify an NTP encoded by AD10-7 DNA molecule is to use concentrated CSF obtained from patients with AD. For this procedure, 30-40 milliliters are concentrated by lyophilization or Amicon filtration or the like, and subjected to two dimensional gel electrophoresis. Proteins are separated in one direction by charge in a pH gradient and then, subjected to molecular sieve chromatography in the other direction by polyacrylamide gel electrophoresis. NTP-immunoreactive proteins are identified as spots by the Th monoclonal antibodies (for example, Th 9) using Western blot anaylsis. The gel is cut and NTP proteins are eluted from the gel. NTP purified in this manner can be sequenced or used to make new monoclonal antibodies.

It will be appreciated that other purification steps may be substituted for the preferred method described above. Those of skill in the art will be able to devise alternate purification schemes without undue experimentation.

### V. Gene Therapy Using Antisense Oligonucleotides and Ribozymes

Antisense oligonucleotides have been described as naturally occurring biological inhibitors of gene expression in both prokaryotes (*Mizuno et al., Proc. Natl. Acad. Sci. USA 81*:1966-1970 (1984)) and eukaryotes (Heywood. *Nucleic Acids Res. 14*:6771-6772 (1986)), and these sequences presumably function by hybridizing to complementary mRNA sequences, resulting in hybridization arrest of translation (Paterson, *et al., Proc. Natl. Acad. Sci. USA, 74*:4370-4374 (1987)).

Antisense oligonucleotides are short synthetic DNA or RNA nucleotide molecules formulated to be complementary to a specific gene or RNA message. Through the binding of these oligomers to a target DNA or mRNA sequence, transcription or translation of the gene can be selectively blocked and the disease process generated by that gene can be halted *(see,* for example, Jack Cohen, *Oligodeoxynucleotides, Antisense Inhibitors of Gene Expression*, CRC Press (1989)). The cytoplasmic location of mRNA provides a target considered to be readily accessible to antisense oligodeoxynucleotides entering the cell; hence much of the work in the field has focused on RNA as a target. Currently, the use of antisense oligodeoxynucleotides provides a useful tool for exploring regulation of gene expression *in vitro* and in tissue culture (Rothenberg, *et al., J. Natl. Cancer Inst*. *81*:1539-1544 (1989)).

Antisense therapy is the administration of exogenous oligonucleotides which bind to a target polynucleotide located within the cells. For example, antisense oligonucleotides may be administered systemically for anticancer therapy (Smith, International Application Publication No. WO 90/09180). As described herein, NTP-related proteins are produced by neuroectodermal tumor cells, malignant astrocytoma cells, glioblastoma cells, and in relatively high concentrations (i.e, relative to controls) in brain tissue of AD patients. Thus, NTP antisense oligonucleotides of the present invention may be active in treatment against AD, as well as neuroectodermal tumors, malignant astrocytomas, and glioblastomas.

The NTP antisense oligonucleotides of the present invention include derivatives such as S-oligonucleotides (phosphorothioate derivatives or S-oligos, *see,* Jack Cohen, *supra*). S-oligos (nucleoside phosphorothioates) are isoelectronic analogs of an oligonucleotide (O-oligo) in which a nonbridging oxygen atom of the phosphate group is replaced by a sulfur atom. The S-oligos of the present invention may be prepared by treatment of the corresponding O-oligos with 3*H*-1,2-benzodithiol-3-one-1,1-dioxide which is a sulfur transfer reagent. *See* lyer *et al., J. Org. Chem*. *55*:4693-4698 (1990); and lyer *et al., J. Am. Chem*. *Soc. 112*:1253-1254 (1990).

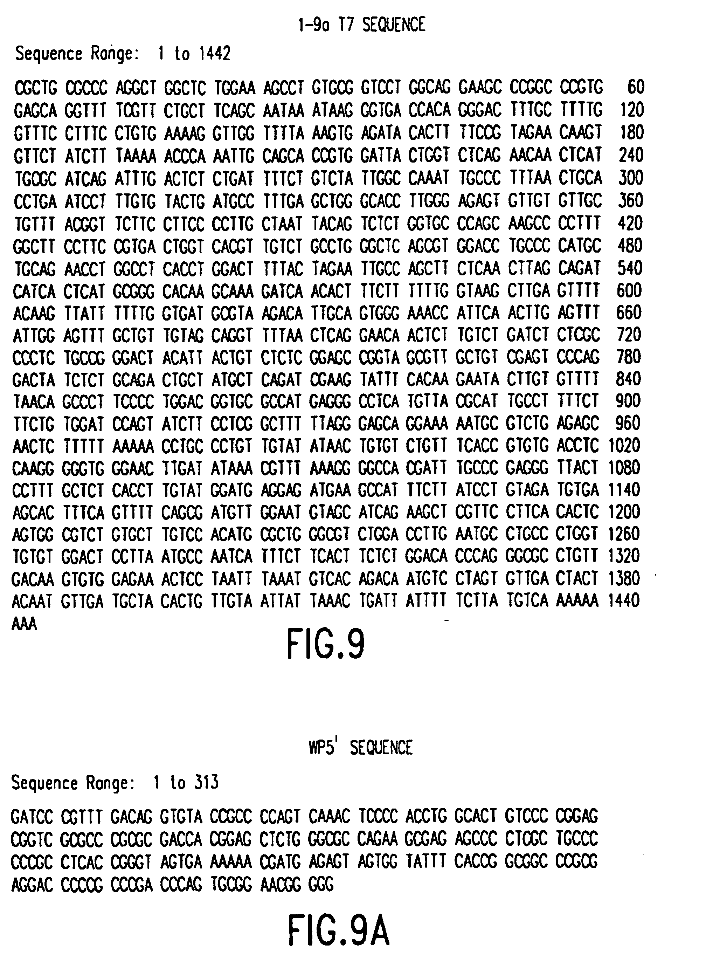

As described herein, sequence anaylsis of an NTP cDNA clone shows that NTP contains sequences which are nonhomologous to PTP DNA sequences (*see* Figure 9). Thus, the NTP antisense oligonucleotides of the present invention may be RNA or DNA which is complementary to and stably hybridizes with such sequences which are specific for the NTP encoded by AD10-7 DNA molecule. Use of an oligonucleotide complementary to this region allows for the selective hybridization to NTP mRNA and not to mRNA specifying PTP. Preferably, the NTP antisense oligonucleotides of the present invention are a 15 to 30-mer fragment of the antisense DNA molecule coding for the nonhomologous sequences of the AD 3-4 cDNA, such as: 1. 5'-CCGATTCCAACAGACCATCAT-3' [SEQ ID NO: 1]; 2. 5'-CCAACAGACCATCATTCCACC-3' [SEQ ID NO: 2]; and

3. 5'-CCAAACCGATTCCAACAGACC-3' [SEQ ID NO: 3].

Preferred antisense oligonucleotides bind to the 5'-end of the AD10-7 mRNA. Such antisense oligonucleotides may be used to down regulate or inhibit expression of the NTP gene. Examples of such antisense oligonucleotides (30-mers) include: 
1. 5'-CCTGGGCAACAAGAGCGAAAACTCCATCTC-3' [SEQ
ID NO: 4];
 
2. 5'-ATCGCTTGAACCCGGGAGGCGGAGGTTGCG-3' [SEQ
ID NO: 5];
 and

3.	5'-GGGGAGGCTGAGGCAGGAGAATCGCTTGAA-3'[SEQ
ID NO: 6].

Included as well in the present invention are pharmaceutical compositions comprising an effective amount of at least one of the NTP antisense oligonucleotides of the invention in combination with a pharmaceutically acceptable carrier. In one embodiment, a single NTP antisense oligonucleotide is utilized. In another embodiment, two NTP antisense oligonucleotides are utilized which are complementary to adjacent regions of the NTP genome. Administration of two NTP antisense oligonucleotides which are complementary to adjacent regions of the genome or corresponding mRNA may allow for more efficient inhibition of NTP genomic transcription or mRNA translation, resulting in more effective inhibition of NTP production.

Preferably, the NTP antisense oligonucleotide is coadministered with an agent which enhances the uptake of the antisense molecule by the cells. For example, the NTP antisense oligonucleotide may be combined with a lipophilic cationic compound which may be in the form of liposomes. The use of liposomes to introduce nucleotides into cells is taught, for example, in U.S. Patent Nos. 4,897,355 and 4,394,448. *See also* U.S. Patent Nos. 4,235,871, 4,231,877, 4,224,179, 4,753,788, 4,673,567, 4,247,411, 4,814,270 for general methods of preparing liposomes comprising biological materials.

Alternatively, the NTP antisense oligonucleotide may be combined with a lipophilic carrier such as any one of a number of sterols including cholesterol, cholate and deoxycholic acid. A preferred sterol is cholesterol.

In addition, the NTP antisense oligonucleotide may be conjugated to a peptide that is ingested by cells. Examples of useful peptides include peptide hormones, antigens or antibodies, and peptide toxins. By choosing a peptide that is selectively taken up by the neoplastic cells, specific delivery of the antisense agent may be effected. The NTP antisense oligonucleotide may be covalently bound via the 5'OH group by formation of an activated aminoalkyl derivative. The peptide of choice may then be covalently attached to the activated NTP antisense oligonucleotide via an amino and sulfhydryl reactive hetero bifunctional reagent. The latter is bound to a cysteine residue present in the peptide. Upon exposure of cells to the NTP antisense oligonucleotide bound to the peptide, the peptidyl antisense agent is endocytosed and the NTP antisense oligonucleotide binds to the target NTP mRNA to inhibit translation (Haralambid *et al.,* WO 8903849; Lebleu *et al*., EP 0263740).

The NTP antisense oligonucleotides and the pharmaceutical compositions of the present invention may be administered by any means that achieve their intended purpose. For example, administration may be by parenteral, subcutaneous, intravenous, intramuscular, intra-peritoneal, or transdermal routes. The dosage administered will be dependent upon the age, health, and weight of the recipient, kind of concurrent treatment, if any, frequency of treatment, and the nature of the effect desired.

Compositions within the scope of this invention include all compositions wherein the NTP antisense oligonucleotide is contained in an amount effective to achieve inhibition of proliferation and/or stimulate differentiation of the subject cancer cells, or alleviate AD. While individual needs vary, determination of optimal ranges of effective amounts of each component is with the skill of the art. Typically, the NTP antisense oligonucleotide may be administered to mammals, e.g. humans, at a dose of 0.005 to I mg/kg/day, or an equivalent amount of the pharmaceutically acceptable salt thereof, per day of the body weight of the mammal being treated.

Alternatively, antisense oligonucleotides can be prepared which are designed to interfere with transcription of the NTP gene by binding transcribed regions of duplex DNA (including introns, exons, or both) and forming triple helices (Froehler *et al*., WO 91/06626; Toole, WO 92/10590). Preferred oligonucleotides for triple helix formation are oligonucleotides which have inverted polarities for at least two regions of the oligonucleotide (*Id*.). Such oligonucleotides comprise tandem sequences of opposite polarity such as 3'---5'-L-5'---3', or 5'---3'-L-3'---5', wherein L represents a 0-10 base oligonucleotide linkage between oligonucleotides. The inverted polarity form stabilizes single-stranded oligonucleotides to exonuclease degradation (Froehler *et al., supra*). Preferred triple helix-forming oligonucleotides are based upon SEQ ID NOs 1-3: 
1.	3′-TACTACCAGACAACCTTAGCC-5′-L-
5′-CCGATTCCAACAGACCATCAT-3′ [SEQ ID NO: 7];
 
2. 5'-CCGATTCCAACAGACCATCAT-3'-L-
3'-TACTACCAGACAACCTTAGCC-5' [SEQ ID NO: 8];
 
3.	3'-CCACCTTACTACCAGACAACC-5'-L-
5'-CCAACAGACCATCATTCCACC-3' [SEQ ID NO: 9];
 
4. 5′-CCAACAGACCATCATTCCACC-3′-L-
3′-CCACCTTACTACCAGACAACC-5′ [SEQ ID NO: 10];
 
5. 3'-CCAGACAACCTTAGCCAAACC-5'-L-
5'-CCAAACCGATTCCAACAGACC-3' [SEQ ID NO: 11];
 and 
6. 5'-CCAAACCGATTCCAACAGACC-3'-L-
3'-CCAGACAACCTTAGCCAAACC-5' [SEQ ID NO: 12].
 Thus, triple helix-forming oligonucleotides 1 and 2 are represented as 3'[SEQ ID NO: 1]5'-L-5'[SEQ ID NO: 1]3' and 5'[SEQ ID NO: 1]3'-L-3'[SEQ ID NO: 1]5', respectively. Triple helix-forming oligonucleotides 3 and 4 are represented as 3'[SEQ ID NO: 2]5'-L-5'[SEQ ID NO: 2]3' and 5'[SEQ ID NO: 2]3'-L-3'[SEQ ID NO: 2]5', respectively. Triple helix-forming oligonucleotides 5 and 6 are represented as 3'[SEQ ID NO: 3]5'-L-5'[SEQ ID NO: 3]3' and 5'[SEQ ID NO: 3]3'-L-3'[SEQ ID NO: 3]5', respectively. Of course, similar triple helix-forming oligonucleotide may be prepared with SEQ ID NOs. 4-6, or fragments thereof.

In therapeutic application, the triple helix-forming oligonucleotides can be formulated in pharmaceutical preparations for a variety of modes of administration, including systemic or localized administration, as described above.

The antisense oligonucleotides of the present invention may be prepared according to any of the methods that are well known to those of ordinary skill in the art, as described above.

Ribozymes provide an alternative method to inhibit mRNA function. Ribozymes may be RNA enzymes, self-splicing RNAs, and self-cleaving RNAs (Cech *et al., Journal of Biological Chemistry 267:*17479-17482 (1992)). It is possible to construct *de novo* ribozymes which have an endonuclease activity directed in *trans* to a certain target sequence. Since these ribozymes can act on various sequences, ribozymes can be designed for virtually any RNA substrate. Thus, ribozymes are very flexible tools for inhibiting the expression of specific genes and provide an alternative to antisense constructs.

A ribozyme against chloramphenicol acetyltransferase mRNA has been successfully constructed (Haseloff *et al*., *Nature 334*:585-591 (1988); Uhlenbeck *et al*., *Nature 328*:596-600 (1987)). The ribozyme contains three structural domains: 1) a highly conserved region of nucleotides which flank the cleavage site in the 5' direction; 2) the highly conserved sequences contained in naturally occurring cleavage domains of ribozymes, forming a base-paired stem; and 3) the regions which flank the cleavage site on both sides and ensure the exact arrangement of the ribozyme in relation to the cleavage site and the cohesion of the substrate and enzyme. RNA enzymes constructed according to this model have already proved suitable *in vitro* for the specific cleaving of RNA sequences (Haseloff *et al., supra*).

Alternatively, hairpin ribozymes may be used in which the active site is derived from the minus strand of the satellite RNA of tobacco ring spot virus (Hampel *et al., Biochemistry 28*:4929-4933 (1989)). Recently, a hairpin ribozyme was designed which cleaves human immunodeficiency virus type 1 RNA (Ojwang *et al., Proc. Natl. Acad. Sci. USA 89*:10802-10806 (1992)). Other self-cleaving RNA activities are associated with hepatitis delta virus (Kuo *et al., J. Virol. 62*:4429-4444 (1988)).

As discussed above, preferred targets for NTP ribozymes are the nucleotide sequences which are not homologous with PTP sequences. Preferably, the NTP ribozyme molecule of the present invention is designed based upon the chloramphenicol acetyltransferase ribozyme or hairpin ribozymes, described above. Alternatively, NTP ribozyme molecules are designed as described by Eckstein *et al*. (International Publication No. WO 92/07065) who disclose catalytically active ribozyme constructions which have increased stability against chemical and enzymatic degradation, and thus are useful as therapeutic agents.

In an alternative approach, an external guide sequence (EGS) can be constructed for directing the endogenous ribozyme, RNase P, to intracellular NTP mRNA, which is subsequently cleaved by the cellular riboryme (Altman *et al.,* U.S. Patent No. 5,168,053). Preferably, the NTP EGS comprises a ten to fifteen nucleotide sequence complementary to an NTP mRNA and a 3'-NCCA nucleotide sequence, wherein N is preferably a purine (*Id*.). After NTP EGS molecules are delivered to cells, as described below, the molecules bind to the targeted NTP mRNA species by forming base pairs between the NTP mRNA and the complementary NTP EGS sequences, thus promoting cleavage of NTP mRNA by RNase P at the nucleodde at the 5'side of the base-paired region (*Id*.).

Alternatively, NTP antisense RNA molecules, NTP ribozymes, and NTP EGS can be coded by DNA constructs which are administered in the form of virions, which are preferably incapable of replicating *in vivo* (*see,* for example, Taylor, WO 92/06693). For example, such DNA constructs may be administered using herpes-based viruses (Gage *et al*., U.S. Patent No. 5,082,670). Alternatively, NTP antisense RNA sequences, NTP ribozymes, and NTP EGS can be coded by RNA constructs which are administered in the form of virions, such as retroviruses. The preparation of retroviral vectors is well known in the art (*see,* for example, Brown *et al.,* "Retroviral Vectors," in *DNA Cloning: A Practical Approach,* Volume 3, IRL Press, Washington, D.C. (1987)).

Specificity for gene expression in the central nervous system can be conferred by using appropriate cell-specific regulatory sequences, such as cell-specific enhancers and promoters. For example, such sequences include the sequences that regulate the oligodendroglial-specific expression of JC virus, glial-specific expression of the proteolipid protein, and the glial fibrillary acidic protein genes (Gage *et al., supra*)*.* Since protein phosphorylation is critical for neuronal regulation (Kennedy, "Second Messengers and Neuronal Function," in *An Introduction to Molecular Neurobiology*, Hall, Ed., Sinauer Associates, Inc. (1992)), protein kinase promoter sequences can be used to achieve sufficient levels of NTP gene expression.

Thus, gene therapy can be used to alleviate AD by inhibiting the inappropriate expression of a NTP encoded by AD10-7 DNA molecule. Moreover, gene therapy can be used to alleviate AD by providing the appropriate expression level of a NTP encoded by AD10-7 DNA molecule. In this case, particular NTP nucleic acid sequences may be coded by DNA or RNA constructs which are administered in the form of viruses, as described above. Alternatively, "donor cells" may be modified *in vitro* using viral or retroviral vectors containing NTP sequences, or using other well known techniques of introducing foreign DNA into cells (see, for example, *Sambrook et al*., *supra*). Such donor cells include fibroblast cells, neuronal cells, glial cells, and connective tissue cells (*Gage et al*., *supra).* Following genetic manipulation, the donor cells are grafted into the central nervous system and thus, the genetically-modified cells provided the therapeutic form of NTP (*Id*.).

Moreover, such virions may be introduced into the blood stream for delivery to the brain. This is accomplished through the osmotic disruption of the blood brain barrier prior to administration of the virions (*see,* for example, Neuwelt, United States Patent No. 4,866,042). The blood brain barrier may be disrupted by administration of a pharmaceutically effective, nontoxic hypertonic solution, such as mannitol, arabinose, or glycerol (*Id*.).

The following clones in *E. coli* were deposited according to the Budapest Treaty with the American Type Culture Collection (12301 Parklawn Drive, Rockville, Maryland, 20852): G2-2 PstI-DH5 (ATCC No. 69257); G5d-PstI-DH5 (ATCC No. 69258); 1-9a-LX-1 blue (ATCC No. 69259); AD3-4-DH1 (ATCC No. 69260); HB4-XL-blue (ATCC No. 69261); AD10-7-DH1 (ATCC No. 69262); AD2-2-DH 1-(ATCC No. 69263); G5d-1PstI-EcoRI-DH5 (ATCC No. 69264); and G2-2PstI-EcoRI-DH5 (ATCC No. 69265).

Having now generally described the invention, the same will be more readily understood through reference to the following Examples which are provided by way of illustration, and are not intended to be limiting of the present invention, unless specified.

### Example 1

### Expression of NTP Immunoreactivity in Cell Lines

Seven cell lines of central nervous system origin were identified that express thread protein immunoreactivity using the Th9 monoclonal antibody which was generated to the pancreatic form of the protein (Gross *et al*., *J. Clin. Invest. 76*:2115-2126 (1985)), but cross-reacts with thread proteins present in brain tissue and cerebrospinal fluid (Ozturk *et al*., *Proc. Natl. Acad. Sci. USA 86*:419-423 (1989); de la Monte *et*. *al*., *J. Clin. Invest. 86*:1004-1013 (1990); de la Monte *et*. *al*., *J. Neurol. Sci*. *113*:152-164 (1992); de la Monte *et al*., *Ann*. *Neurol*. *32*:733-742 (1992)). Among them were the following: two primitive neuroectodermal tumor (PNET) cell lines designated PNET1 and PNET2; three glioblastoma cell lines Hgl 16, Hgl 17, and C6; the A172 glial cell line; and the SH-Sy5y neuroblastoma cell line. The glioblastoma cell lines and the A172 cells were obtained from the American Type Culture Collection (ATCC). SH-Sy5y cells were obtained from Dr. Biedler at Sloan-Kettering Memorial Hospital. The PNET cell lines have been described previously (The *et al., Nature genetics 3*:62-66 (1993)), and were obtained from Dr. Rene' Bernards at the MGH Cancer Center. All cell lines were maintained in Earl's Modified Eagle Medium supplemented with 10% fetal calf serum, and without antibiotics.

To examine the cells for thread protein and other immunoreactivities, the cultures were harvested in phosphate buffered saline (PBS) (137 mM NaCl, 2.7 mM KCI, 4.3 mM Na₂HPO4, 1.4 mM KH₂PO₄, pH 7.3) containing 2 mM EDTA, and cytospin preparations were made using 10⁵ cells per slide. The cytospin preparations were fixed immediately in 100% methanol (-20°C), air-dried, and then stored at -80°C until used. Prior to immunostaining, the slides were equilibrated to room temperature and hydrated in PBS. Nonspecific antibody binding was blocked with 3% nonimmune horse serum. Replicate cytospin preparations from the same cultures were incubated overnight at 4°C with 5 or 10 µg/ml of primary antibody. Immunoreactivity was revealed by the avidin-biotin horseradish peroxidase method using the Vectastain Elite kit (Vector Laboratories, Burlingame, CA) according to the manufacturer's protocol, and with 3-3' diaminobenzidine (0.5 mg/ml plus 0.03% hydrogen peroxide) as the chromogen. The cells then were counterstained with hematoxylin, dehydrated in graded alcohol solutions, cleared in xylenes, and preserved under coverglass with Permount (Fisher Scientific).

Cytospin preparations of each cell line were immunostained with the thread protein monoclonal antibodies Th9, Th7, Th10, Th29, Th34, TH46, Th67, and Th90. In addition, replicate slides were immunostained with positive (neurofilament, glial fibrillary acidic protein (GFAP), and vimentin) and negative (desmin, Hepatitis B surface antigen-5C3) control monoclonal antibodies. Except for 5C3 which was generated in the inventor's laboratory (Fujita *et al., Gastroenterology 91*:1357-1363 (1986)), the control antibodies were purchased (Boehringer-Mannheim). All serological reagents were diluted in PBS containing 1 % bovine serum albumin (BSA), and all incubations except the one with primary antibody were carried out at room temperature in humidified chambers. The slides were washed in 3 changes of PBS between each step.

Both PNET1 and PNET2 cells expressed high and middle molecular weight neurofilament proteins and little or no glial fibrillary acidic protein or vimentin. The PNET1, PNET2, and SH-Sy5y cells expressed GAP-43, an abundant calmodulin-binding phosphoprotein that is highly expressed in immature neurons and in neurons undergoing regenerative cell growth (Benowitz *et al., J. Neurosci. 3*:2153-2163 (1983); DeGraan *et al*., *Neurosci. Lett. 61*:235-241 (1985); Kalil *et al., J. Neurosci. 6*:2563-2570 (1986)). The A172 and C6 cells expressed GFAP and vimentin. However, A172 also exhibited neurofilament immunoreactivity, raising doubt about its purely glial cell nature. None of the cell lines manifested immunoreactivity with monoclonal antibodies to desmin or to Hepatitis B surface antigen. As a negative control cell line, the Huh7 hepatocellular carcinoma cell line was similarly immunostained, and found not to exhibit any immunoreactivity with the above antibodies. However, the Huh cells were immunoreactive with monoclonal antibodies to the insulin receptor substrate protein, 1RS-1 (data not shown) which was used as a positive control for this cell line (Sasaki *et al*., *J. Biol*. *Chem. 268*:1-4 (1993)).

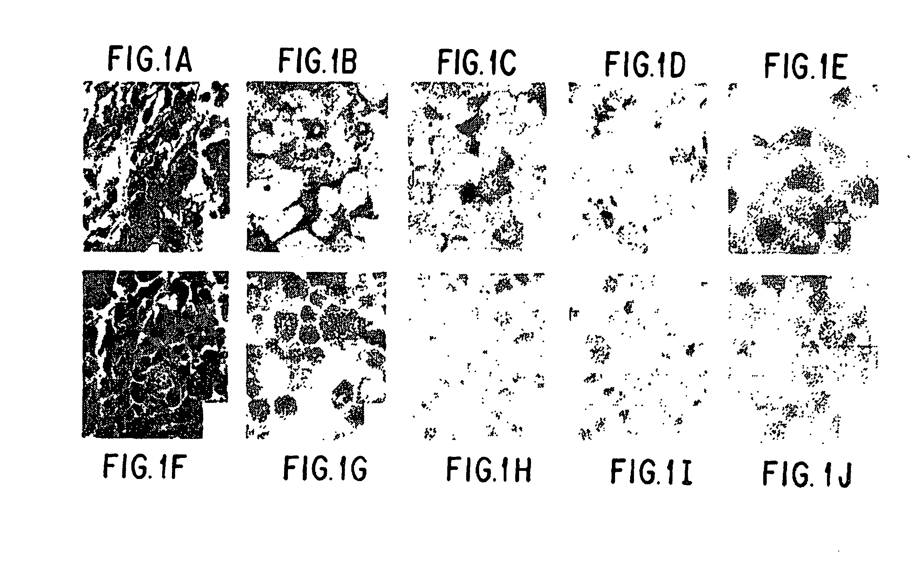

Using the Th9 monoclonal antibody, thread protein immunoreactivity was detected in primary PNET (A), primary glioblastoma (F), PNET1 (B), and C6 cells (G), but not in hepatocellular carcinoma cell lines (Figure 1). In addition, Th9 immunoreactivity was detected in histological sections from 8 of the 9 primary human CNS PNETs, and from all 5 of the primary human glioblastomas studied (Figure 1). Although all 5 cell lines exhibited intense immunoreactivity with the Th9 monoclonal antibody, they differed with respect to immunoreactivity for other Th monoclonal antibodies. The immunostaining reaction generated with the Th10 (C,H), Th7 (D,I), or Th46 monoclonal antibodies was either low-level (C,D) or absent (H,I,E,J) in PNET1 (C-E) and C6 (H-J). PNET2 cells exhibited only low levels of immunoreactivity with Th7 and Th29, and they manifested no immunostaining with the other Th monoclonal antibodies. A172, C6, and SH-Sy5y cells displayed little or no immunoreactivity with Th monoclonal antibodies other than Th9. Huh7 cells exhibited no immunoreactivity with any of the thread protein monoclonal antibodies employed, whereas human pancreatic tissue was immunoreactive with all of the Th antibodies, which had been generated against the purified pancreatic form of thread protein (Gross *et al*., *J. Clin. Invest. 76*:2115-2126 (1985)).

### Example 2

### Analysis of Thread Proteins by Monoclonal Antibody-Based Immunoradiometric Assay (M-IRMA)

Cultured cells were washed in PBS and recovered in PBS containing 2 mM EDTA. The cells were pelleted by centrifugation at 1000 x g for 15 min, and then resuspended in lysis buffer containing 50 mM Tris-HCl (pH 7.5), 1% Triton X-100, 2 mM EGTA. 10 mM EDTA, 100 mM NaF, 1 mM Na₄P₂O₇, 2 mM Na₃VO₄, 100 µg/ml phenylmethylsulfonyl fluoride, 1 µg/ml aprotinin, 1 µg/ml pepstatin A, and 1 µg/ml leupeptin. The supernatant fractions obtained by centrifugation of the lysates at 14,000 x g for 10 min were used for the Western blot analysis, immunoprecipitation studies, and M-IRMA. Protein concentration was determined by the Lowry colorimetric assay. The samples were stored at -40°C.

M-IRMA is a highly sensitive two- or three-site forward sandwich assay which permits quantitation of picomolar NTP in cell lysates, tissue culture medium, tissue homogenates, and body fluids (Ozturk *et al., Proc. Natl. Acad. Sci. USA 86*:419-423 (1989); de la Monte *et. al., J. Clin. Invest. 86*:1004-1013 (1990); de la Monte *et. al., J. Neurol. Sci. 113*:152-164 (1992): de la Monte *et al*., *Ann. Neurol. 32*:733-742 (1992); Gross *et al., J. Clin. Invest. 76*:2115-2126 (1985)). In addition, when combined with SDS-PAGE, M-IRMA can be used to determine molecular size of thread proteins and related species (Ozturk *et al*., *Proc. Natl. Acad. Sci. USA 86*:419-423 (1989); de la Monte *et. al., J. Clin. Invest. 86*:1004-1013 (1990); de la Monte *et. al., J. Neurol. Sci. 113*:152-164 (1992); de la Monte *et al., Ann. Neurol. 32*:733-742 (1992)). M-IRMA involves capturing the immunoreactive thread proteins present in biological samples using monoclonal antibodies Th7 and Th10 affixed to a solid-phase matrix, and then detecting the captured antigen with a third radiolabeled tracer monoclonal antibody (Th9) to the same protein. Briefly, 1/4" polystyrene beads (Precision Ball, Inc) were coated with one or two monoclonal antibodies to thread proteins (usually Th7 + Th10). Cell lysates or supernatant fractions of tissue homogenates (Ozturk *et al*., *Proc. Natl. Acad. Sci. USA 86*:419-423 (1989); de la Monte *et. al., J. Clin. Invest. 86*:1004-1013 (1990); de la Monte *et*. *al*., *J. Neurol. Sci. 113*:152-164 (1992); de la Monte *et al*., *Ann. Neurol. 32*:733-742 (1992)) were incubated over night with the coated beads to capture thread proteins present in the samples. The beads were washed 5x in PBS, and then incubated with ¹²⁵-I labeled Th9 as a tracer to detect the captured thread proteins. The concentration of thread protein in the lysate or tissue homogenate was determined from a standard curve generated with known quantities of purified thread protein. This highly sensitive assay can detect as little as 10 pmol of thread protein in solution. To assay for thread proteins fractionated by SDS-PAGE, the wet gels were sliced at 2 mm intervals, and the proteins were eluted from each fraction into 0.5 ml of PBS by shaking for 24 hours at room temperature. The eluates were assayed directly for thread proteins by M-IRMA.

Corresponding with the widespread immunocytochemical staining of PNET1 cells with Th7, Th10, Th34, and Th29, thread protein immunoreactivity was readily measured in these cells by M-IRMA. In other words, with Th7, Th10, Th34, and Th29 monoclonal antibodies (MoAb) used as capture antibodies, either singularly or with two of them together, and ¹²⁵-I labeled Th9 was used as the tracer, similarly high levels of thread protein were measured (Figure 2). In contrast, in PNET2, C6, and A172 cells, which exhibited intense immunoreactivity with Th9, but little or no immunocytochemical staining with the Th monoclonal antibodies that were used to capture antigen, the levels of thread protein detected by M-IRMA were much lower than those measured in the PNET1 cells (Figure 2). Similarly, Huh7 cells, which manifested no immunocytochemical staining with any of the thread protein monoclonal antibodies, had virtually nondetectable levels of thread proteins in the cellular lysates by M-IRMA. The concentrations of thread protein in the cell lysates were computed from a standard curve generated with purified PTP using Th7 and Th10 as capture antibodies. The results expressed as mean S.D. pg/mg of total protein were as follows: PNET1-13.1 ± 0.39; PNET2-2.06 ± 0.10; A172-3.38 ± 0.37; C6-2.52 ± 0.22; and Huh7-0.34 ± 0.05.

### Example 3

### Characterization of Neural Thread Proteins in Tumor Cell Lines

In Western Blot anaylsis, samples containing 100 µg of protein were fractionated by SDS-PAGE, along with pre-labeled molecular weight standards. The proteins were blotted onto nylon membranes (Immobilon-P transfer membrane, Millipore) using a semi-dry transfer apparatus (Integrated Systems). The membranes were washed in Tris buffered saline (TBS; 10 mM Tris. 0.85% sodium chloride, pH 7.5), and then blocked with TBS containing 3% BSA. The blots were incubated overnight at 4°C with ¹²⁵-I labeled Th9 monoclonal antibody. Unspecifically bound probe was removed by washing the membranes at room temperature in TBS-BSA 3 x 15 min, and 1 x 30 min. The results were analyzed by autoradiography using Kodak XAR film.

To prepare samples for immunoprecipitation studies, one milliliter samples of cell lysate containing approximately 1 mg/ml of protein were used for immunoprecipitation studies. The lysates were initially pre-cleared with non-relevant antibody (5C3 or antidesmin), and then with Protein A sepharose. Thread proteins were immunoprecipitated using 5-10 µg of Th9 and Protein A sepharose (Sasaki *et al., J. Biol. Chem. 268*:1-4 (1993)). The immune complexes collected by centrifugation were resuspended in buffer containing 2 % SDS and 10 mM β-mercaptoethanol, and then subjected to SDS-PAGE under denaturing and reducing conditions (*Id*.). Crude cellular lysates (100 µg protein) were analyzed simultaneously. The proteins were blotted onto Immobilon-P membranes and probed with ¹²⁵-I labeled (*ld*.) Th9 to detect thread proteins and related molecules. Negative control experiments were performed simultaneously using either monoclonal antibodies to Hepatitis B surface antigen (SC3) or to desmin.

Metabolic labeling experiments were performed using monolayers of cells cultured in 100 mm² petri dishes. Prior to labeling, the cells were exposed to methionine- and cysteine-free medium for 2 h. The medium was then replaced with 3 ml of DMEM containing 300 µCi each of [³⁵S] methionine or [³⁵S] cysteine. After labeling for 3 hours, the cells were incubated for various intervals with complete medium devoid radiolabeled amino acids and supplemented with 10 mM methionine. Cell lysates were prepared as described above. Thread proteins were immunoprecipitated using the Th9 monoclonal antibody and protein A sepharose, and the immunoprecipitation products were analyzed by SDS-PAGE and film autoradiography.

For the *in vivo* phosphorylation studies, cells cultured as described for metabolic labeling studies were washed twice with TBS and incubated for 2 h with phosphate-free Dulbecco's MEM containing 10% dialyzed fetal calf serum. Then the cells were washed with TBS and incubated for 3 h with the same medium containing 400 µCi/ml of [³²P] orthophosphoric acid. The cell lysates were analyzed by immunoprecipitation with thread protein, and both positive (p36) and negative (desmin) control monoclonal antibodies, followed by SDS-PAGE.

In order to study the glycosylation state of neural thread proteins, cell culture lysates containing approximately 100 µg or protein were subjected to SDS-PAGE, and the fractionated proteins were transferred to Immobilon-P membranes (Millipore). O- and N-glycans were detected by periodate oxidation followed by biotinylation, and then Western blot analysis with a Streptavidin-alkaline phosphatase probe and NBT/BCIP as the colorimetric substrate. The assays were performed using the GlycoTrack Kit (Oxford Glycosystems, Rosedale, NY) according to the protocol provided by the manufacturer.

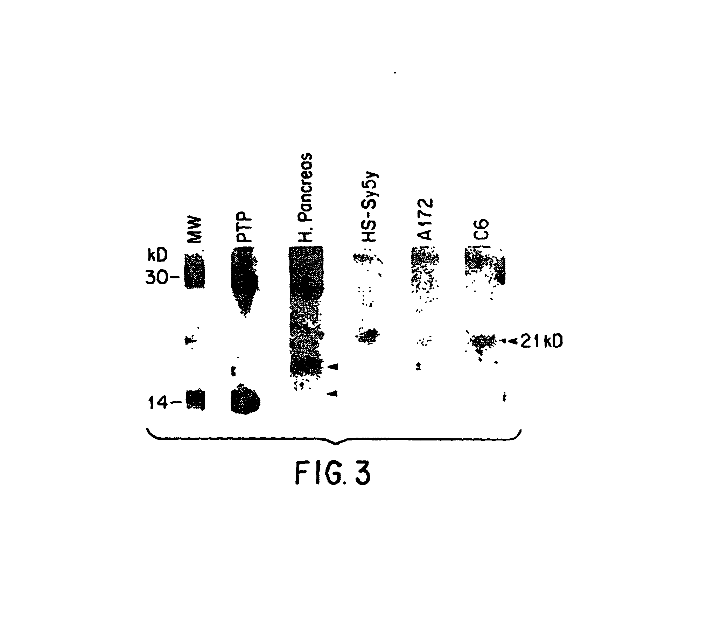

Th9-immunoreactive proteins were detected in lysates of PNET1. PNET2, SH-Sy5y, C6, and A 172 cells by four different methods: Western blot analysis, immunoprecipitation followed by Western blot analysis, metabolic labeling followed by immunoprecipitation, and SDS-PAGE combined with M-IRMA. Western blot analysis of crude cellular lysates using ¹²⁵I-labeled Th9 demonstrated ~21 kDa bands in the above cell lines (as indicated by the arrow in Figure 3), but the signal intensity was low. In contrast, in lysates of human pancreatic tissue, the expected 17 kDa uncleaved and 14 kDa cleaved forms of pancreatic thread protein were readily detected by Western blot analysis (Figure 3). Thread proteins were not detected in lysates of human hepatocellular carcinoma cell lines. The strikingly greater abundance of thread proteins in pancreatic tissue compared with neuronal and glial cell lines is consistent with a previous finding of 10⁶-fold higher levels of thread proteins in pancreas and pancreatic juice compared with brain tissue and cerebrospinal fluid (Ozturk *et al., Proc. Natl. Acad. Sci. USA 86*:419-423 (1989): de la Monte *et. al., J. Clin. Invest. 86*:1004-1013 (1990); de la Monte *et. al., J. Neurol. Sci. 113*:152-164 (1992); de la Monte *et al., Ann. Neurol. 32*:733-742 (1992)). Although one would expect that thread proteins synthesized by PNET and glial cells are secreted as is the case for PTP and NTP, thread proteins were not detected in the tissue culture medium by Western blot analysis, even after concentrating the medium four- or five-fold by lyophilization.

Th9-immunoreactive thread proteins were more readily detected in PNET and glial cell lines by first immunoprecipitating from the lysates with either Th7+Th10 or Th9, and then performing Western blot analysis using ¹²⁵I-labeled Th9 (direct) (Figure 3), or unlabeled Th9 with ¹²⁵I-labeled Protein A (indirect). Both methods demonstrated 21 kDa thread protein-related species, similar to those detected by Western blot analysis. In addition, ~ 17 kDa bands were also observed in both PNET and glial cells, but the signal was inconsistent and low-level, as determined by Western blot analysis. As negative controls, the Huh7, HepG2, and FOCUS (Lun *et al*., *In Vitro (Rockville) 20*:493-504 (1984)) human hepatocellular carcinoma cell lines were studied simultaneously under identical conditions, and Th9-immunoreactive proteins were not detected in the cellular lysates.

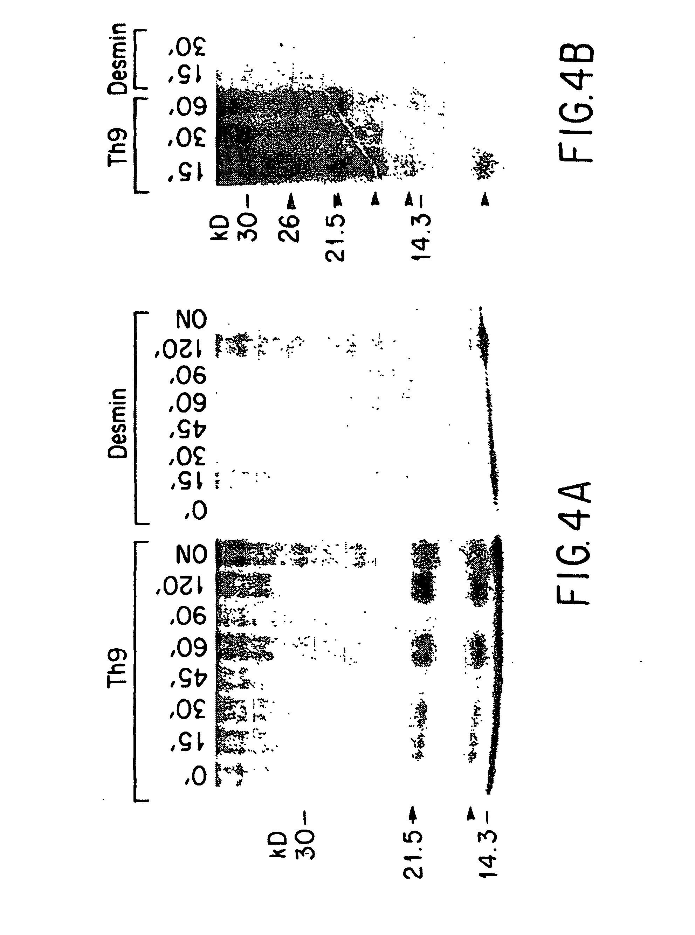

The molecular sizes of thread proteins present in PNET and glial cells were most prominently demonstrated by metabolical labeling with ³⁵S-methionine or ³⁵S-cysteine, followed by immunoprecipitation using Th9 monoclonal antibody. Monoclonal antibodies to desmin or to hepatitis B surface antigen (5C3) were used as negative controls for immunoprecipitation. In both PNET and glial cell lines, ~ 26 and ~ 21 kDa Th9-immunoreactive proteins were detected by SDS-PAGE analysis of the immunoprecipitated products (Figure 4, right panel). In PNET1 cells, the 21 kDa band appeared as a doublet (left panel); the accompanying slightly higher molecular weight species appeared to be less abundant than the dominant band at ~ 21 kDa. In addition, in both PNET and glial cell lines, there were also ~ 17 kDa Th9-immunoreactive proteins associated with bands of nearly the same intensity as the ~ 21 kDa bands. In C6 cells, there were also ~ 26 kDa, ~ 14-15 kDa and ~ 8 kDa Th9-immunoreactive proteins which were not detected in PNET cells (Figure 4, arrows).

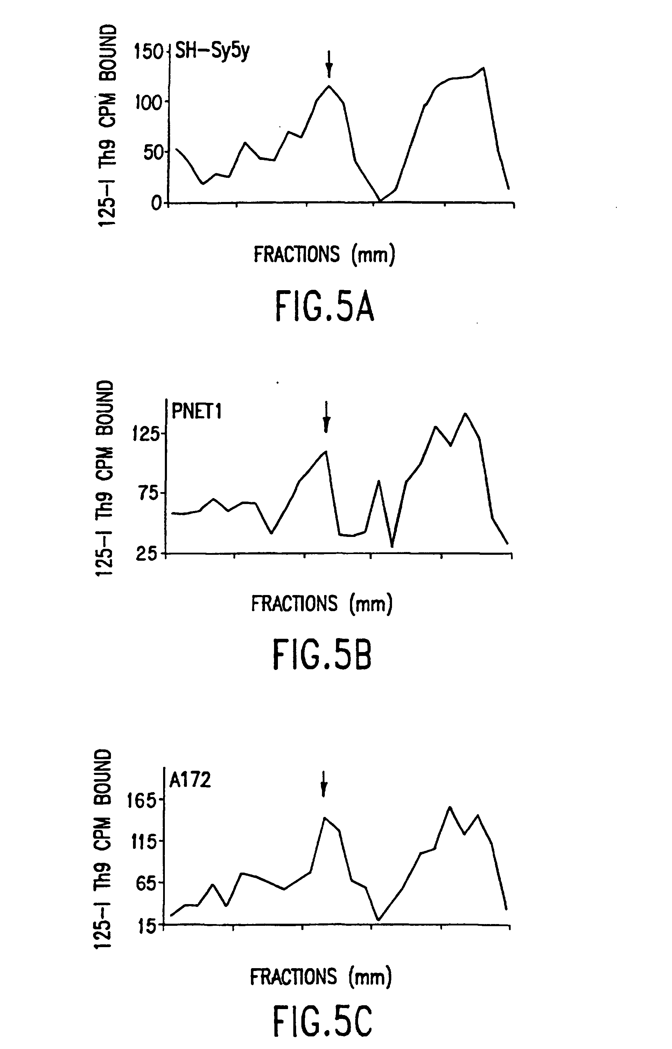

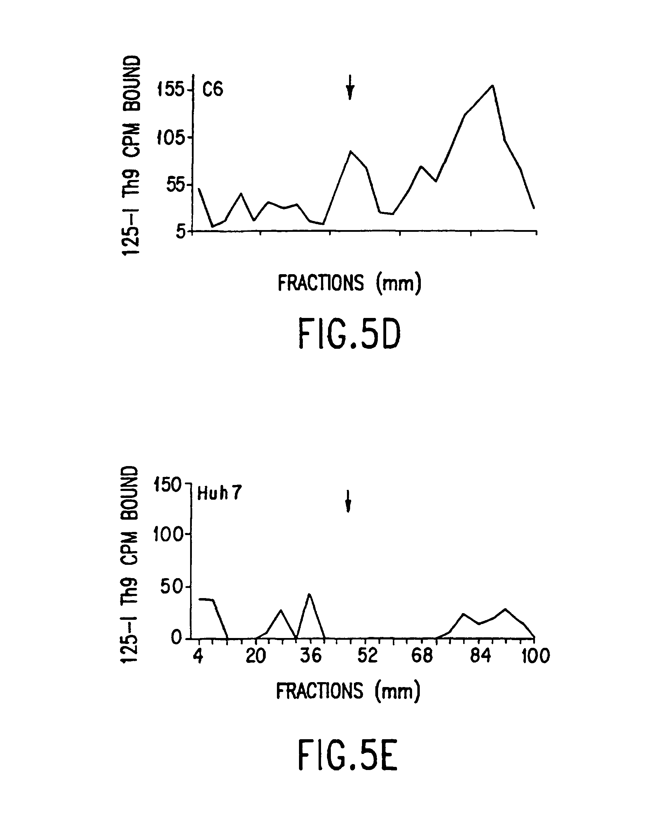

The 21 kDa and 17 kDa thread proteins in SH-Sy5y, PNET1, A172, and C6 cells, and their absence in hepatocellular carcinoma cells were also demonstrated by SDS-PAGE/M-IRMA (Figure 5). Cellular proteins fractionated by SDS-PAGE were eluted from the gels sliced at 2 mm intervals, and assayed directly for thread protein immunoreactivity by M-IRMA using Th7+Th10 as capture antibodies, and ¹²⁵I-labeled Th9 as the tracer. Despite low levels, two distinct peaks were evident in all neuroectodermal cell lines, but not in Huh7 hepatocellular carcinoma cells assayed simultaneously and in the same manner. The resolution of these gels did not permit distinction of ~ 17 kDa from ~ 14-15 kDa proteins which might have been present.

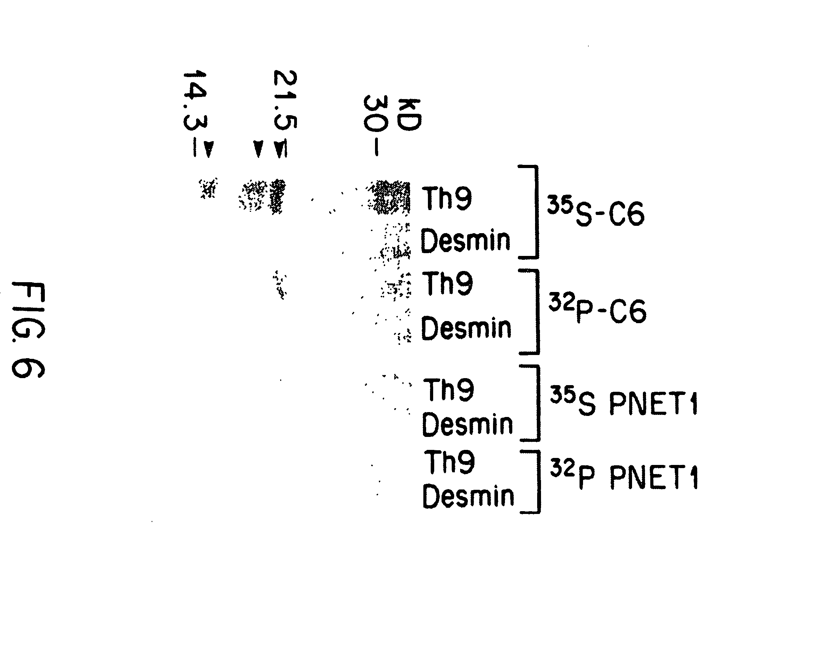

PNET1 and C6 cells were metabolically labeled with ³²P or ³⁵S-methionine, and thread proteins were immunoprecipitated from the lysates using Th9 monoclonal antibody (Figure 6). As a negative control, immunoprecipitation studies were conducted using an equal portion of the cellular lysate and monoclonal antibodies to desmin protein (Figure 6, right panel). In the cells labeled with ³⁵S methionine, Th9-immunoreactive bands were detected at ~ 26 kDa and ~ 21 kDa (upper arrows), ~ 17 kDa (lower arrows), and also at ~ 14-15 kDa (Figure 6). After ³²P labeling, only the 21 kDa band was observed by immunoprecipitation with Th9 monoclonal antibody; the other molecular weight species did not appear to be phosphorylated (Figure 6). Phosphorylated Th9-immunoreactive proteins were detected in C6 cells, but not in PNET1 cells, but this might be due to less efficient labeling since PNET1 cells grow slower than C6 cells. No bands in the 14 kDa to 26 kDa range were detected using monoclonal antibodies to desmin for immunoprecipitation (Figure 6). Carbohydrate moieties were not detected in Th9 immunoprecipitated proteins (data not shown).

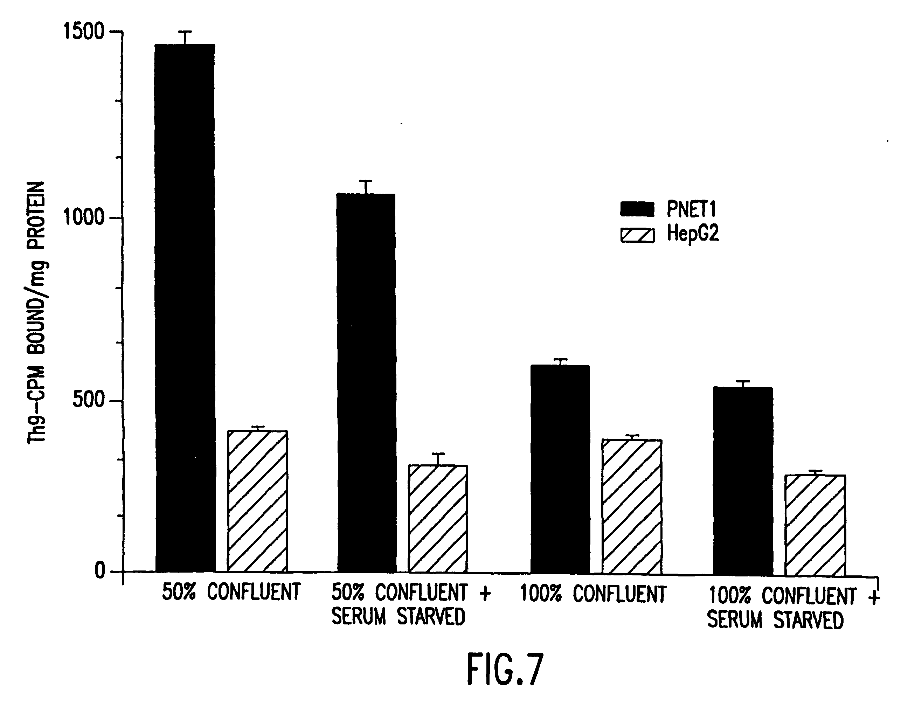

The highest concentrations of thread protein were measured in subconfluent cultures of PNET1 cells, i.e. during the log phase of growth, and the lowest concentrations in overnight serum-starved cultures (growth arrest) (Figure 7). Cultures that were 100% confluent also had lower levels of thread protein expression compared with proliferating cultures. Huh7 hepatocellular carcinoma cells (negative control) were simultaneously studied using identical culture conditions, but the levels of thread protein remained low throughout.

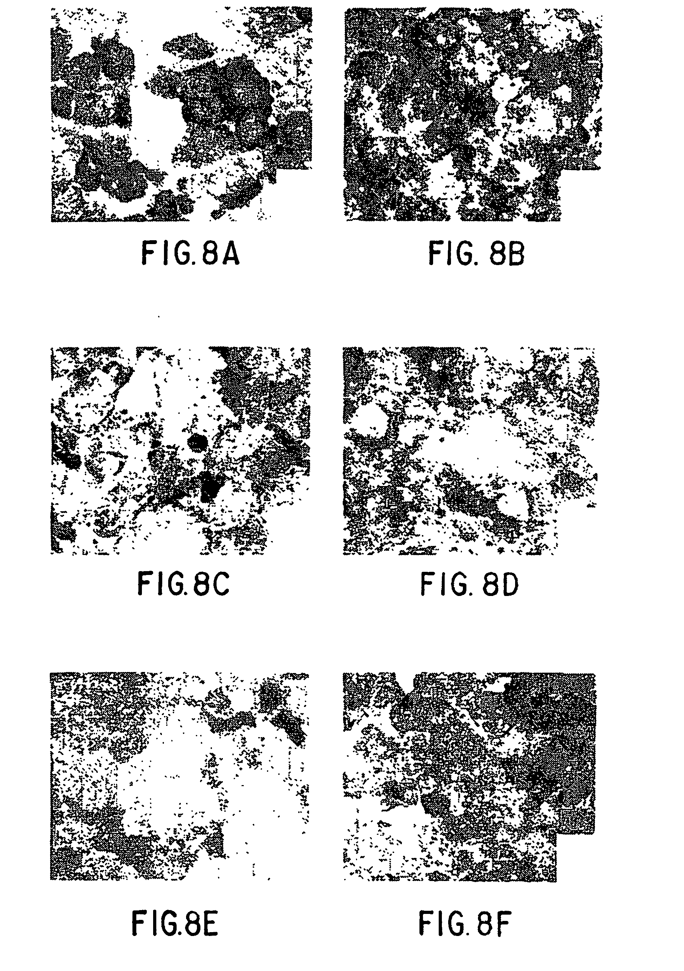

Surprisingly, there was no change in the degree of thread protein immunocytochemical staining of PNET cells cultured under these various conditions. However, the degree to which the levels of thread proteins changed by M-IRMA measurement may not have been detectable by immunocytochemistry. Nevertheless, the reduction in cellular thread protein content induced by serum starvation was associated with a change in the phenotype of the cells. When the cells achieved 100% confluence or after they had been subjected to overnight serum starvation, the cell bodies reduced in size, and they exhibited striking changes in the degree and distribution of immunoreactivity for neurofilament protein, GAP-43, and GFAP (Figure 8). In PNET cultures that were 50% confluent, the cells exhibited punctate and often a polar distribution of neurofilament and GAP-43 immunoreactivity, whereas 100% confluent and serum-starved PNET cultures exhibited diffuse perikaryal immunoreactivity for both neurofilament and GAP-43. The punctate immunoreactivity may have corresponded with distribution of neurofilament and GAP-43 in neurites. In contrast, 50% confluent PNET cultures were devoid of GFAP immunoreactivity, while 100% confluent and serum-starved cultures contained conspicuous proportions of GFAP-positive cells. Moreover, the proportion of GFAP-immunoreactive cells was greatest in 100% confluent serum-starved cultures, followed by 50% confluent serum-starved cultures, and then 100% confluent cultures with medium containing 10% fetal calf serum. Therefore, the reduction in thread protein levels measured in PNET cells subjected to overnight serum starvation may have been due to differentiation of the cells toward an astrocytic phenotype. C6 cells and other glioblastoma cell lines exhibited intense immunoreactivity with the Th9 monoclonal antibody, but the levels of thread protein measured by M-IRMA were often low, possibly due to low-level immunoreactivity with other thread protein antibodies, including Th7 and TH10 (*see* Figure 1).

### Example 4

### Cloning of Thread Proteins from Human cDNA Libraries

Human brain cDNA libraries made from 17-18 week old fetal brain (Stratagene, Inc., La Jolla, CA), 2 year-old temporal lobe neocortex (Stratagene), and end-stage Alzheimer's disease cerebral cortex (In Vitrogen; San Diego, CA) were screened using probes generated from a 416 bp DNA fragment corresponding to nucleotides 235-650 of the rat PTP cDNA. The rat PTP cDNA, designated 018, was isolated from a rat pancreatic cDNA library using synthetic 60mer DNA probes corresponding to nucleotides 45-104 and 345-404 of the published sequence (Terazono *et al*., *J. Biol. Chem. 263*:2111-2114 (1988); Watanabe *et al., J. Biol. Chem. 265*:7432-7439 (1990)). Approximately 2 x 10⁶ plaques or colonies from each library were screened with low-stringency hybridization using standard techniques (*see Sambrook et al*., *supra*). Putative clones were plaque/colony purified, and the DNA inserts were sequenced by the dideoxynucleotide chain termination method using T7 polymerase (USB Sequenase; United States Biochemical Corp.. Cleveland, OH). The sequences were compared with the Genebank database, and aligned with the nucleic acid sequences of other thread protein cDNAs.

### 1. CNS Neural Thread Protein cDNA Isolated from Human Fetal Brain Library

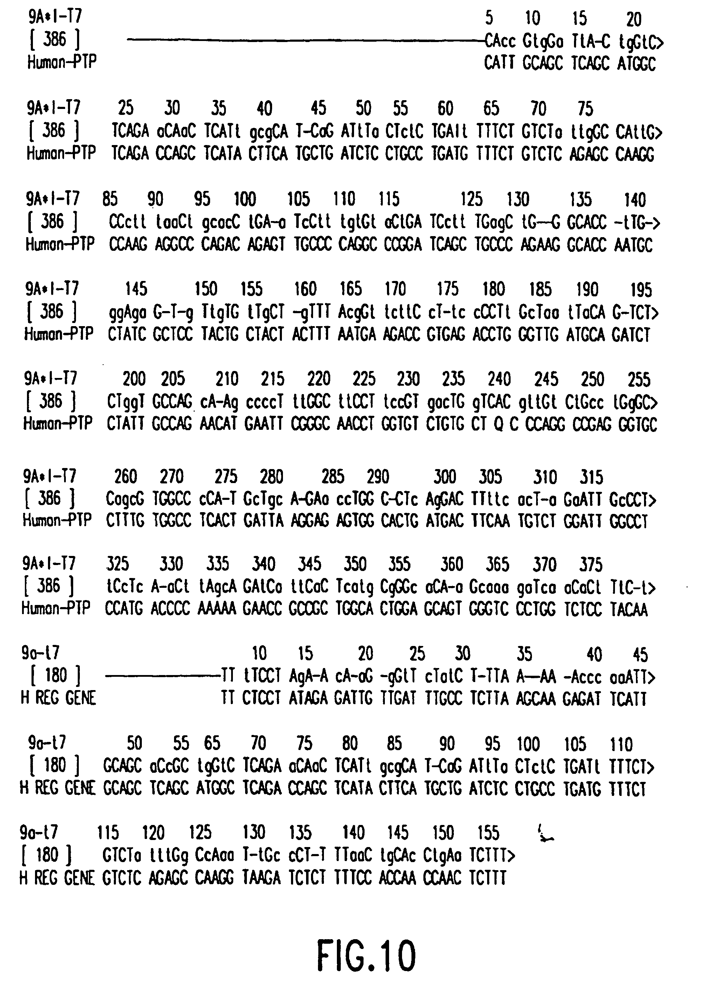

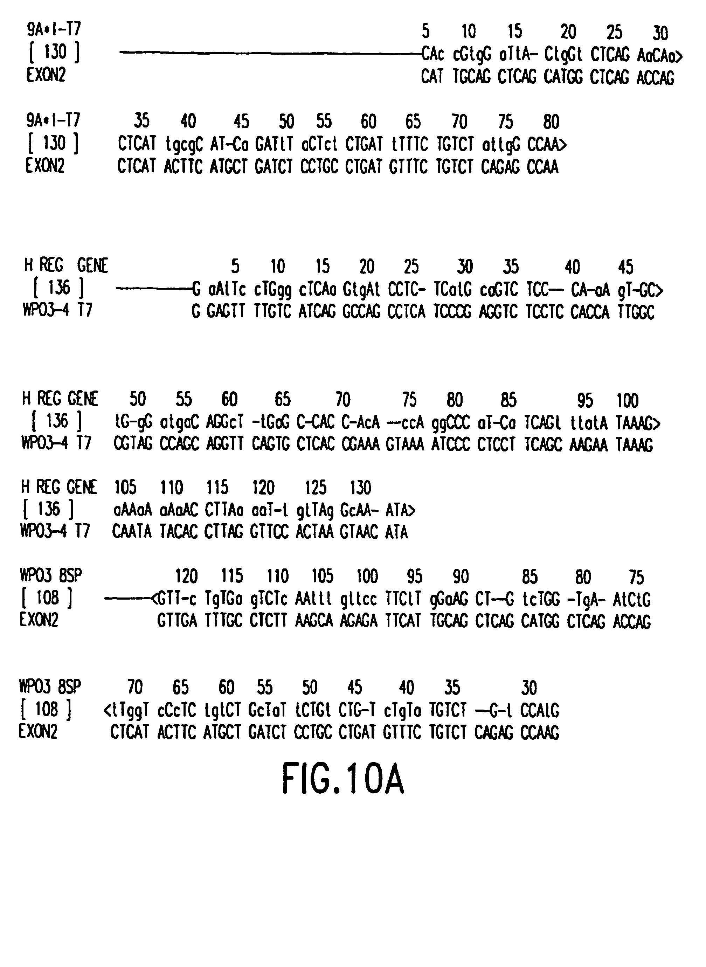

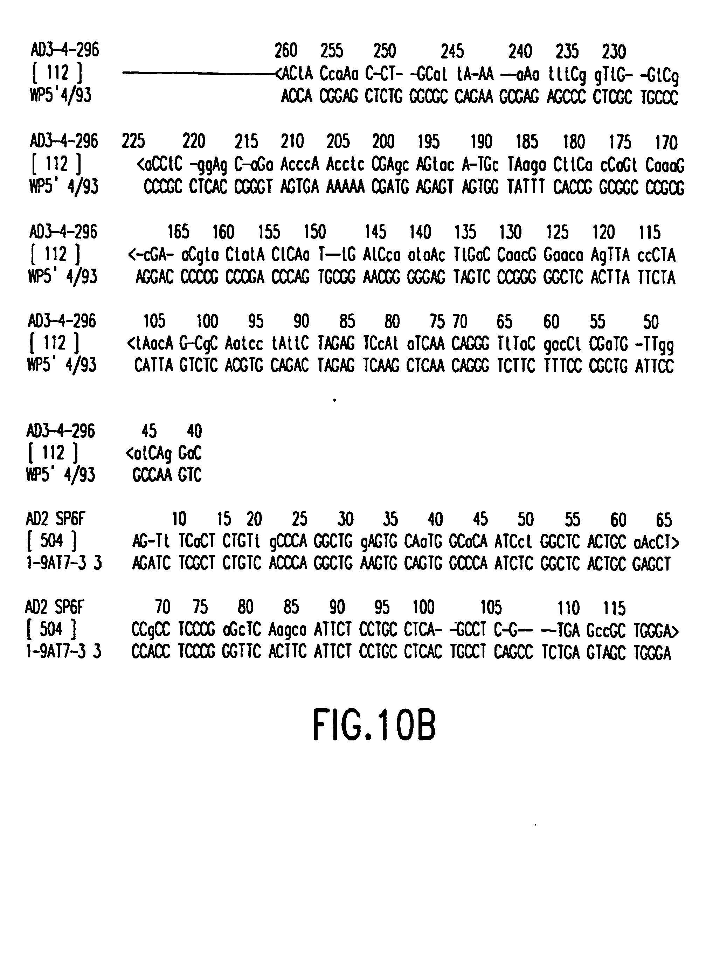

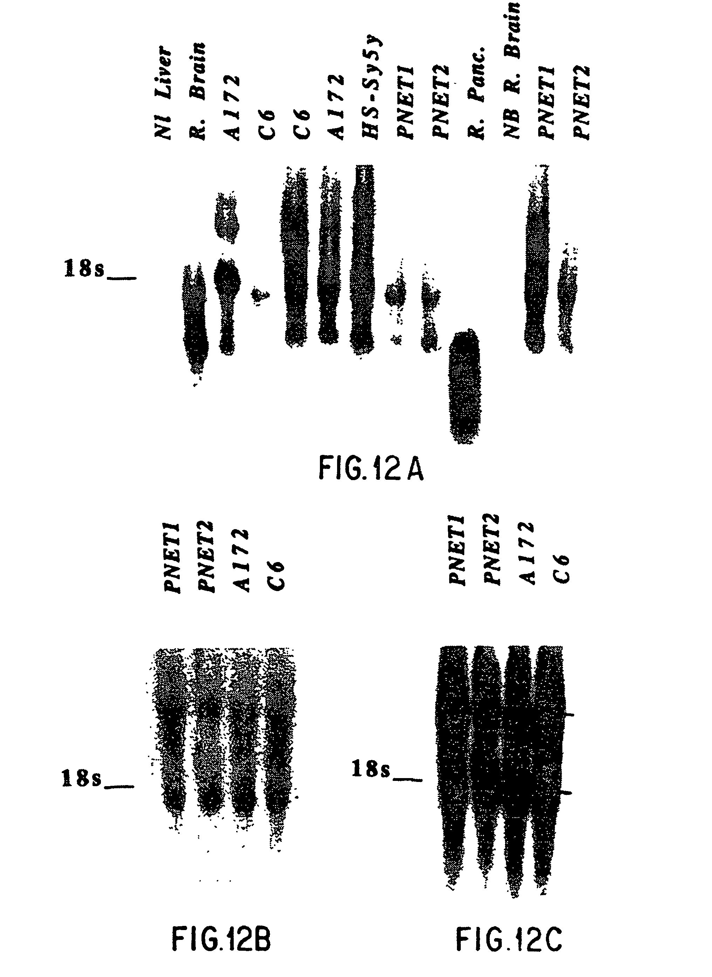

A 1.35 kilobase (kb) 1-9a CNS thread protein partial cDNA was isolated in which only a small segment corresponds to an open reading frame, and the remainder, to a 3' untranslated region (Figure 9a). The sequence of an additional 150 nucleotides was obtained from 5' anchor PCR amplification products. A second round of 5' anchor PCR amplification yielded a further upstream 600 bp product (Figure 9b). A portion of the 1-9a cDNA sequence shares significant homology with the 5' end of the human PTP cDNA and the Reg gene (Figure 10a). In addition, the initial 5' anchor PCR product has 60% homology with the 5' end of the Reg gene, and 63% homology with Exon 2 of the human Reg gene (Figure 10b). Moreover, probes generated from the 590 bp 5'-end fragment of 1-9a cDNA hybridized with human brain and pancreas mRNA (Figure 12). The 1-9a sequence is also homologous with the AD2-2 and AD3-4 cDNAs in that at one end of their completed sequences, the overlaps are substantial (Figure 10c).

### 2. CNS Neural Thread Protein cDNA Isolated from a Two-Year Old Temporal Cortex Library

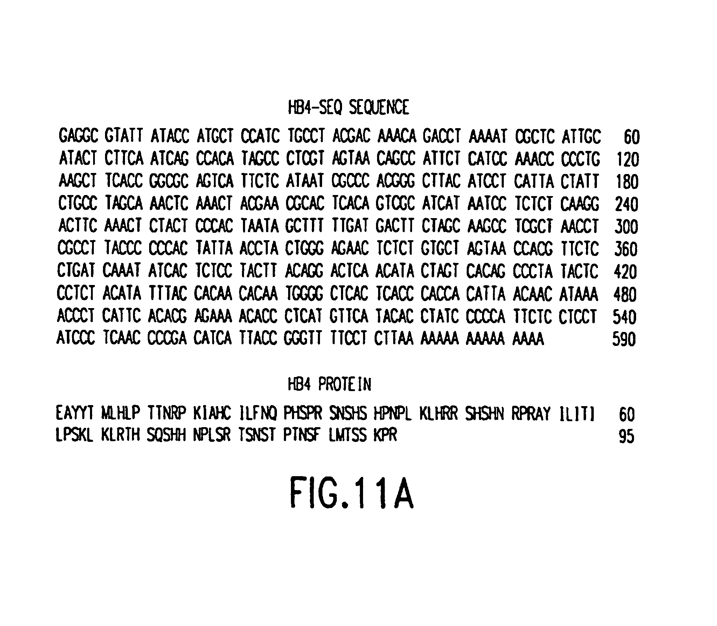

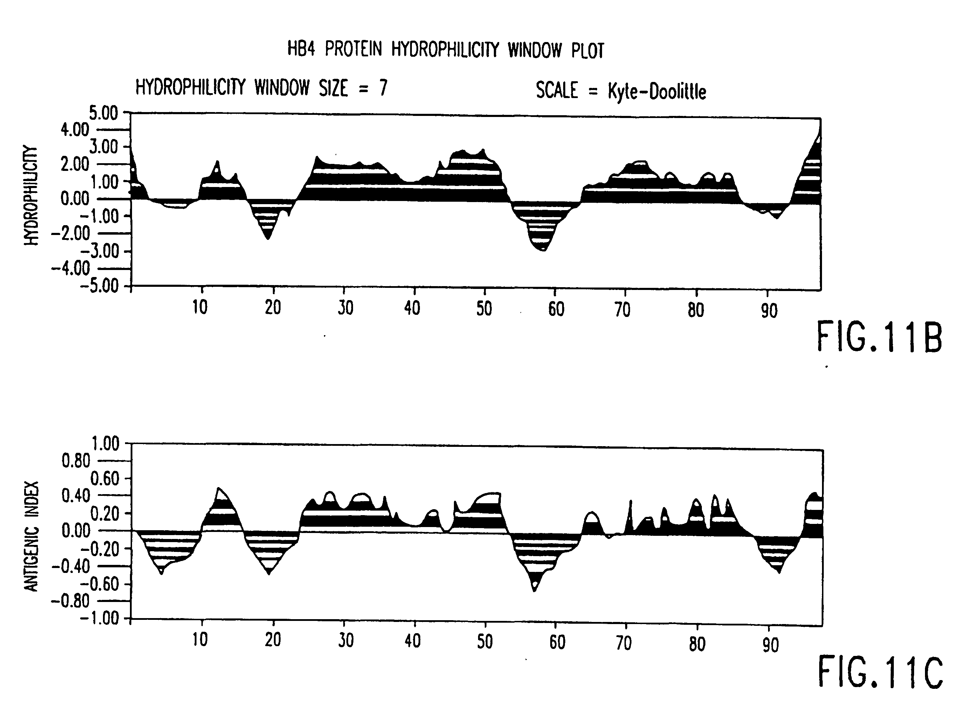

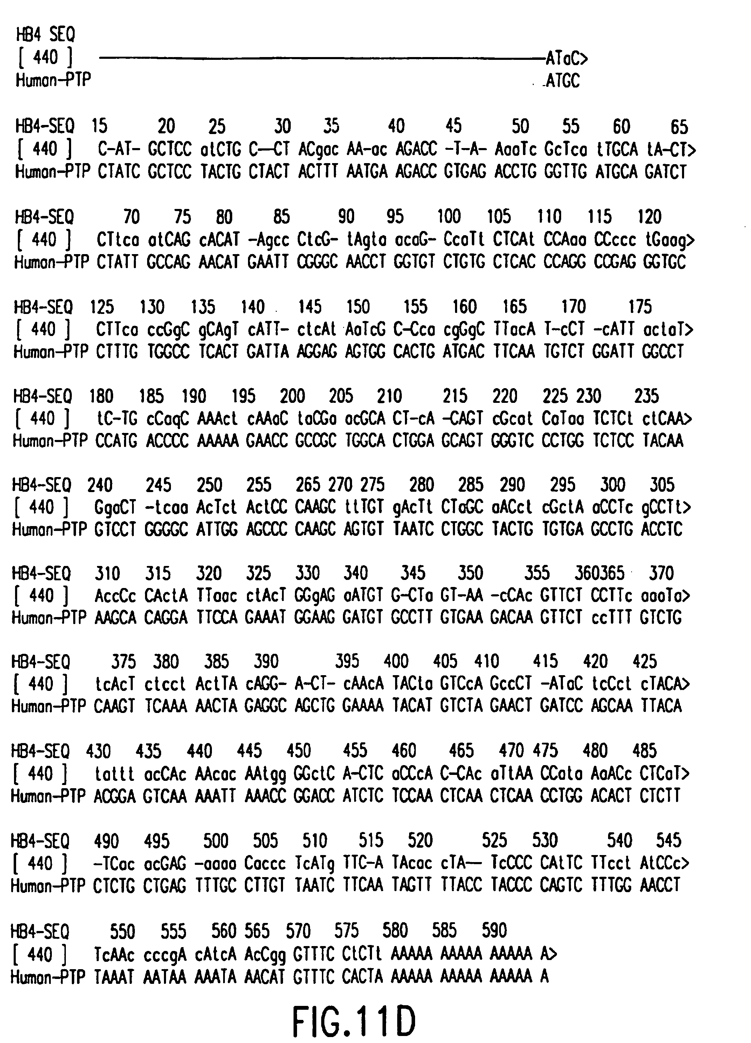

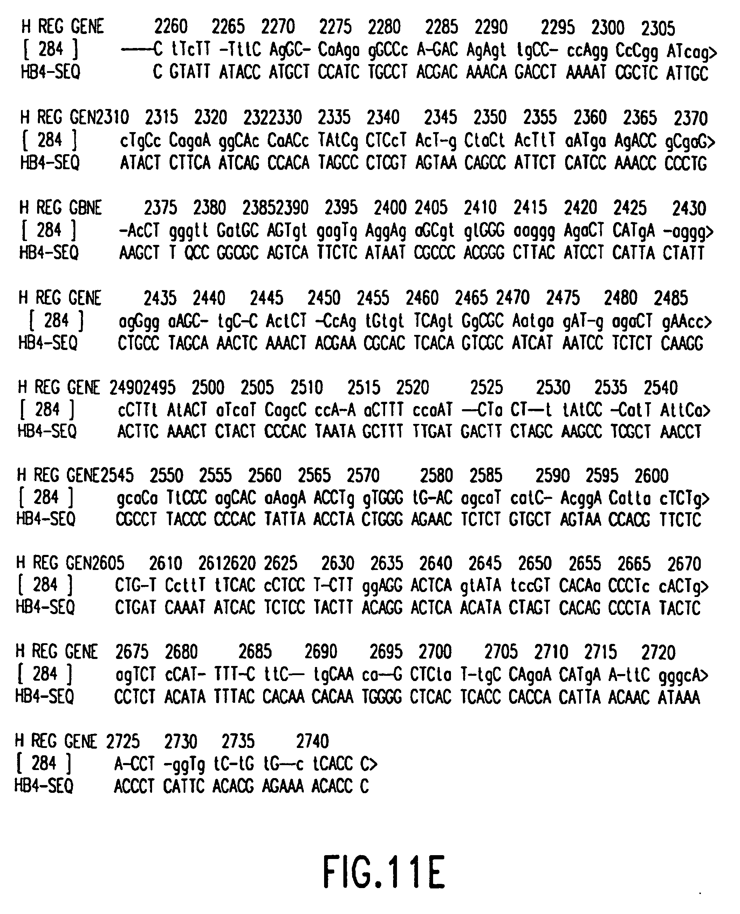

The HB4 clone is a 593 base pair partial cDNA that was isolated from a 2-year old temporal cortex library. This cDNA contains an open reading frame at its 5' end and terminates at nucleotide 275. There is a polyadenylation signal beginning at nucleotide 475, and the sequence ends with a poly-A tail (Figure 11a). The deduced amino acid sequence of the partial HB4 clone predicts a protein with a molecular weight of 10.4 kDa, and a pI of 12.1. The HB4 cDNA exhibits 50% overall nucleic acid homology with the human PTP cDNA (Figure 11b), a segment of the human Reg gene (Figure 11c).

### 3. Isolation of Neural Thread Protein cDNAs from an Alzheimer's Disease Library

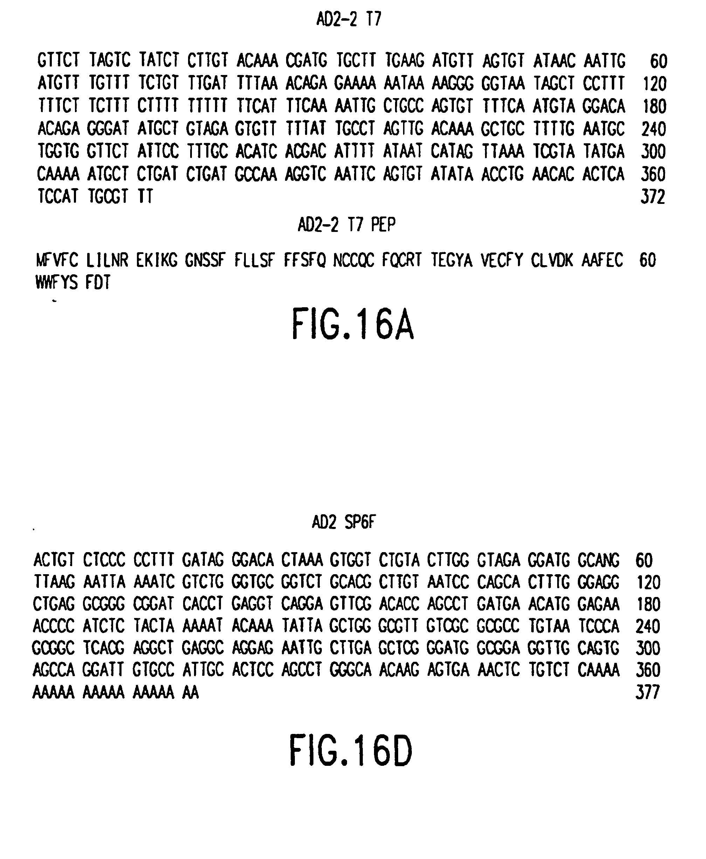

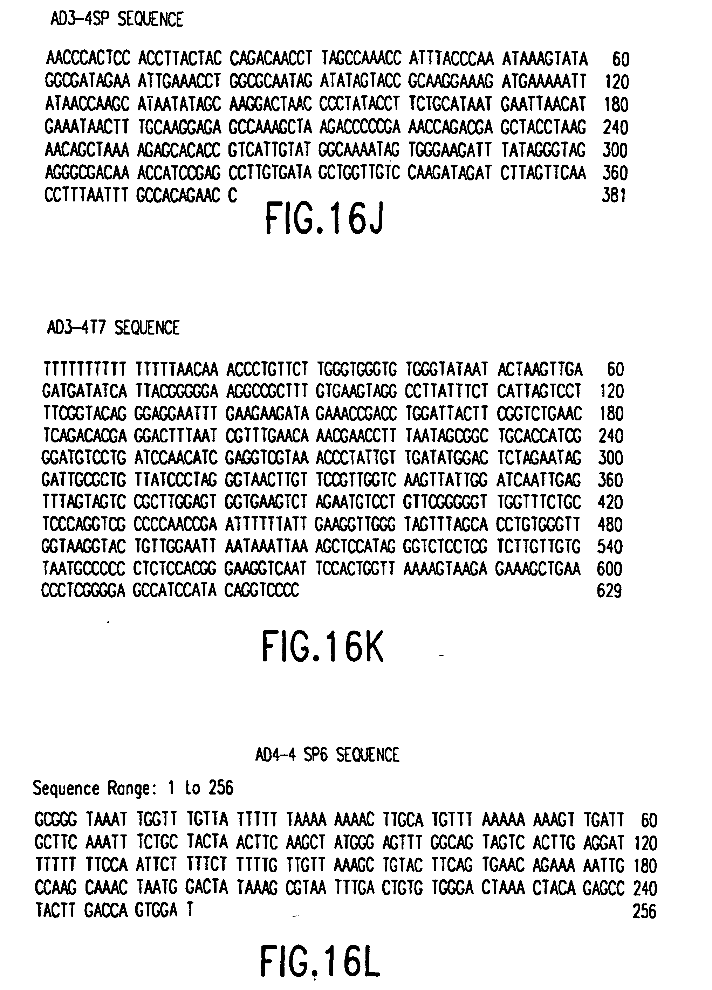

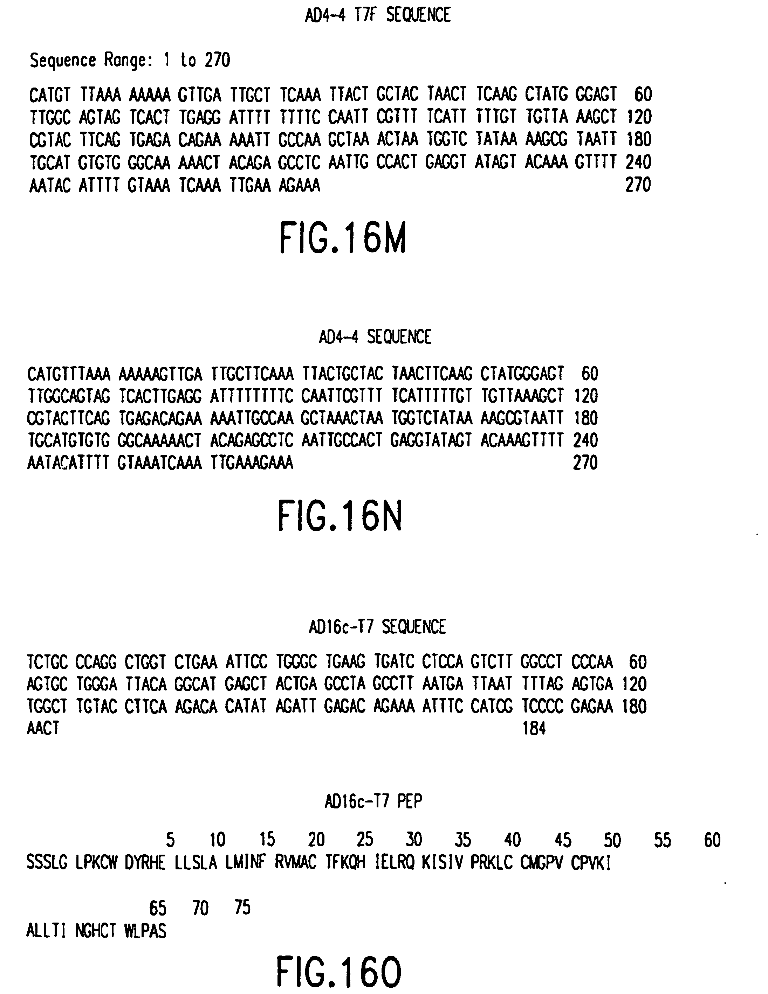

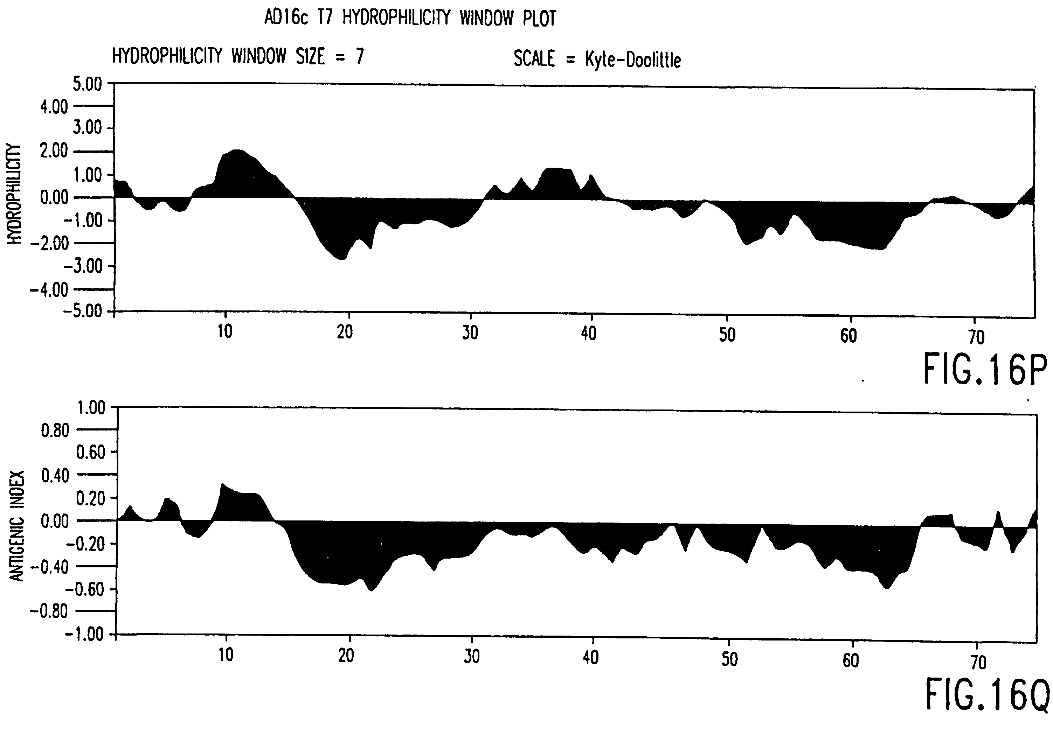

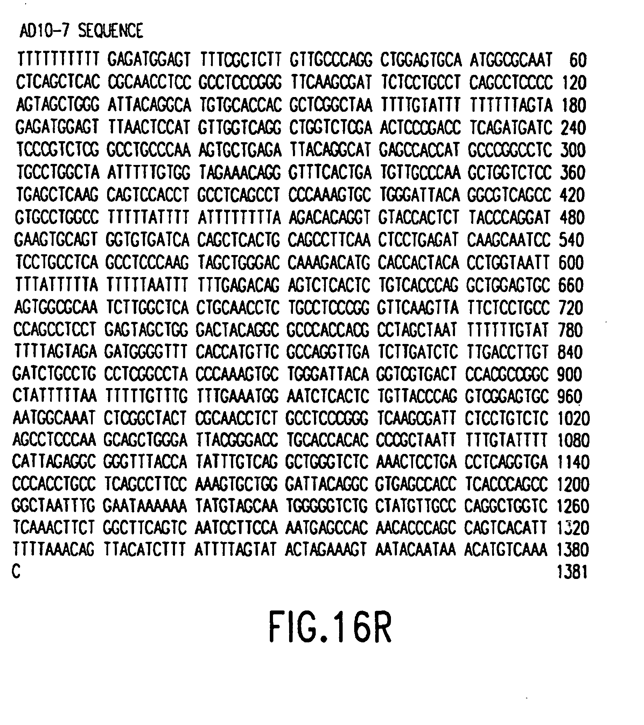

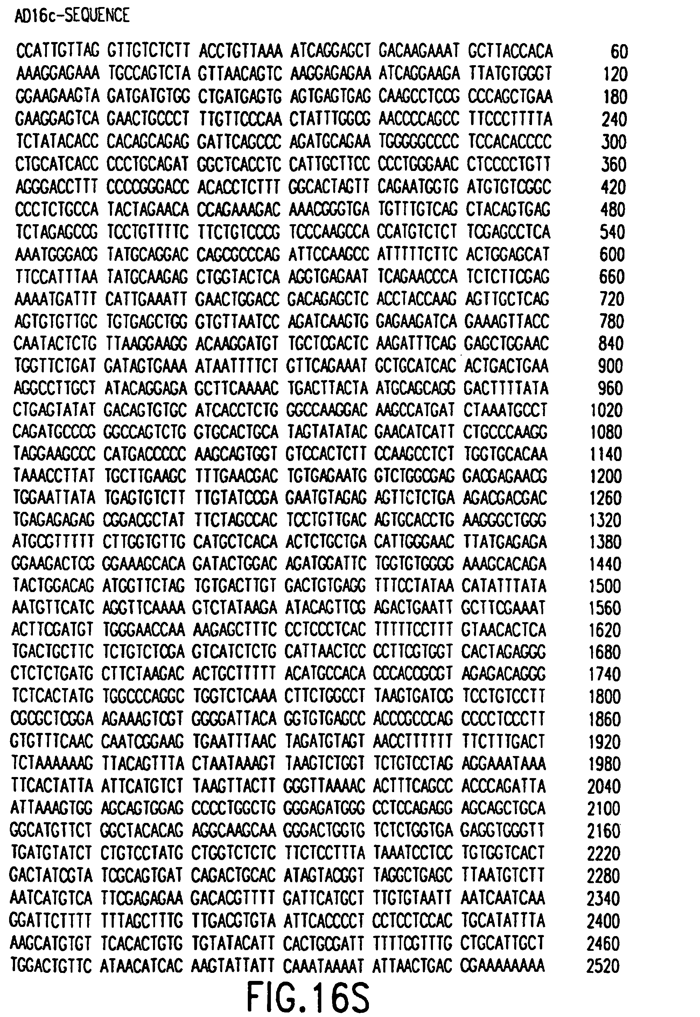

Using the O18 rat PTP cDNA probe, four related cDNAs were isolated from an AD brain library. These clones were designated: AD 2-2, AD 3-4, AD 4-4 and AD 16c (also called AD 10-7) (Figures 16a-16l).

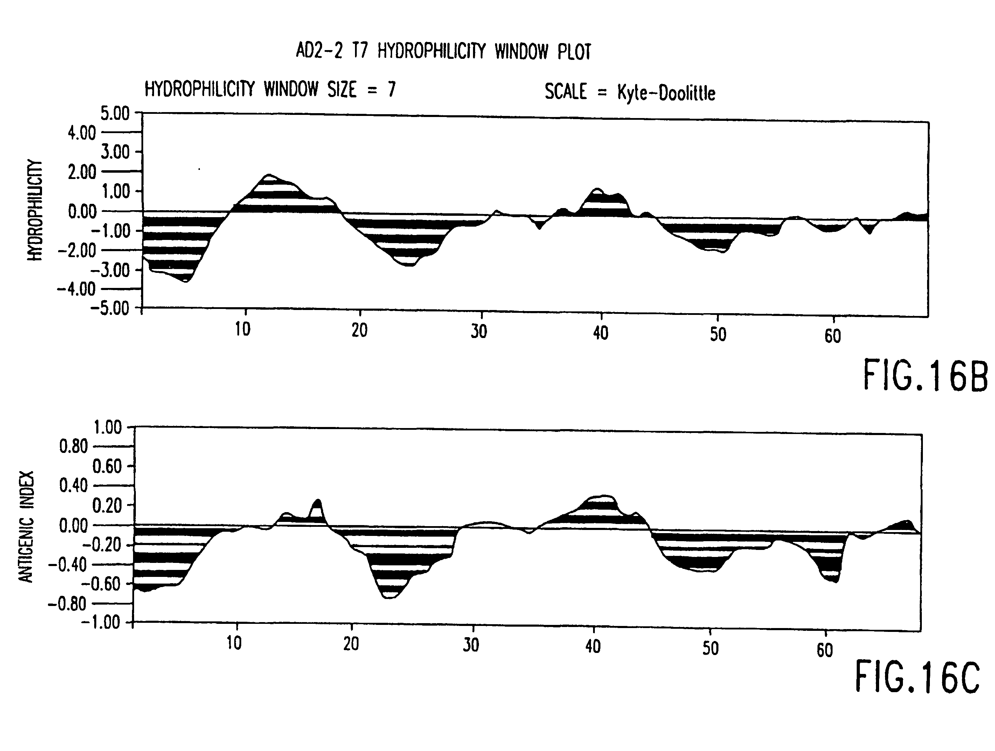

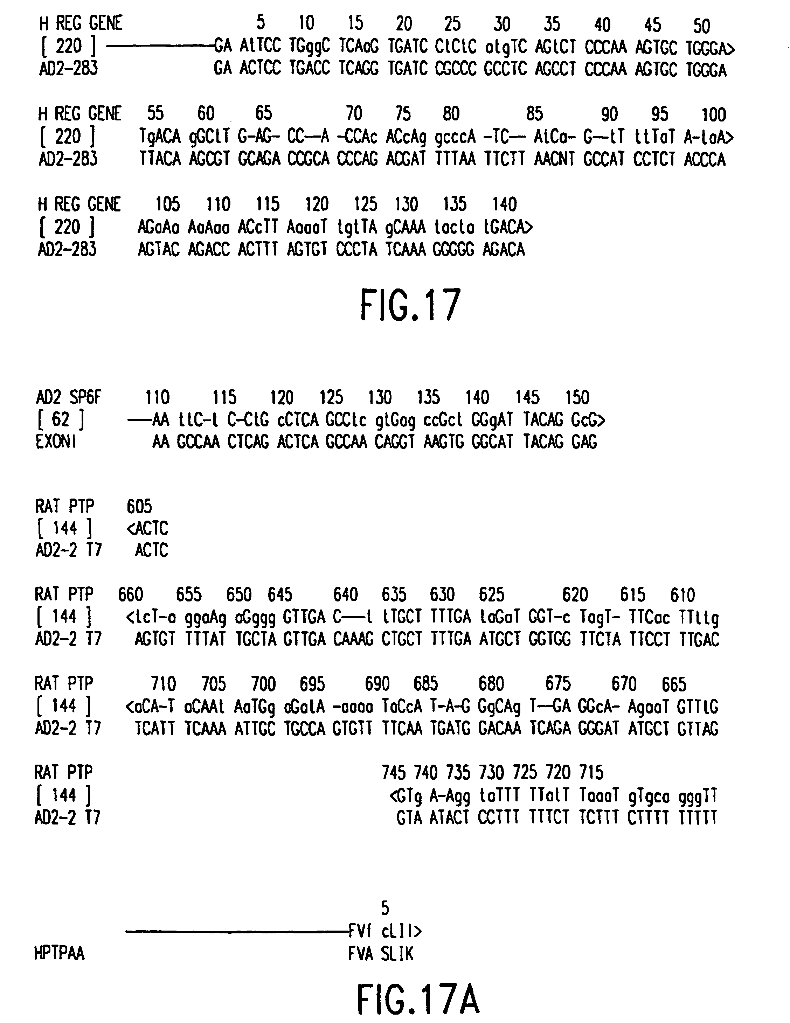

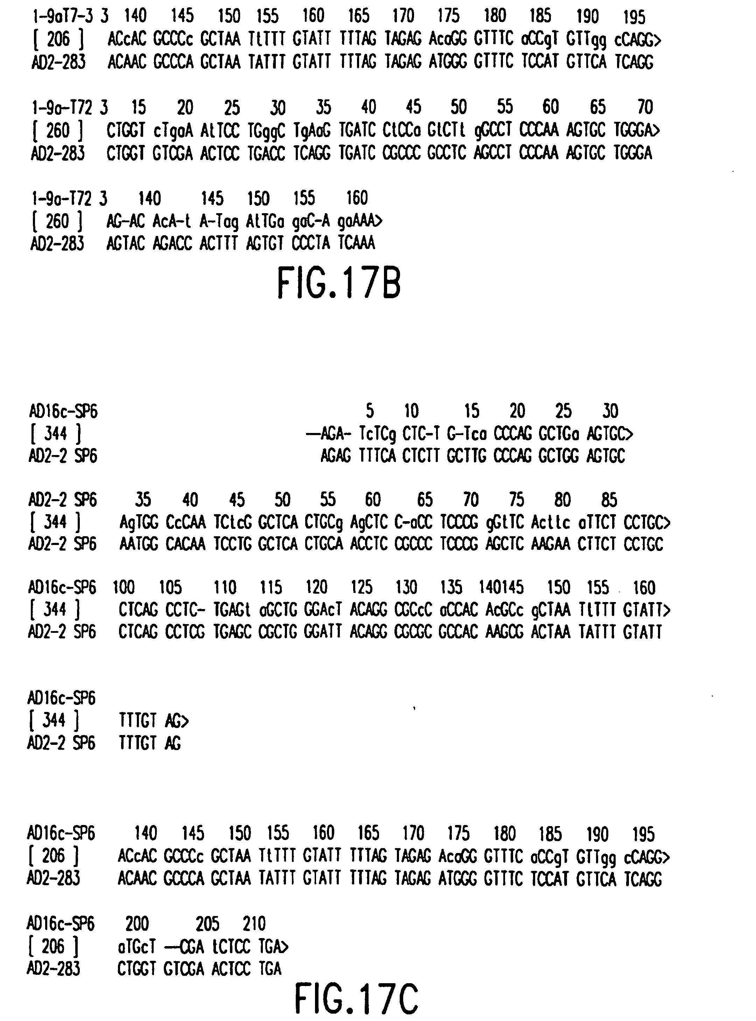

The AD 2-2 cDNA is approximately 1.2 kb and it shares significant homology with the 1-9a cDNA, AD 16c, rat PTP cDNA, and Exon I of the human Reg gene (Figure 17). The AD 2-2 probe generates a genomic Southern blot pattern similar to that obtained with the AD 3-4 probe. Figure 16b depicts the complete nucleotide sequence of the AD2-2 cDNA clone that was isolated from an AD brain library. Random primer generated probes based on this sequence hybridized with human brain and neuronal samples but not with glial cell lines of with pancreatic RNA.

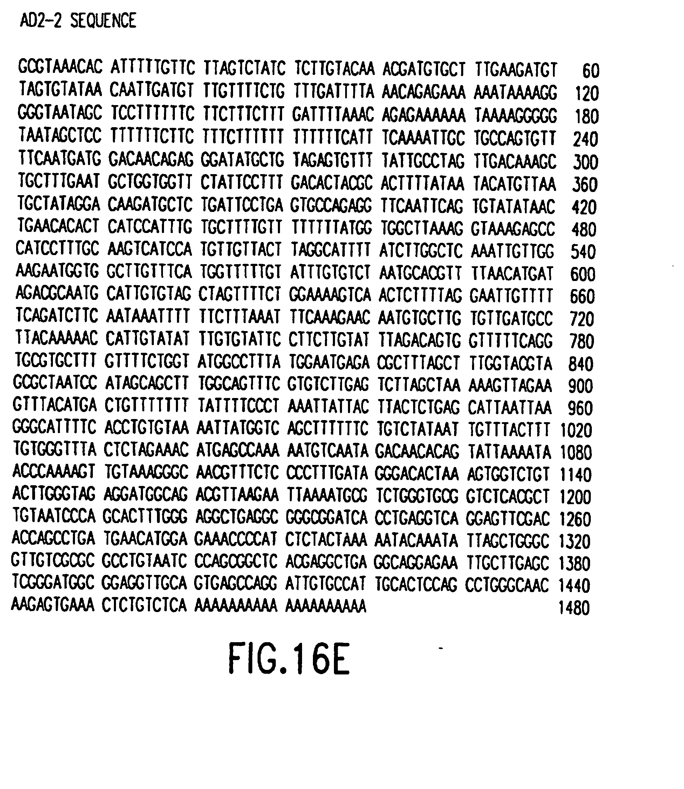

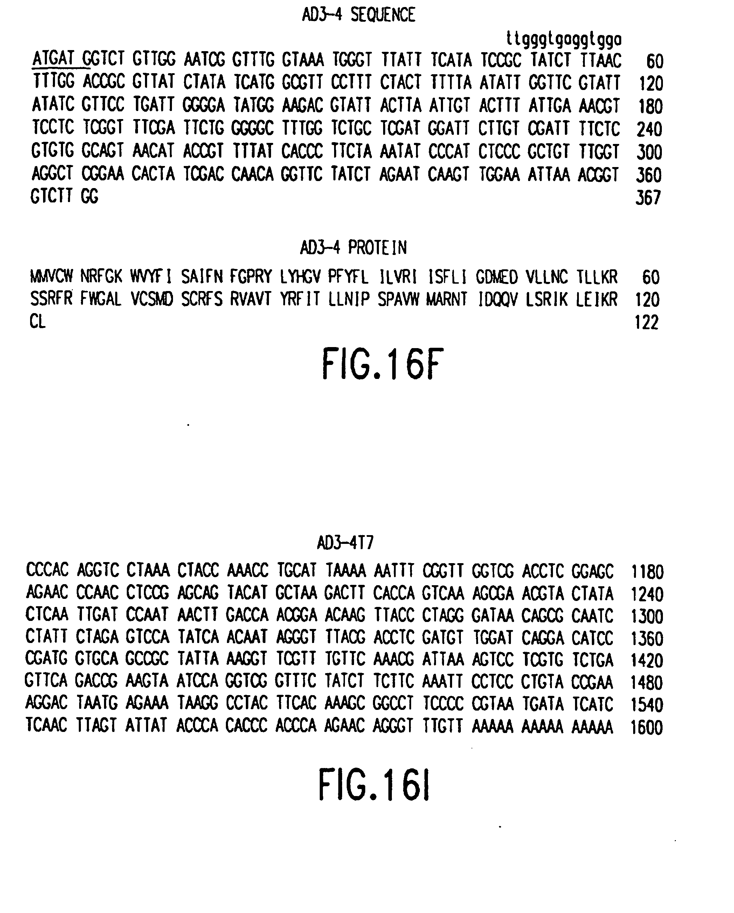

Figures 16c, 16d, 16e and 16f depict partial nucleotide sequences of the AD3-4 cDNA clones that were isolated from an AD brain library. Rnadom primer generated AD3-4 probes yielded two mRNA transcripts, 1.6 kB and 3.4 kB. These mRNA species are over-expressed in AD brains, with an average of two-fold elevation compared with aged matched controls (N=8).

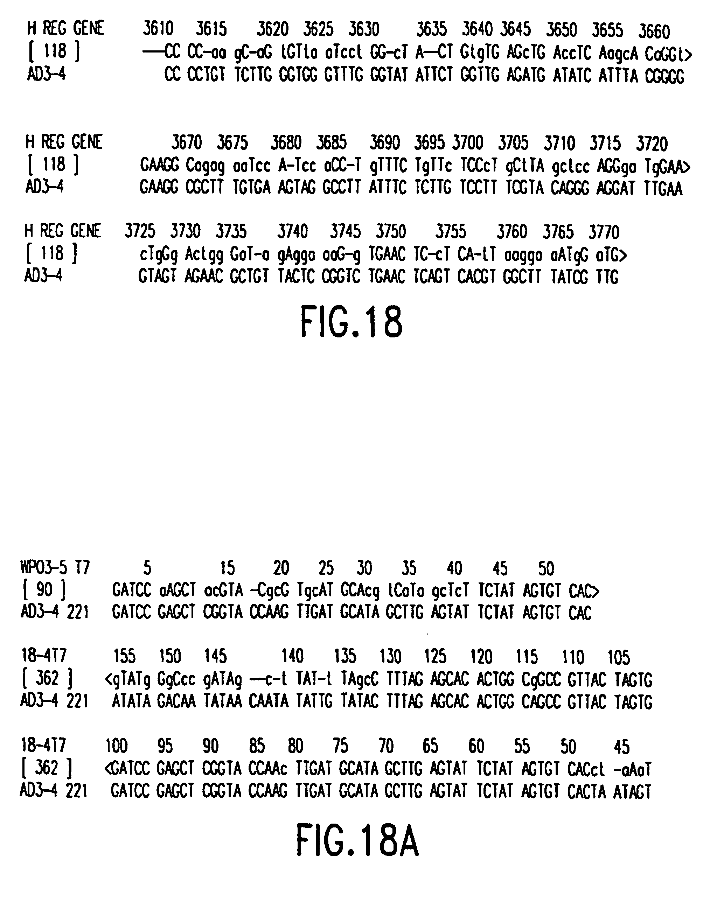

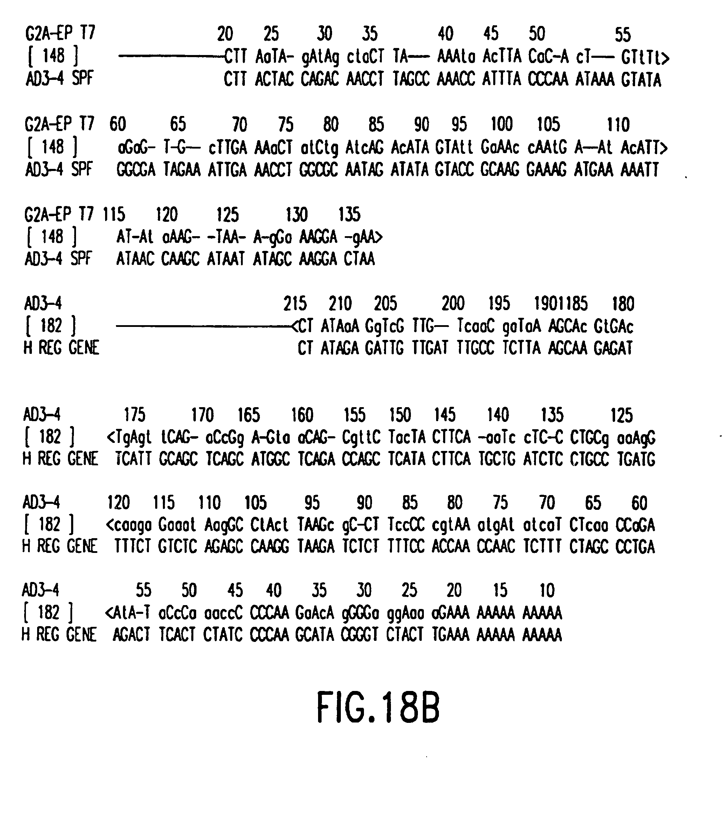

The AD 3-4 cDNA 1.6 kb clone is identical to another clone isolated at the same time (AD 5-3) (Figure 18a). The AD 3-4/AD 5-3 cDNA exhibits substantial homology with the 1-9a 5' anchor PCR products (Figure 18b), as well as with the human Reg gene and the Gen2a-EP genomic clone (Figure 18c). Southern blot analysis of human genomic DNA with the AD 3-4 probe revealed a pattern similar to that obtained with the AD 2-2 probe.

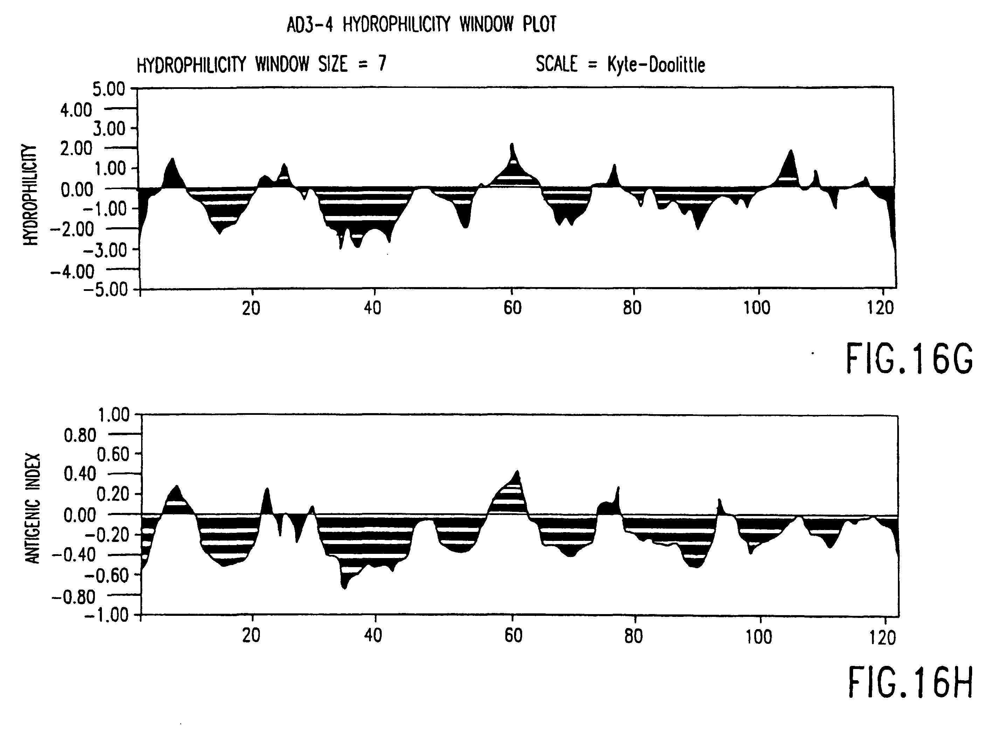

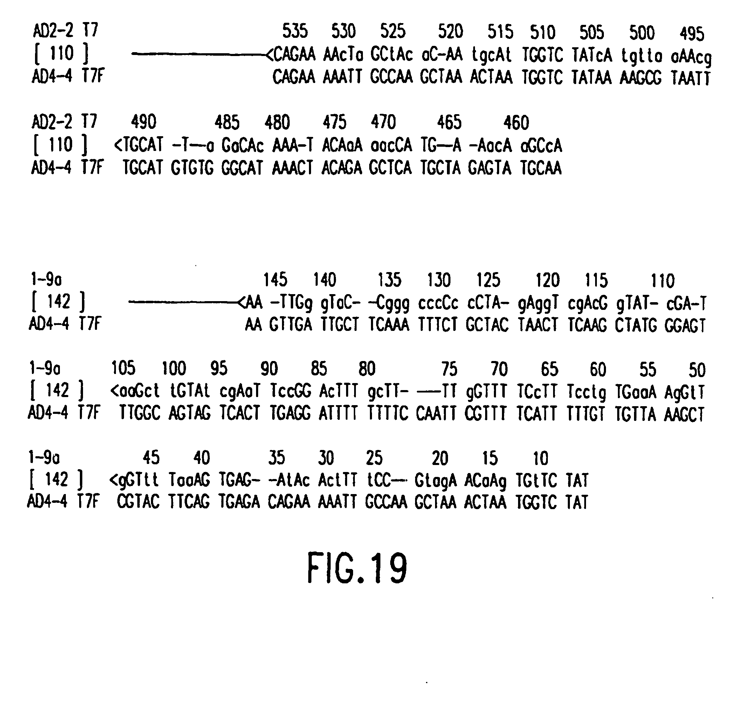

Figures 16g and 16h depict the partial nucleotide sequence of AD 4-4 which is a 0.8 kb partial cDNA clone which is identical to another cDNA isolated at the same time (AD 3-5). This AD 4-4 clone shares substantial sequence homology with AD 2-2 and 1-9a cDNAs (Figure 19). Figure 16i depicts the complete nucleotide sequence of a partial cDNA clone isolated from an AD brain library. This cDNA hybridized with brain and neuronal cell line mRNA, yielding a single 1.4 kB transcript.

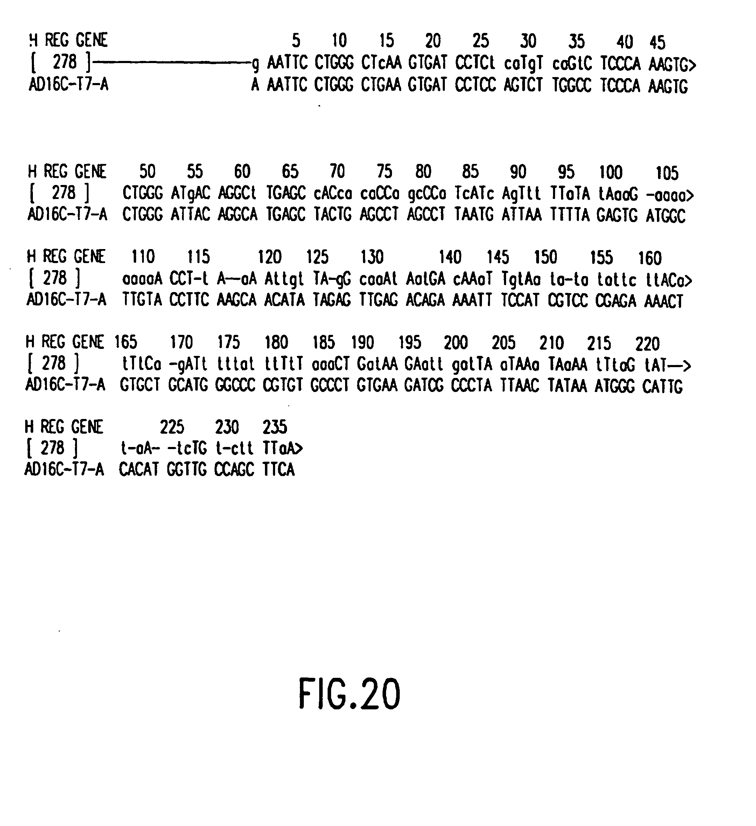

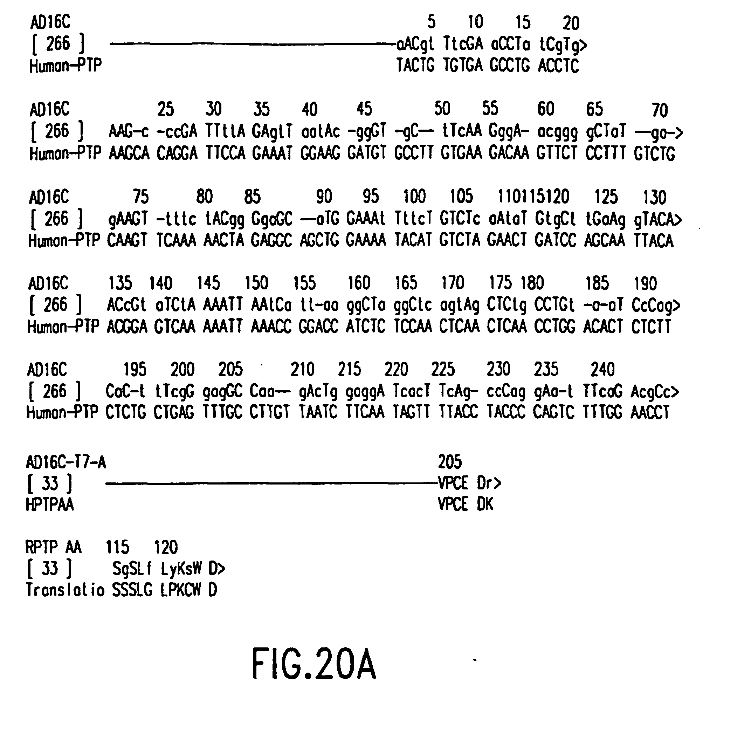

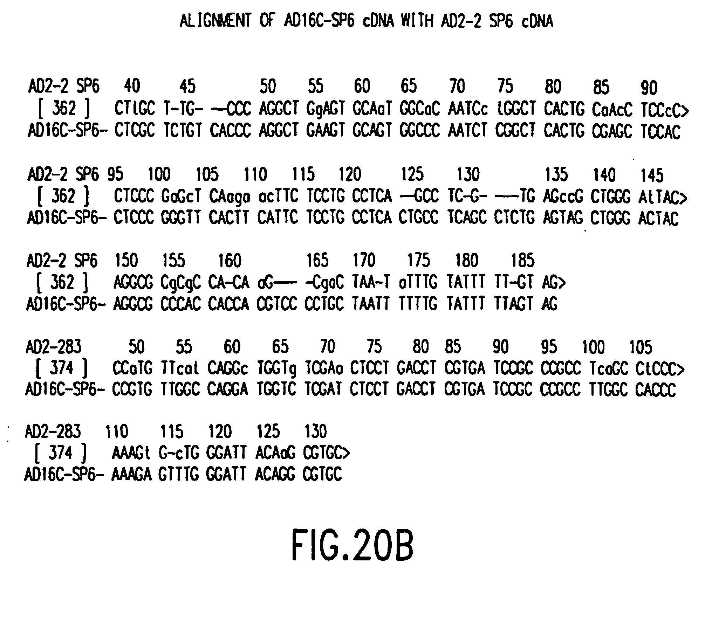

Figure 16j depicts the nucleotide sequence of the 0.5 kb partial cDNA clone AD 16c (also called AD 10-7) that is 72% homologous with AD 2-2, and also aligns with human PTP and the human Reg gene sequences (Figures 20a and b).

Figure 16k depicts the complete nucleotide sequence of the AD10-7 clone that was isolated from an AD brain library. Hybridization of Northern blots using either antisense cRNA probes or random primer generated DNA probes detected 2.6, 1.9. 1.4 and 0.9 kB mRNA transcripts in neuronal cells. Neuronal cell lines expressed only the two largest transcripts, while mature adult human brains expressed predominantly the two smallest transcripts, and either very low or nondetectable levels of the 2.6 kB and 1.9 kB transcripts. Using an AD 10-7 probe, Northern blot analysis of RNA obtained from human liver, ovary, fallopian tube, colon, stomach, spleen, rectum, thyroid, 12 week placenta and kidney was negative.

Figure 16l depicts the complete nucleotide sequence of the AD16c cDNA clone that was isolated from an AD brain library. Hybridization of Northern blots using random primer generated DNA probes yielded the same results as obtained with the AD10-7 cDNA clone. The AD16c clone shares a 650 bp segment of near identity with AD10-7. In addition, elevated levels of AD16c mRNA were detected in AD brains compared with aged control brains by Northern blot analysis.

### Example 5

### Analysis of Brain Thread Protein Gene Expression

Thread protein mRNA expression was examined in the following neuroectodermal tumor derived cell lines: central nervous system primitive neuroectodermal tumor cells designated PNET1 and PNET2; HGL-16 and HGL-17 human glioblastoma cells; A172 human glioma cells; C6 rat glioma cells; and SH-Sy5y neuroblastoma cells. In addition, human brain tissue from patients with Alzheimer's disease or no neurological disease (aged controls), and embryonic and postnatally developing rat brain were assayed for thread protein mRNA expression. RNA extracted from human and rat pancreas served as positive controls.

RNA was extracted in 5 M guanidinium isothiocyanate, and then isolated by centrifugation through a cesium chloride step gradient (*see* Sambrook *et al*., *supra*). RNA was quantified by measuring the absorbance at 260 nm and 280 nm. The thread protein mRNA transcript sizes were assessed by northern blot analysis, and the levels of expression were evaluated by RNA dot blot hybridization. Northern blot analysis was performed by electrophoresing samples containing 15 µg of total cellular RNA through 1% agarose-formaldehyde gels. The RNA was transferred to nylon membrane, cross-linked with ultraviolet light, and hybridized with probes generated from a 600 bp fragment of the 1-9A cDNA clone. The fragment used for hybridization studies contained the regions most homologous with the human PTP cDNA. The probes were labeled with [³²P] α-dCTP by the random primer method (Amersham Corporation; Arlington Heights, IL). The blots were hybridized overnight at 42°C with 2 x 10⁶ dpm /ml of probe in buffer containing 50% formamide, 5x SSPE, 10x Denhardt's (100x Denhardt's is 2% Ficoll, 2% bovine serum albumin, 2% polyvinylpyrollidine), 0.5% SDS (sodium dodecyl sulfate), and 100 µg/ml of sheared denatured salmon sperm DNA. The membranes were washed in SSPE containing 0.25% SDS using standard methods. Autoradiograms were generated by exposing the membranes to Kodak XAR film at -80°C. The membranes were subsequently stripped of probe and then rehybridized with a synthetic 30mer corresponding to 18s RNA to evaluate sample loading.

Northern analysis of total cellular RNA using probes made from the 1-9a cDNA disclosed two dominant transcripts in central nervous system (CNS) tumor cell lines: one transcript was 1.6 kb, and the other was 0.9 kb (Figure 12A). In addition, in the SH-Sy5y neuroblastoma and PNETl cell lines, a larger 4.2 kb mRNA transcript was also detected. The 4.2 kb transcript may represent preprocessed mRNA. The same size transcripts were detected in adult (R. Brain) and newborn (NB) rat, but the 0.9 kb transcript was more abundant in the adult brain whereas the 1.6 kb transcript was more abundant in the newborn rat brain. In rat pancreas (R. Panc.), only a 0.9 kB transcript was detected, corresponding to the size of rat PTP mRNA (Terazono *et al*., *J. Biol*. *Chem. 263*:2111-2114 (1988); Watanabe *et al*., *J. Biol*. *Chem. 265*:7432-7439 (1990)). mRNA transcripts were not detected in normal liver (NI Liver). Using a probe generated from the 3' region of the 1-9a cDNA, the 1.6 kb, but not the 0.9 kb transcript was revealed (Figure 12b). Using a 30-mer probe corresponding to the most 5'-end of the 1-9a cDNA, the higher molecular weight mRNA transcripts were detected (Figure 12c). The 0.9 kb transcript was also evident with longer exposure of the blot.

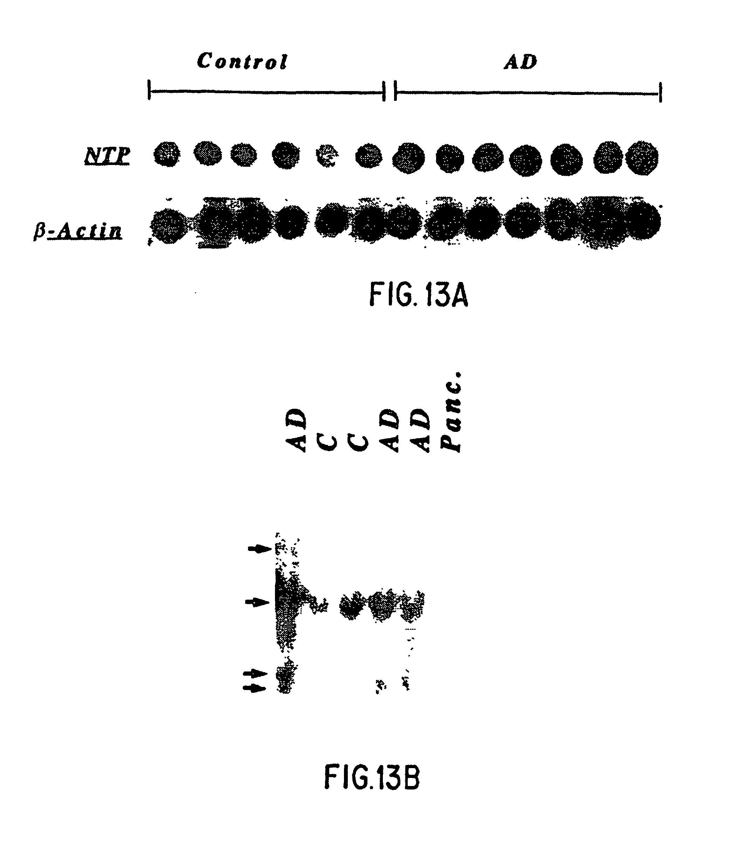

Northern analysis of human brain RNA disclosed a dominant 1.6 kb transcript, but also two and sometimes three smaller transcripts of 1.2 kb, 0.9 kb, and 0.8 kb (Figure 13, bottom). In contrast to the findings in cell lines, the 4.2 kb mRNA transcript was seldom observed in adult human brain. Hybridization with human pancreas disclosed a 0.8 kb transcript, corresponding with the size of PTP mRNA. The transcripts detected in human brain and pancreas using 1-9a probes were identical in size to the transcripts observed using PTP cDNA probes.

Dot blot RNA hybridization to 5 µg of total RNA using the 600 bp fragment of the 1-9a cDNA (NTP) demonstrated higher levels of expression in AD, compared with aged control brains (Figure 13, top). Rehybridization of the same membrane with a cDNA corresponding to β-actin demonstrated similar loading of RNA in each dot. The observation of elevated levels of 1-9a-related mRNA in AD brain tissue is similar to that reported previously using 60mer probes corresponding to human PTP cDNA (de la Monte *et. al., J. Clin. Invest. 86*:1004-1013 (1990)). The differences between AD and control brains appeared to be due to differences in the levels of the 1.6 kb, 0.9 kb and 0.8 kb transcripts, as shown in Figure 13.

The AD-NTP 3-4 cDNA, isolated from the AD library, hybridizes with RNA from neuronal-derived neuroectodermal tumor cell lines, and human brain tissue. In the cell lines, 1.6 kb and 0.9 kb transcripts as observed with the 1-9a probe were detected (Figure 21c). However, in human brain, ~4 kb, 1.6 kb, and 0.9 kb transcripts were detected, and the levels of expression for all three transcripts were higher in AD compared with aged control brains (Figure 21d).

AD 4-4 cDNA probe hybridized only with a 0.9 kb transcript, and only in neuronal cell lines. ***Example 6***

### Direct Cloning and Sequencing of Thread Protein cDNAs from Neuroectodermal Tumor Cell Lines and Alzheimer's Disease Brain

Thread protein cDNAs were cloned directly from PNET1, PNET2, SH-Sy5y, and A172 cells, and from Alzheimer's disease and aged control brain RNA using the 3'- and 5'-RACE methods (Frohman *et al*., *Proc. Natl*. *Acad*. *Sci. USA 85*:8998 (1988); Ohara *et al*., *Proc. Natl. Acad. Sci. USA 86*:5673 (1989); Loh *et al*., *Science 243:217* (1989)). Briefly, RNA was reverse transcribed using oligo-dT primers. For the 5'-RACE reaction, the cDNAs were amplified by polymerase chain reaction (PCR) using a specific 17-mer corresponding to a 5'-region of the 1-9a sequence, and a 17 dT primer. The resulting PCR products were subjected to another round of amplification using another internal but overlapping 5'-end primer, and a specific 3'-17-mer corresponding to a 3' region of the 1-9a sequence. For the 3'-RACE reactions, the cDNAs were first tailed with dCTP using terminal deoxynucleotide transferase, and then they were amplified using a specific 17-mer corresponding to nucleotides 781-797 of the 1-9a clone and dG (17mer). A second nested PCR amplification was performed using a specific 17mer corresponding to nucleotides 766-792 at the 3' end, and dGTP (17mer) for the 5' end. The PCR products were subjected to Southern blot analysis using probes generated from an internal DNA fragment of the 1-9a cDNA clone, and from the 018 rat PTP cDNA clone. The PCR products were gel purified and ligated into pAmpl vectors using uracil deoxytransferase. The subcloned DNA inserts were sequenced by the dideoxynucleotide chain termination method using T7 DNA polymerase.

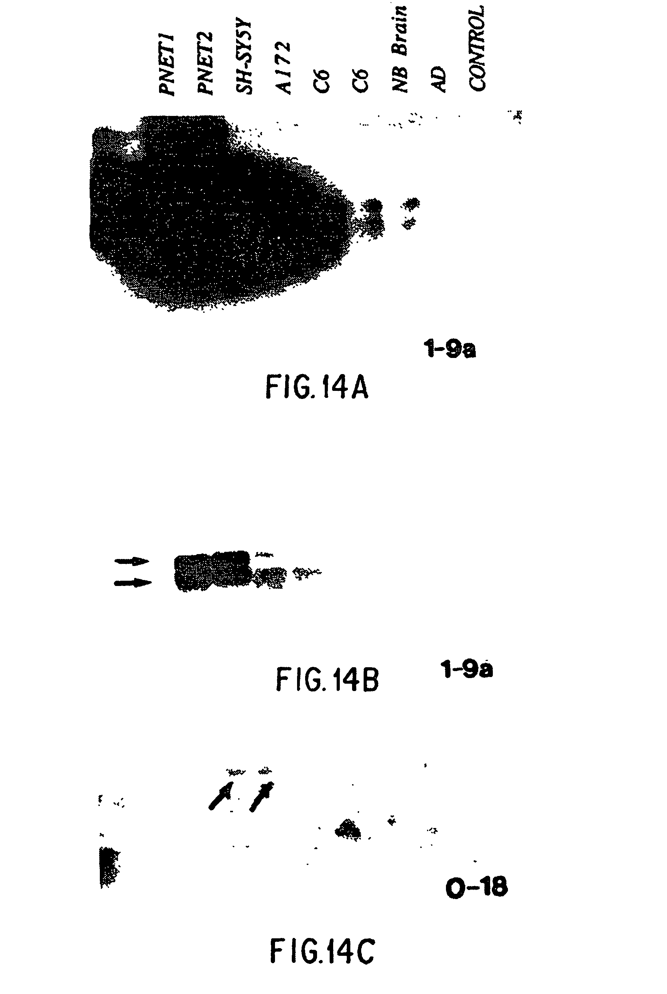

CNS thread protein transcripts were detected in neuroectodermal tumor cell lines and in AD human brain tissue by reverse transcription followed by PCR using specific primers corresponding to the 5' and 3' regions of the 1-9a cDNA sequence. Southern blot analysis of the PCR products demonstrated two dominant cross-hybridizing species, 0.8 kb and 1.0 kb (Figures 14a and 14b). In addition, in the SH-Sy5y cells, a larger 1.8 kb PCR product was also detected. In the PNET1, PNET2, SH-Sy5y, and A172 cells, a 0.4 kb PCR product that hybridized with the 1-9a probe was observed. Corresponding with the higher levels of thread protein mRNAs in Alzheimer's disease brains, the hybridization signal was more intense in AD samples compared with aged control samples.

The PCR products generated from the SH-Sy5y cells were subcloned and sequenced. Southern analysis of the cloned fragments exhibited intense hybridization with the 1-9a cDNA, and less intense but definite hybridization with the O18 cDNA (rat PTP) (Figure 14c). The nucleic acid sequence of the SH-Sy5y PCR clone (Sy-NTP) was identical to the 1-9a cDNA sequence.

### Example 7

### Isolation of Genomic Clones Coding for Human Brain Thread Proteins

A human genomic DNA library was screened using probes made with a 600 bp fragment of the 1-9a human brain thread protein cDNA that was isolated from the two year-old temporal cortex library. The 1-9a cDNA fragment contained a region with 60% nucleic acid sequence homology with human PTP. After colony purification, the putative genomic clones were checked for cross-hybridization with the O18 rat PTP cDNA fragment. *EcoRl, Pst*I, and *Eco*RI/*Pst*I restriction fragments that hybridized with both the 1-9a and O18 probes were subcloned into pBluescript II vectors (Promega, Inc., Madison, WI) and then sequenced by the dideoxynucleotide chain termination method using either T7 polymerase (USB Sequenase) or polymerase chain reaction amplification and Vent polymerase.

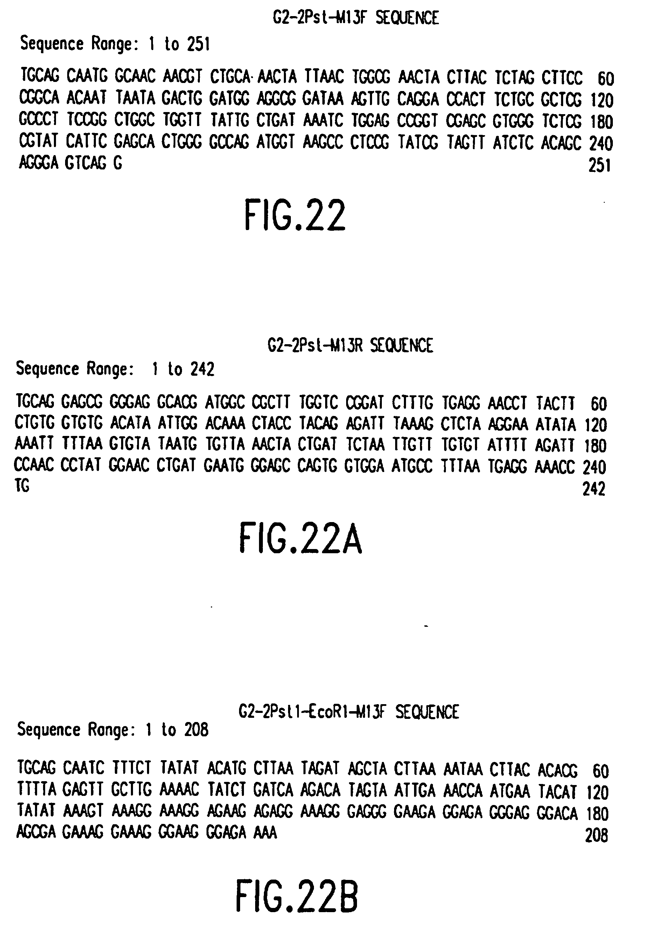

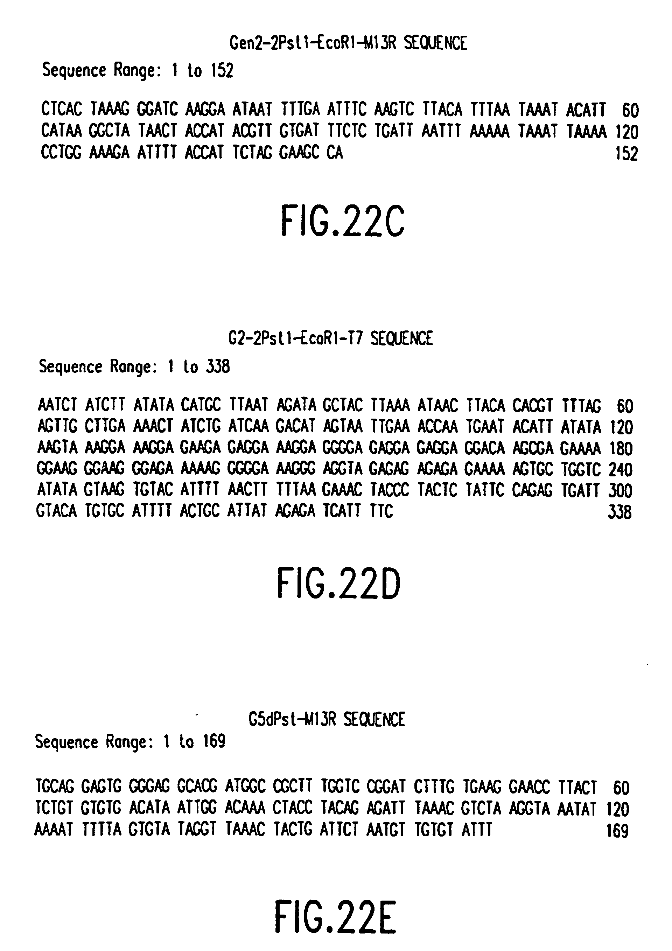

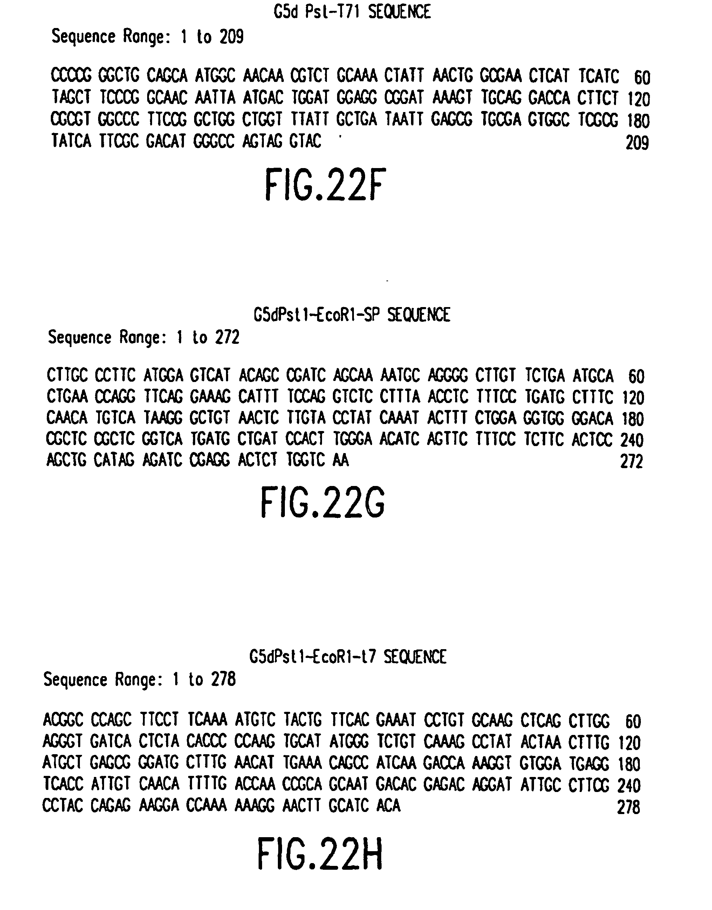

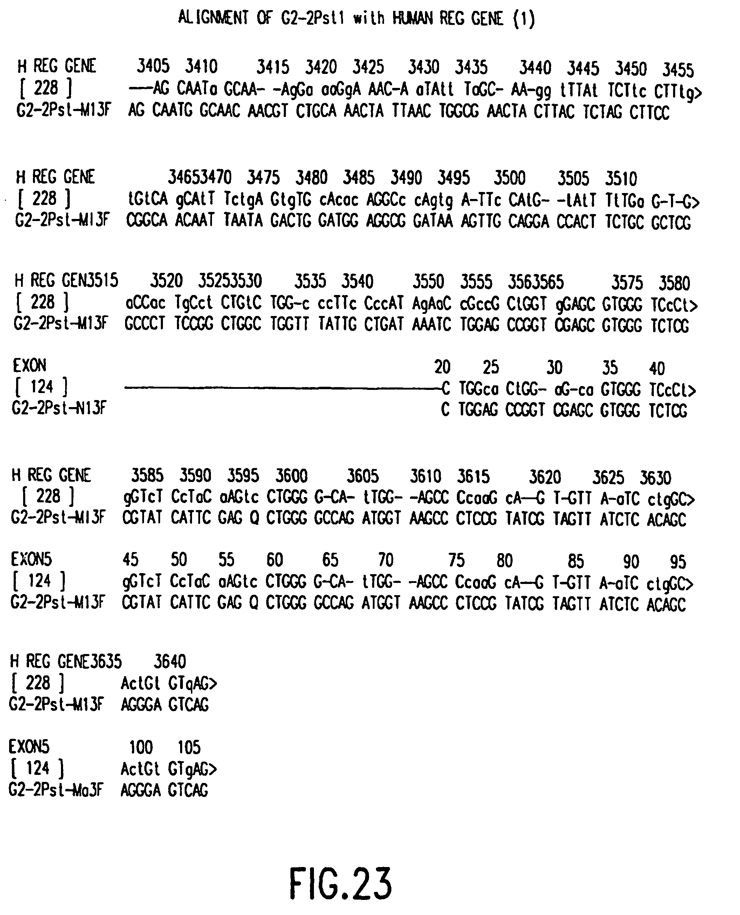

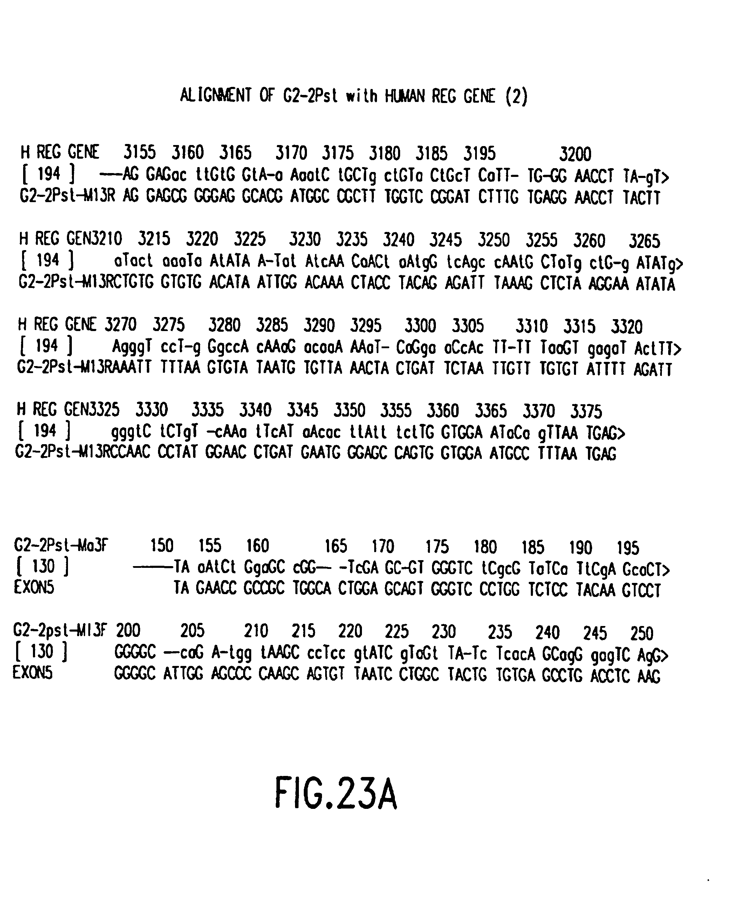

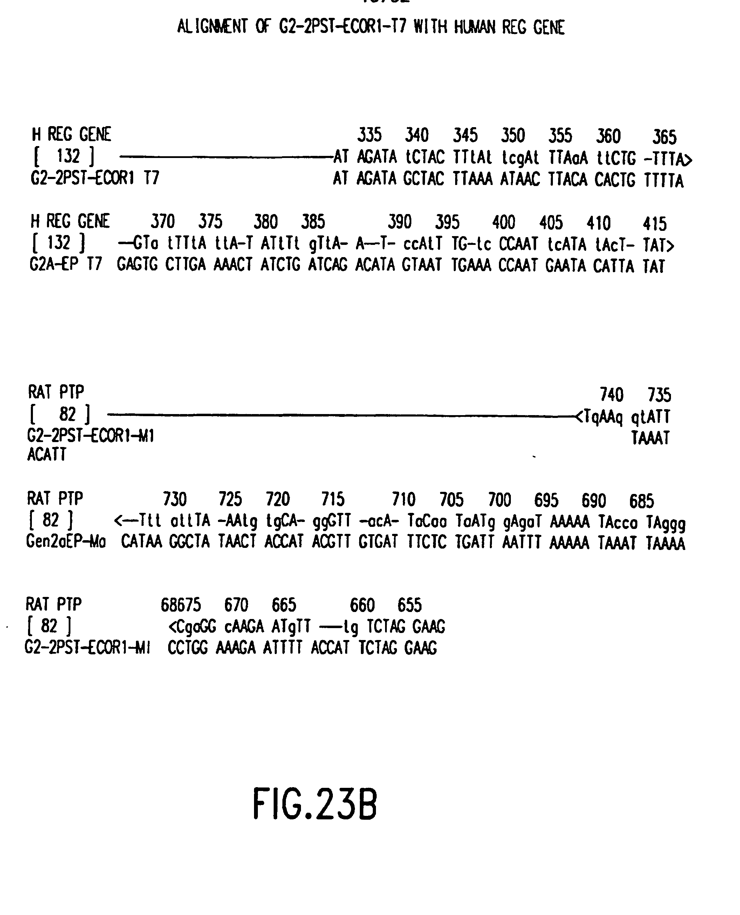

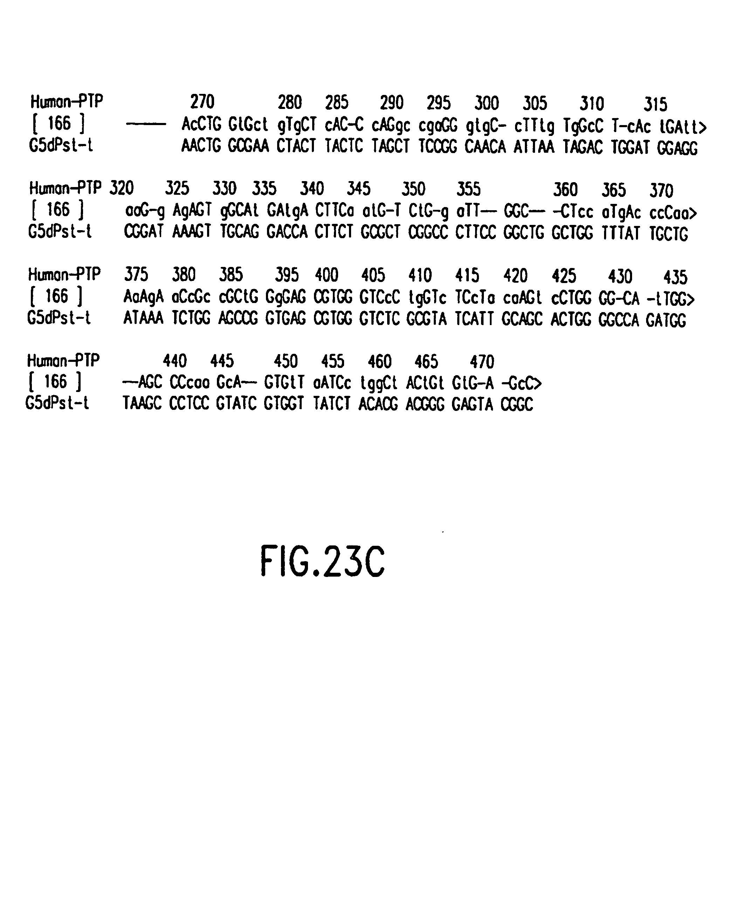

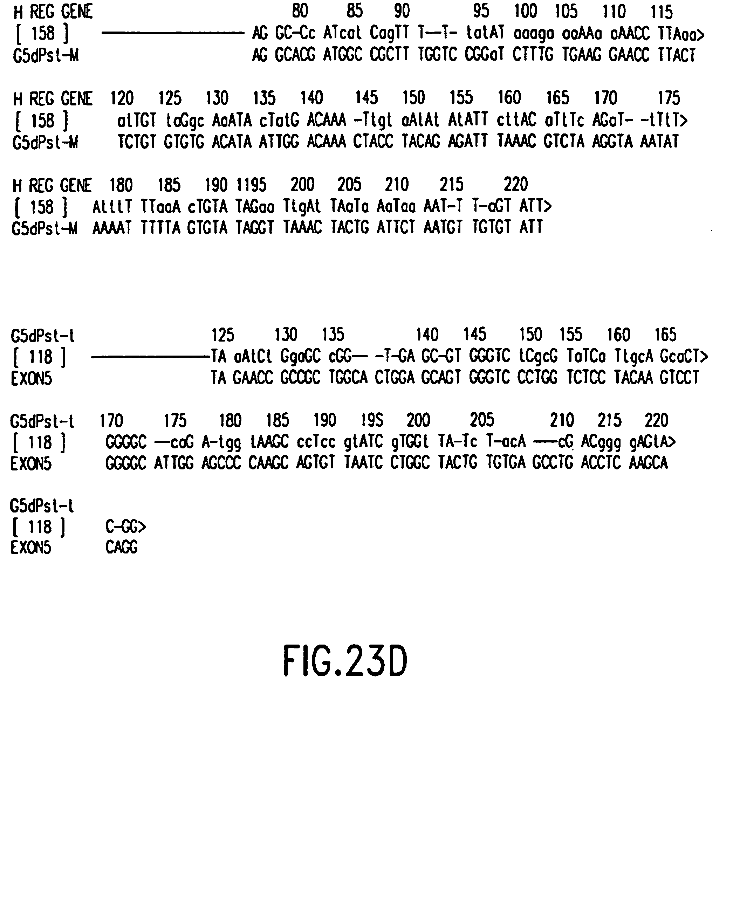

Four genomic fragments designated G2-2 *Pst*I, G2-2 *Pst*I-*Eco*RI, G5d-1 *Pst*I, and G5d-1 *Pst*I-*Eco*RI were isolated from a human genomic DNA library (Figures 22a-22d). These genomic fragments all hybridized with both the 1-9a and O18 cDNA probes, and they ranged in size between 1.5 kb and 3 kb. Partial nucleic acid sequence information demonstrated homology between G2-*2Pstl* and the human Reg gene and human and rat PTP cDNAs (Figure 23a); between G2-2 *Pst*I-*Eco*RI and both the Reg gene and rat PTP cDNA (Figure 23b), and also with AD 2-2, AD 3-4, and the 1-9a cDNAs (data not shown); between G5d-1 *Pst*I and the Reg gene and human PTP (Figure 23c); and between G5d-1 *Pst*I-*Eco*RI and Reg gene, human PTP, 1-9a, and AD 4-4.

### Example 8

### In vitro Expression of the LacZ Fusion Protein and Demonstration of its Relatedness to Thread Proteins

Fusion protein expression in bacteria containing the 1-9a cDNA clone, or one of the four genomic clones was induced with isopropylthio-β-D-galactoside (IPTG) using standard techniques (Sambrook *et al*., *supra*). Crude bacterial lysates from induced and uninduced cultures were subjected to SDS-PAGE and Western blot analysis using the Th9 monoclonal antibody to thread protein (Sasaki *et al*., *J. Biol. Chem. 268*:1-4 (1993)), and ¹²⁵-I labeled protein A to detect the bound antibody. In addition, bacterial lawns containing cloned DNA were induced to express the fusion protein with IPTG, and replica filters were probed directly with Th9 monoclonal antibody followed by ¹²⁵-I labeled protein A.

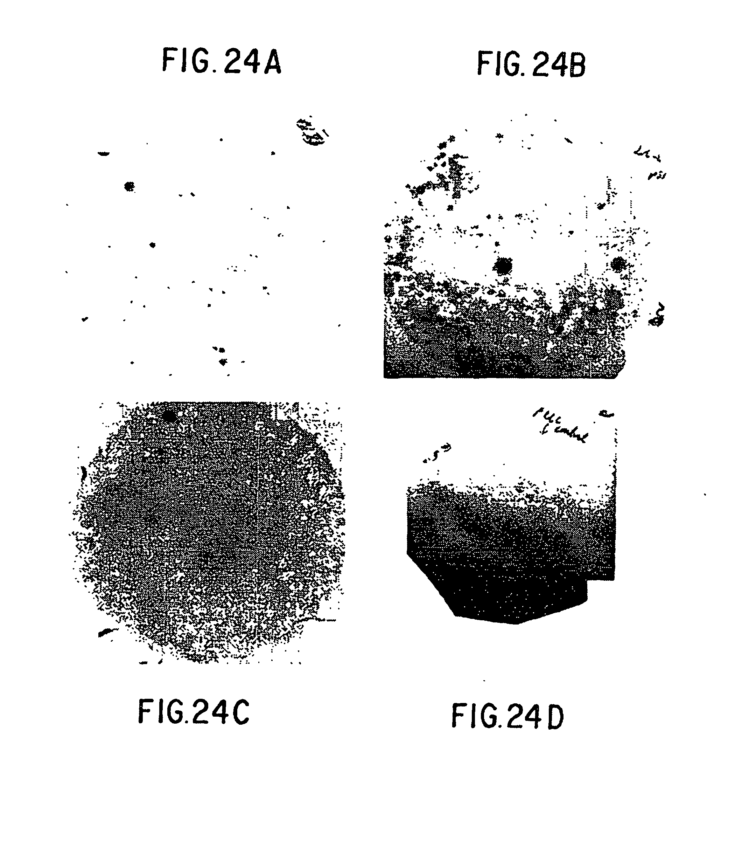

Thread protein immunoreactivity was demonstrated in the bacterial fusion proteins by direct antibody binding to the IPTG-induced colonies (Figure 24). Thread protein immunoreactivity was detected using a cocktail of Th9, Th7, and Th10 monoclonal antibodies to PTP (Sasaki *et al*., *J. Biol. Chem. 268:1-4* (1993), and ¹²⁵-I labeled Protein A.

### Example 9

### Relative Levels of AD16c mRNA in AD and Aged Control Brains

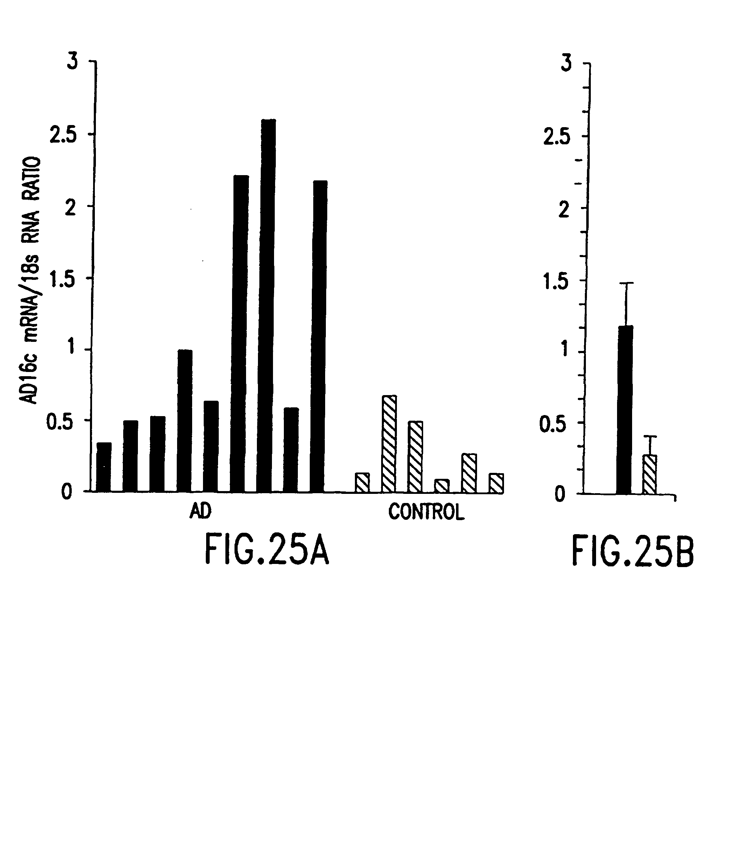

Northern blot analysis was performed using an AD16 cDNA probe. The blots were re-probed to detect 18s ribosomal RNA to evaluate loading of RNA in each lane. The unsaturated autoradiograms were subjected to densitometric analysis using a Molecular Dynamics Image Analyzer. The ratios of the AD16c and 18s RNA hybridization signals were plotted for each case, and the results are depicted graphically in Figure 25. The mean ratios (relative levels of AD16c) with standard errors are depicted in the smaller right hand graph. The findings confirm that there are elevated levels of AD16c mRNA expression in 6 of 9 AD brains compared to 1 of 6 age-matched controls. The difference between the mean levels is highly statistically significant (P<0.005). Similar results were obtained using AD10-7 probes. Theses results demonstrate that there is a statistically significant increase in levels of expression in AD brains compared to control brains.

### Example 10

### Preparation of Recombinant AD10-7 Fusion Protein and Detection Thereof With Monoclonal Antibodies

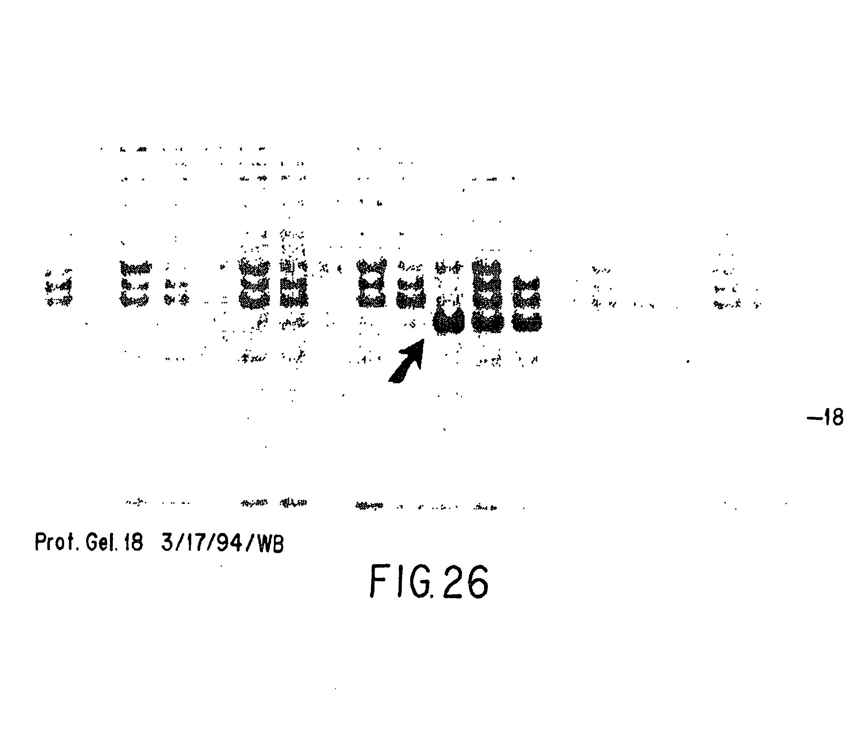

AD10-7 cDNA was ligated into pTrcHIS vectors (In Vitrogen, San Diego) in three different reading frames (two incorrect-A and B, and one correct-C). Bacteria transformed with one of the three plasmids were induced with IPTG and bacterial lysates were examined for protein expression 0, 1 and 5 hours later. The proteins were fractionated by SDS-PAGE, and Western blot analysis was performed using monoclonal antibodies against the expressed tag protein (T7-tag mouse monoclonal antibodies; Novogen). The blots were developed using the avidin-biotin, horseradish peroxidase method, with diaminobenzidine as the chromogen (Figure 26). A band corresponding to ~45 kDA was detected in bacteria that had been transformed with plasmid DNA which contained AD10-7 ligated only in the correct reading frame (C) (arrow). The same size protein was observed by *in vitro* translation of the AD10-7 cDNA in a rabbit reticulocyte lysate assay system. In both systems, the fusion partner peptide was ~ 3 kDA, indicating that the cDNA encodes a protein of about ~ 42 kDA. A ~ 42 kDA NPT species is routinely detected by Western Blot analysis of neuronal cell lines and of human brain tissue.

### Example 11

### Demonstration of Neuronal Localization of AD10-7 mRNA Expression by In Situ Hybridization

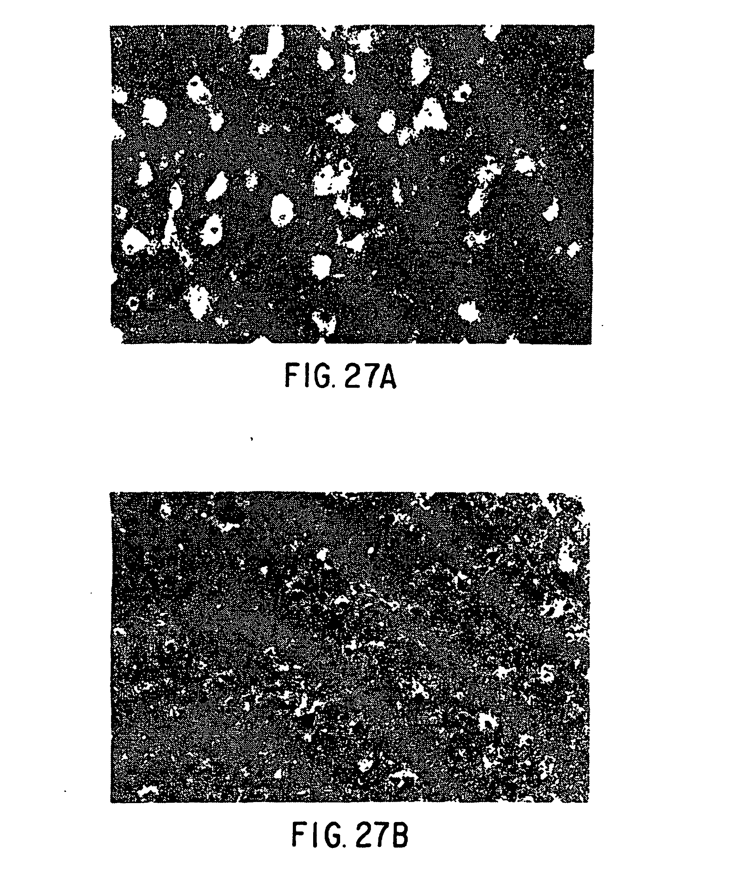

Sense and antisense cRNA probes were generated from linearized AD10-7 plasmid DNA using SP6 or T7 DNA-dependent RNA polymerase, respectively. The antisense probes hybridized with neuronal cell line mRNA as described above for this clone. The cRNA sense probes, on the other hand, failed to hybridize with RNA by Northern blot analysis, cRNA probes labeled with digoxigenin-UTP were hybridized with human brain tissue sections from early AD. After washing the sections extensively (de la Monte *et al., J. Clin. Invest. 86*:1004-1013 (1990)), the hybridized probes were detected using peroxidase or alkaline phosphatase conjugated monoclonal antibodies to digoxigenin, and the colorimetric reactions were revealed using standard methods. Examination of the sections by brightfield and darkfield microscopy demonstrated hybridization of AD10-7 only in neurons (Fig. 27; dense aggregates of white grains over cell bodies in (**A**)). In contrast, and similar to the findings by Northern blot analysis, the *sense* AD10-7 cRNA probes failed to hybridize with brain tissue (**B**).

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention, which is defined by the following Claims.

All publications and patent applications mentioned in this specification are indicative of the level of skill of those in the art to which the invention pertains. All publications and patent applications are herein incorporated by reference to the same extent as if each individual publication or patent application was specifically and individually indicated to be incorporated by reference in their entirety.

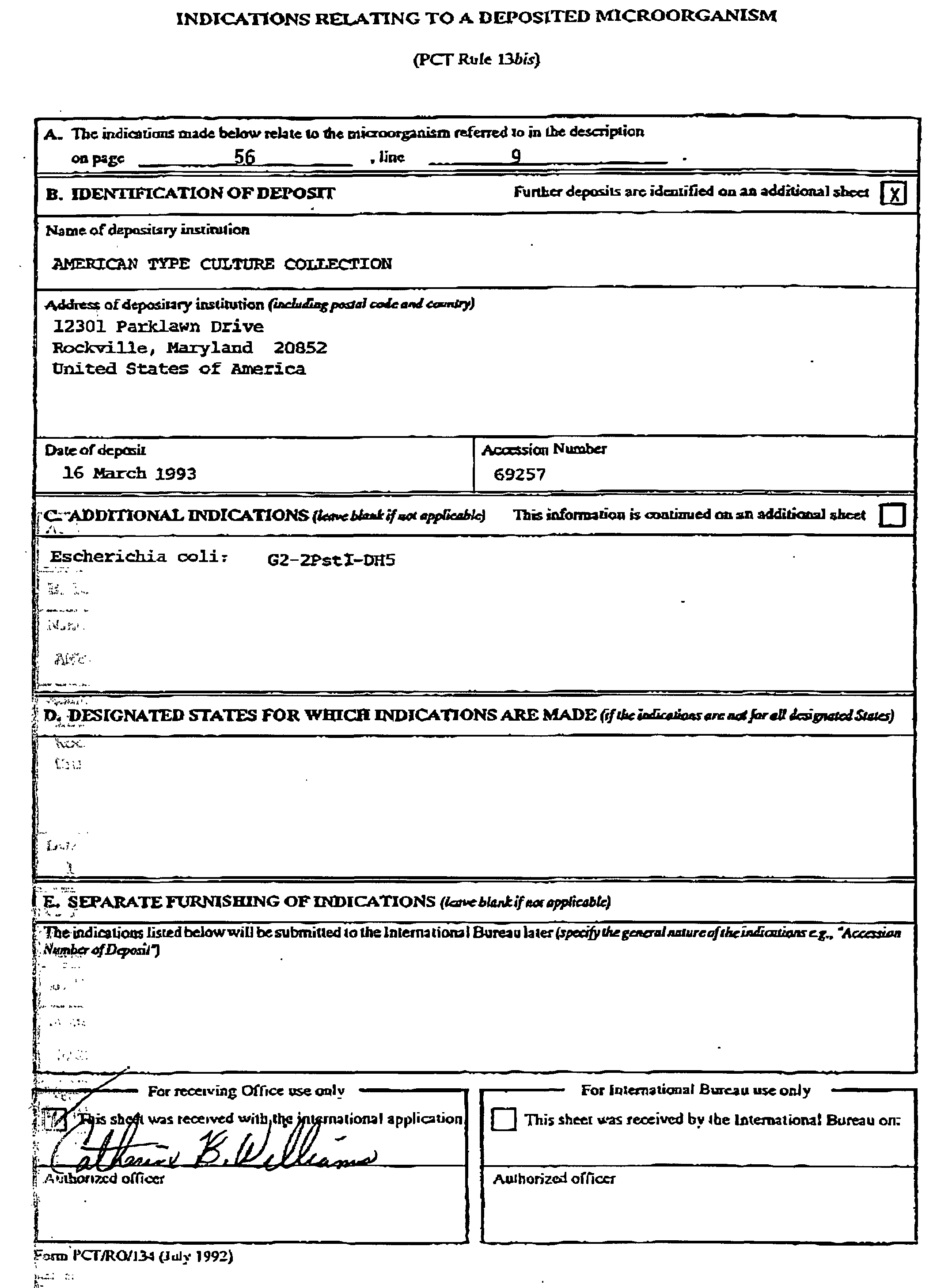
INDICATIONS RELATING TO A DEPOSITED MICROORGANISM

(PCT Rule 13bis)

A. The indications made below relate to the microorganism referred to in the description
on page 56 , line 9 .

B. IDENTIFICATION OF DEPOSIT | Further deposits are identified on an additional sheet [X]

Name of depositary institution
AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69257

C. ADDITIONAL INDICATIONS (leave blank if not applicable) | This information is continued on an additional sheet [ ]

Escherichia coli: G2-2PstI-DH5

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[X] This sheet was received with the international application | [ ] This sheet was received by the International Bureau on:
 |
Authorized officer | Authorized officer

Form PCT/RO/134 (July 1992)

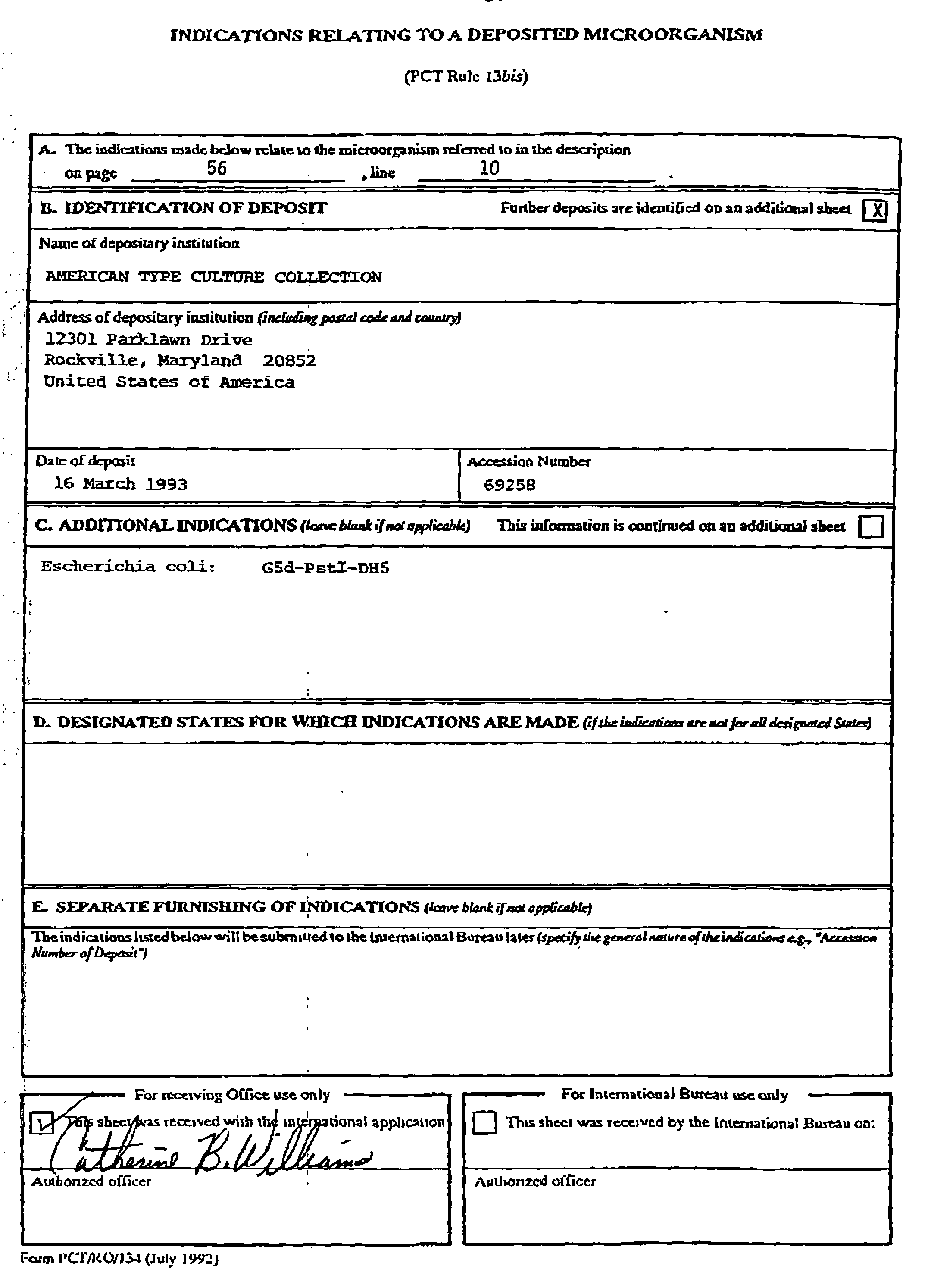
INDICATIONS RELATING TO A DEPOSITED MICROORGANISM

(PCT Rule 13bis)

A. The indications made below relate to the microorganism referred to in the description
on page 56 , line 10 .

B. IDENTIFICATION OF DEPOSIT | Further deposits are identified on an additional sheet [X]

Name of depositary institution
AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69258

C. ADDITIONAL INDICATIONS (leave blank if not applicable) | This information is continued on an additional sheet [ ]

Escherichia coli: G5d-PstI-DH5

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[✓] This sheet was received with the international application | [ ] This sheet was received by the International Bureau on:
 |
Authorized officer | Authorized officer

Form PCT/RO/134 (July 1992)

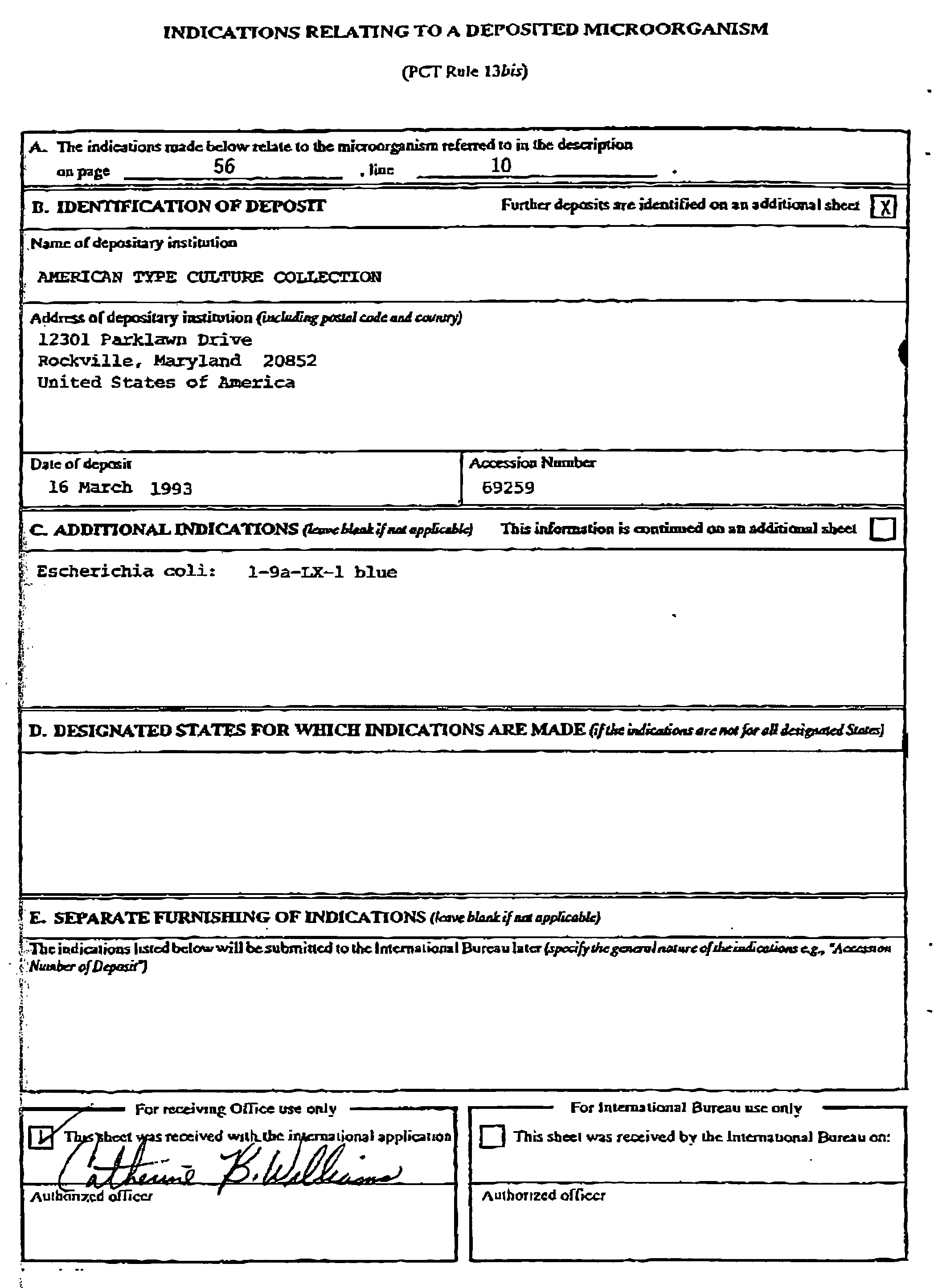
INDICATIONS RELATING TO A DEPOSITED MICROORGANISM

(PCT Rule 13bis)

A. The indications made below relate to the microorganism referred to in the description
on page 56 , line 10 .

B. IDENTIFICATION OF DEPOSIT | Further deposits are identified on an additional sheet [X]

Name of depositary institution
AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69259

C. ADDITIONAL INDICATIONS (leave blank if not applicable) | This information is continued on an additional sheet [ ]

Escherichia coli: 1-9a-LX-1 blue

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[✓] This sheet was received with the international application | [ ] This sheet was received by the International Bureau on:
 |
Authorized officer | Authorized officer

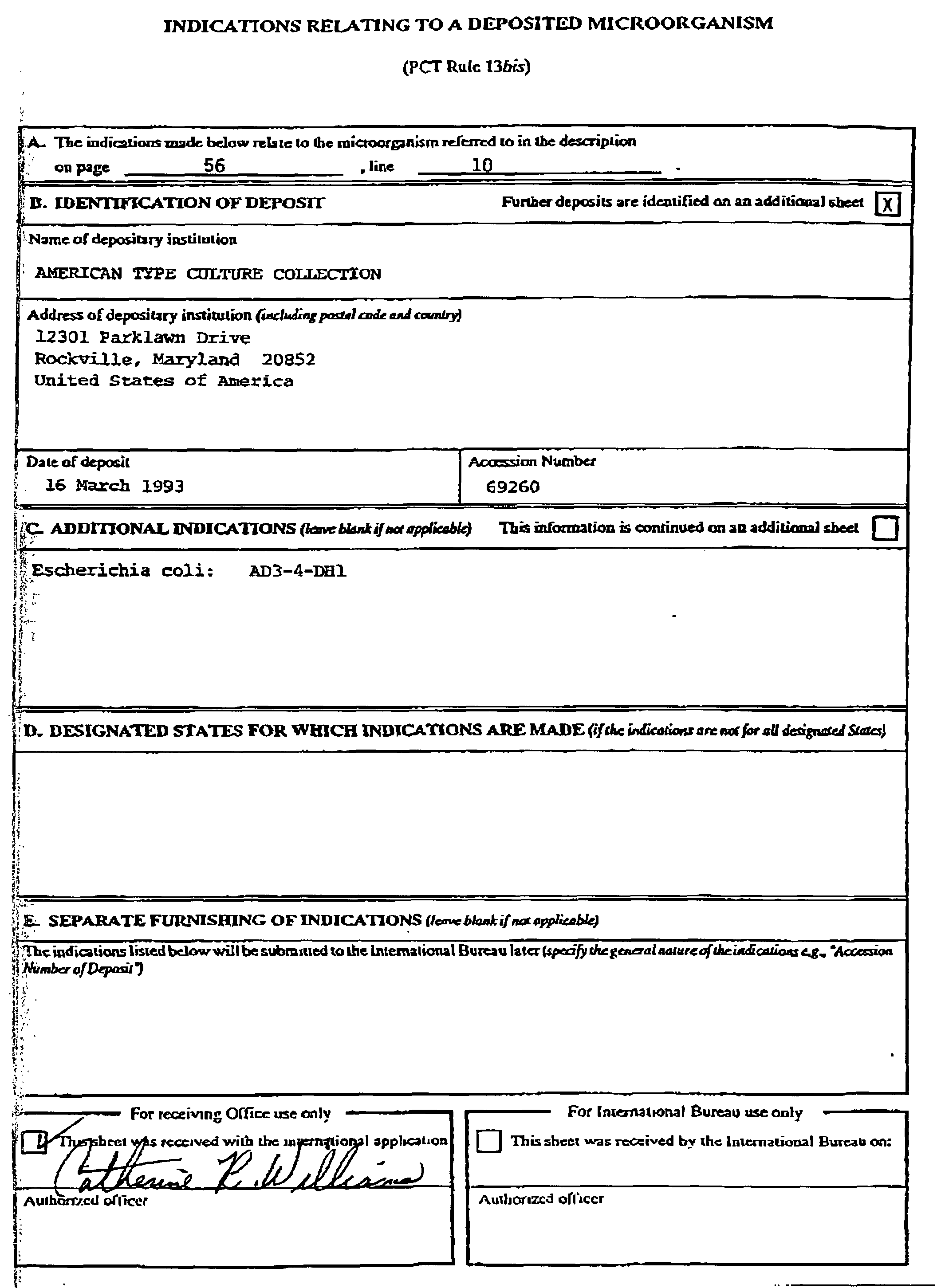
INDICATIONS RELATING TO A DEPOSITED MICROORGANISM

(PCT Rule 13bis)

A. The indications made below relate to the microorganism referred to in the description
on page 56 , line 10 .

B. IDENTIFICATION OF DEPOSIT | Further deposits are identified on an additional sheet [X]

Name of depositary institution
AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69260

C. ADDITIONAL INDICATIONS (leave blank if not applicable) | This information is continued on an additional sheet [ ]

Escherichia coli: AD3-4-DH1

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[✓] This sheet was received with the international application | [ ] This sheet was received by the International Bureau on:
 |
Authorized officer | Authorized officer

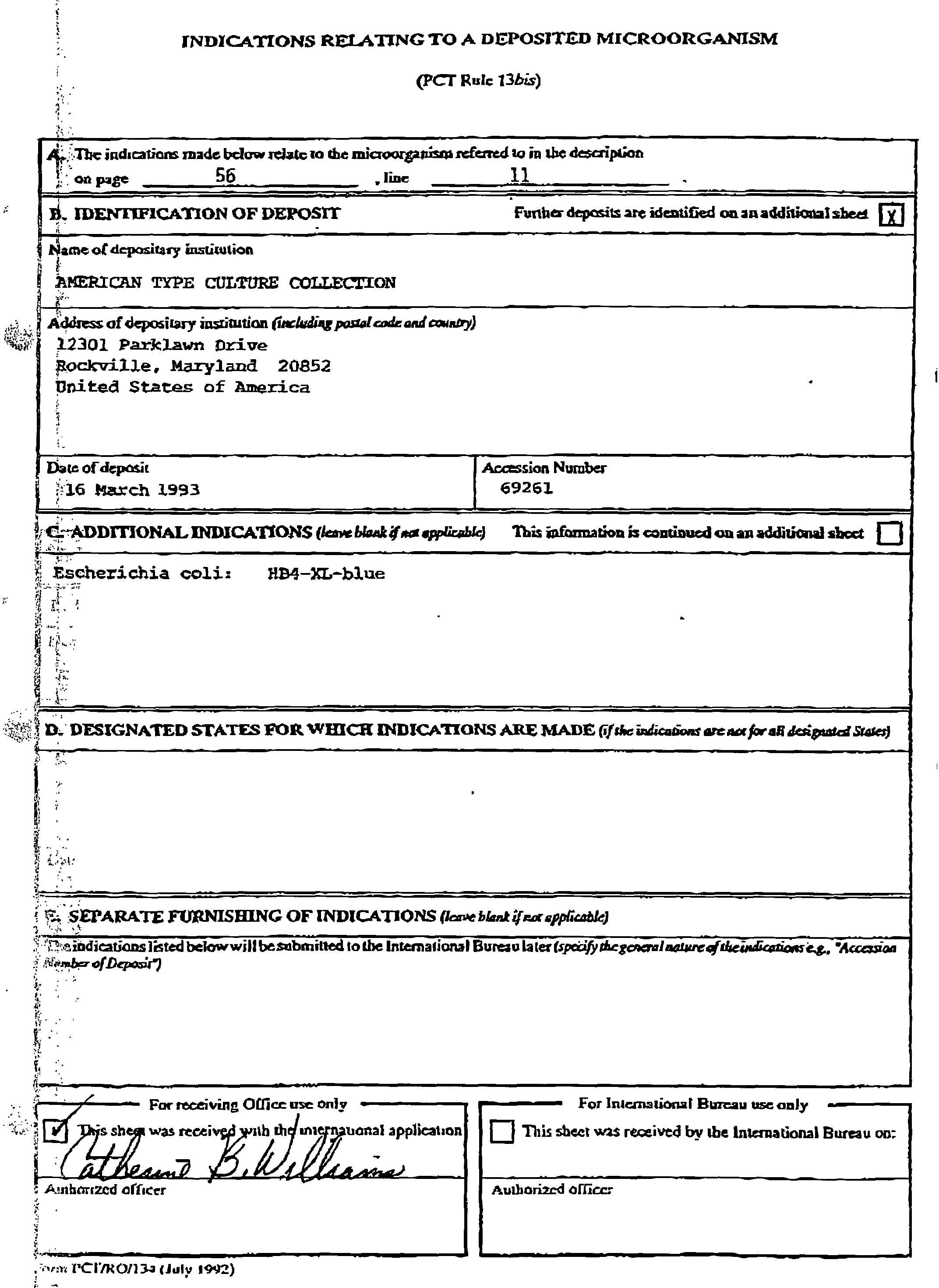
INDICATIONS RELATING TO A DEPOSITED MICROORGANISM

(PCT Rule 13bis)

A. The indications made below relate to the microorganism referred to in the description
on page 56 , line 11 .

B. IDENTIFICATION OF DEPOSIT | Further deposits are identified on an additional sheet [X]

Name of depositary institution

AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69261

C. ADDITIONAL INDICATIONS (leave blank if not applicable) | This information is continued on an additional sheet [ ]

Escherichia coli: HB4-XL-blue

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[✓] This sheet was received with the international application | [ ] This sheet was received by the International Bureau on:


Authorized officer | Authorized officer

Form PCT/RO/134 (July 1992)

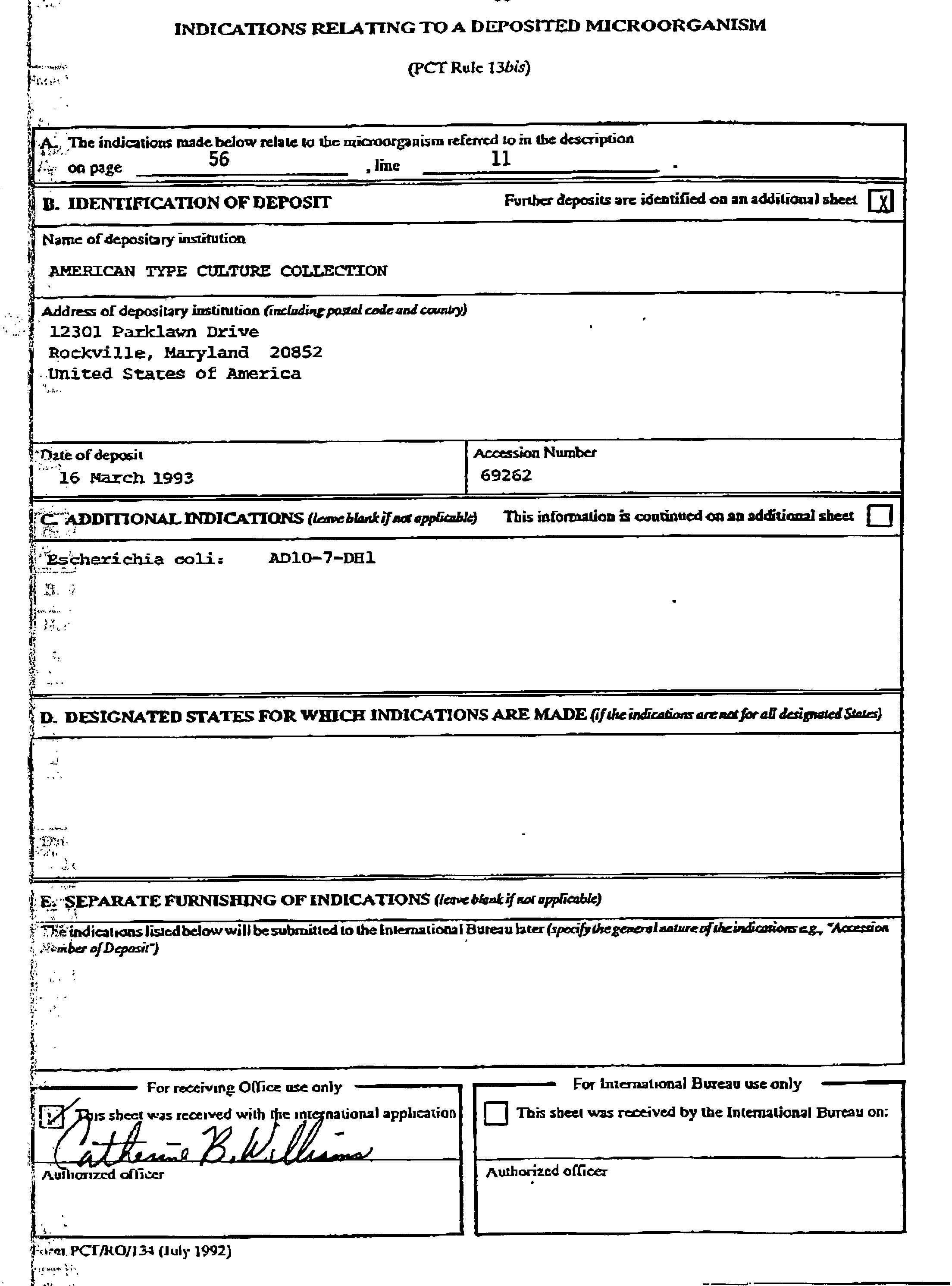
INDICATIONS RELATING TO A DEPOSITED MICROORGANISM

(PCT Rule 13bis)

A. The indications made below relate to the microorganism referred to in the description
on page 56 , line 11 .

B. IDENTIFICATION OF DEPOSIT | Further deposits are identified on an additional sheet [X]

Name of depositary institution
AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69262

C. ADDITIONAL INDICATIONS (leave blank if not applicable) | This information is continued on an additional sheet [ ]

Escherichia coli: AD10-7-DH1

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[✓] This sheet was received with the international application | [ ] This sheet was received by the International Bureau on:
 |
Authorized officer | Authorized officer

Form PCT/RO/134 (July 1992)

INDICATIONS RELATING TO A DEPOSITED MICROORGANISM

(PCT Rule 13bis)

A. The indications made below relate to the microorganism referred to in the description
on page 56 , line 12 .

B. IDENTIFICATION OF DEPOSIT | Further deposits are identified on an additional sheet [X]

Name of depositary institution
AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69263

C. ADDITIONAL INDICATIONS (leave blank if not applicable) | This information is continued on an additional sheet [ ]

Escherichia coli: AD2-2-DH1

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[X] This sheet was received with the international application | [ ] This sheet was received by the International Bureau on:
[signature] |
Authorized officer | Authorized officer

Form PCT/RO/134 (July 1992)

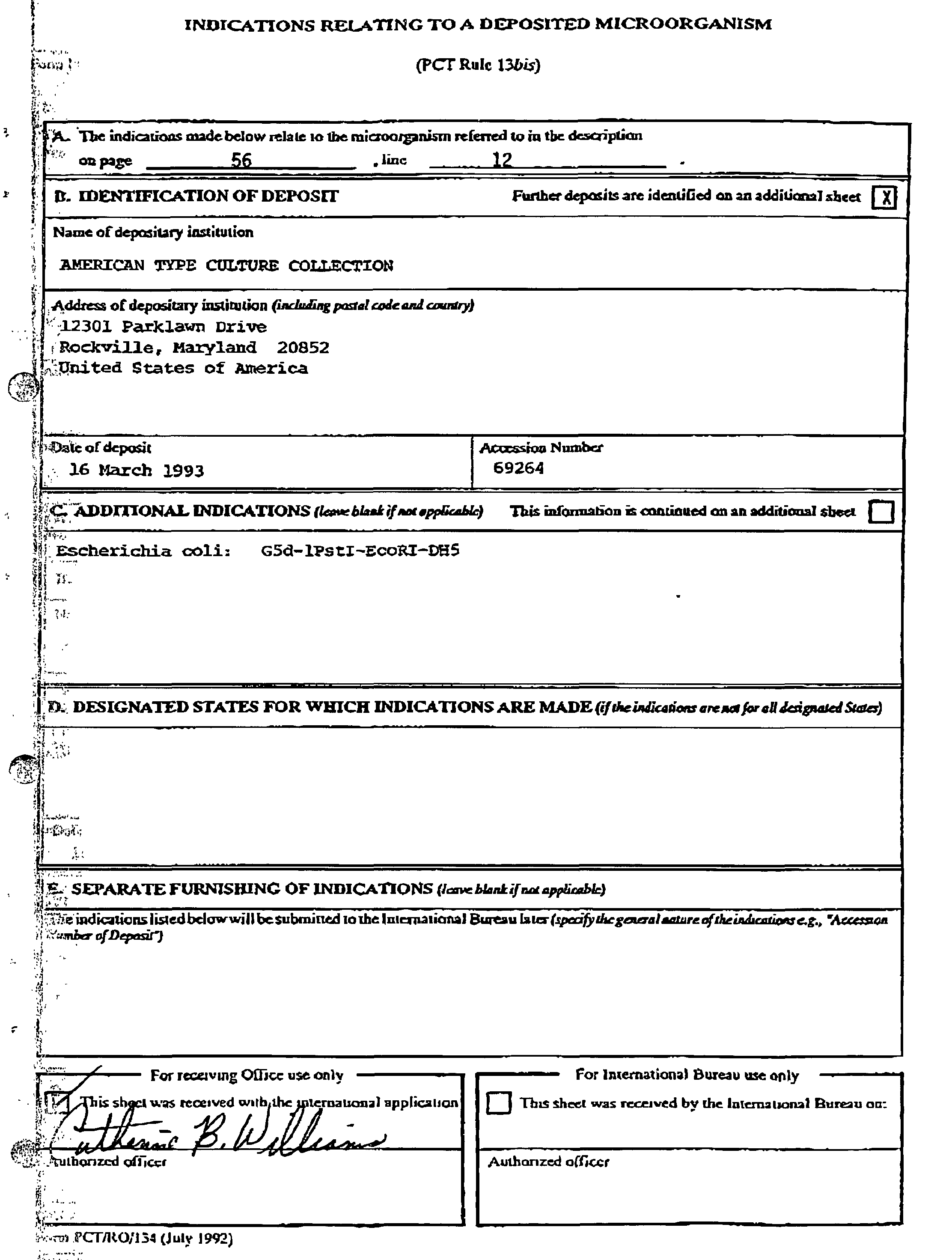
INDICATIONS RELATING TO A DEPOSITED MICROORGANISM

(PCT Rule 13bis)

A. The indications made below relate to the microorganism referred to in the description
on page 56 , line 12 .

B. IDENTIFICATION OF DEPOSIT | Further deposits are identified on an additional sheet [X]

Name of depositary institution

AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69264

C. ADDITIONAL INDICATIONS (leave blank if not applicable) | This information is continued on an additional sheet [ ]

Escherichia coli: G5d-1PstI-EcoRI-DH5

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only

[X] This sheet was received with the international application | [ ] This sheet was received by the International Bureau on:


Authorized officer | Authorized officer

Form PCT/RO/134 (July 1992)

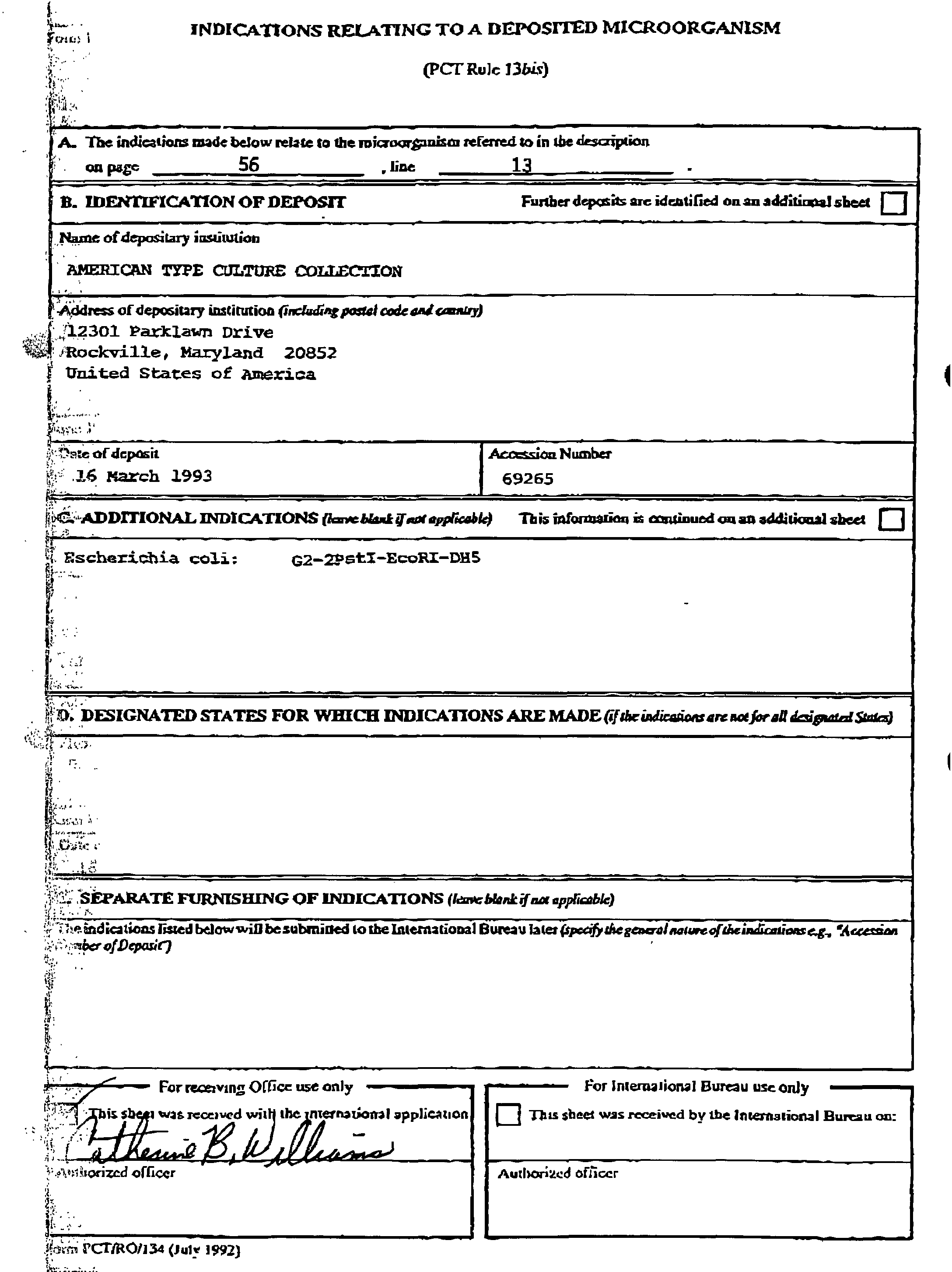
INDICATIONS RELATING TO A DEPOSITED MICROORGANISM

(PCT Rule 13bis)

A. The indications made below relate to the microorganism referred to in the description
on page 56 , line 13 .

B. IDENTIFICATION OF DEPOSIT | Further deposits are identified on an additional sheet [ ]

Name of depositary institution

AMERICAN TYPE CULTURE COLLECTION

Address of depositary institution (including postal code and country)
12301 Parklawn Drive
Rockville, Maryland 20852
United States of America

Date of deposit | Accession Number
16 March 1993 | 69265

C. ADDITIONAL INDICATIONS (leave blank if not applicable) | This information is continued on an additional sheet [ ]

Escherichia coli: G2-2PstI-EcoRI-DH5

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[ ] This sheet was received with the international application | [ ] This sheet was received by the International Bureau on:
 |
Authorized officer | Authorized officer

Form PCT/RO/134 (July 1992)

## Claims

1. A Neural Thread Protein (NTP) substantially free of any natural impurities and coded for by the AD10-7 DNA molecule present in the *E*. *coli* cells that are on deposit at the American Type Culture Collection, Manassass, Va., under accession no. 69262.

2. An isolated nucleic acid molecule encoding a Neural Thread Protein (NTP) comprising the AD10-7 DNA molecule present in the *E. coli* cells that are on deposit at the American Type Culture Collection, Manassass, Va., under accession no. 69262.

3. The nucleic acid molecule of claim 2 comprising a genetic sequence coding for an NTP wherein said NTP is human NTP.

4. A plasmid comprising the nucleic acid molecule of claim 2 or claim 3.

5. A non-human host transformed with the plasmid of claim 4.

6. An in vitro method of using the plasmid of claim 4 to prepare an NTP, said method comprising:
(a) introducing said plasmid comprising said genetic sequence coding for said NTP into a host cell to produce a recombinant host cell;
(b) culturing said recombinant host cell; and
(c) isolating said NTP.

7. The method of claim 6, wherein said genetic sequence coding for said NTP is obtained from brain tissue of a patient with Alzheimer's Disease or from a human neural tumor cell line.

8. The method of claim 6 or claim 7, wherein said host is *E. coli*.

9. The method as claimed in any one of claims 6 to 8, wherein said gene is contained by a vector.

10. The method as claimed in any one of claims 6 to 9, wherein said gene is under control of an inducible promoter.

11. The method of claim 10, wherein said promoter is a lambda P₁ promoter, or a *tac* promoter.

12. An expression vector comprising the nucleic acid molecule of claim 2.

13. A virion comprising the expression vector of claim 12.

14. A 15- to 30-mer antisense oligonucleotide selected from the group consisting of SEQ ID NO:1, SEQ ID NO:2, SEQ ID NO:3, SEQ ID NO:4, SEQ ID NO: 5 and SEQ ID NO:6, which is complementary to a nucleic acid sequence coding for the NTP of claim 1, and which is nonhomologous to the PTP nucleic acid sequence.

15. The antisense oligonucleotide of claim 14 which is DNA.

16. The antisense oligonucleotide of claim 14 or claim 15 which is an o-oligonucleotide.

17. The antisense oligonucleotide of claim 14 or claim 15 which is an S-oligonucleotide.

18. A ribozyme comprising a target sequence which is complementary to a nucleic acid sequence coding for the NTP of claim 1, and non-homologous to the PTP nucleic acid sequence.

19. A DNA molecule which codes for the ribozyme of claim 18.

20. An oligonucleotide comprising the sequence 3'X5'-L-5'X3', wherein X comprises a nucleic acid sequence selected from the group consisting of SEQ ID Nos: 1 - 6 and wherein L represents an oligonucleotide linkage.

21. An oligonucleotide comprising a sequence 5'X3'-L-3'X5', wherein X comprises a nucleic acid sequence selected from the group consisting of SEQ ID Nos: 1 - 6 and wherein L represents an oligonucleotide linkage.

22. A ribonucleotide NTP External Guide Sequence comprising:
(a) a 10-15 nucleotide sequence which is complementary to a nucleic acid sequence which codes for the NTP of claim 1, and which is non-homologous to the PTP nucleic acid sequence; and
(b) a 3'-NCCA nucleotide sequence wherein N is purine.

23. The NTP External Guide Sequence of claim 22 which is DNA.

24. An *in vitro* method for detecting the presence of Neural Thread Protein (NTP) encoded by the nucleic acid of claim 2, said method comprising:
(a) contacting a biological sample from a human subject that is suspected of containing an NTP encoded by the nucleic acid of claim 2 with at least one molecule capable of binding to said NTP;
(b) detecting any of molecule bound to said NTP.

25. A method as claimed in claim 24, wherein said molecule is:
(a) an antibody substantially free of natural impurities;
(b) a monoclonal antibody; or
(c) a fragment of (a) or (b).

26. A method as claimed in claim 24 or claim 25, wherein the detecting of any of said molecule bound to said NTP is performed by *in situ* imaging.

27. A method as claimed in any one of claims 24 to 26, wherein the detecting of any of said molecule bound to said NTP is performed by *in vitro* imaging.

28. A *in vitro* method of diagnosing the presence of a Neural Thread Protein (NTP) - related condition in a human subject suspected of having such a condition, which comprises:
(a) incubating a biological sample, which is suspected of containing an NTP encoded for by said nucleic acid molecule of claim 2, in the presence of at least one binding molecule capable of identifying said NTP; and
(b) detecting said binding molecule which is bound in said sample, wherein said detection indicates that said subject has an NTP-related condition;
wherein said NTP-related condition is Alzheimer's Disease, the presence of neuroectodermal tumors, the presence of a malignant astrocytoma or the presence of glioblastomas.

29. A diagnostic method as claimed in claim 28, wherein said detection is by immunometric assay.

30. A diagnostic method as claimed in claim 29, wherein said immunometric assay is a monoclonal antibody-based immunometric assay.

31. A diagnostic method as claimed in claim 28, wherein said method comprises:
(a) incubating said biological sample with two different NTP monoclonal antibodies bound to a solid phase support; and
(b) detecting NTP as defined in claim 1 bound to said monoclonal antibodies with a third different detectably labelled NTP monoclonal antibody solution, wherein said NTP monoclonal antibodies are specific for the NTP molecule of claim 1.

32. A diagnostic method as claimed in any one of claims 28 to 31, wherein said incubating step further includes adding a known quantity of labelled NTP as defined in claim 1 whereby a competitive immunoassay is established.

33. A diagnostic method as claimed in claim 32, wherein said label is capable of emitting radiation.

34. A diagnostic method as claimed in claim 33, wherein said label is ¹²⁵I.

35. A diagnostic method as claimed in any one of claims 28 to 34, wherein said detection is by imuno-polymerase chain reaction.

## Patentansprüche

1. Neurofilamentprotein (NFP), das im Wesentlichen frei von natürlichen Verunreinigungen ist und durch das AD10-7-DNA-Molekül codiert ist, das in den E.coli-Zellen, die in der American Type Culture Collection [Amerikanischen Typus-Kultursammlung], Manassass, Virginia, unter der Zugangsnr. 69262 hinterlegt sind, anwesend ist.

2. Isoliertes Nucleinsäuremolekül, das ein Neurofilamentprotein (NFP) codiert, das das AD10-7-DNA-Molekül enthält, das in den *E.coli*-Zellen, die in der American Type Culture Collection [Amerikanischen Typus-Kultursammlung], Manassass, Virginia, unter der Zugangsnr. 69262 hinterlegt sind, anwesend ist.

3. Nucleinsäuremolekül nach Anspruch 2, das eine Gensequenzcodierung für ein NFP aufweist, wobei das NFP menschliches NFP ist.

4. Plasmid, das das Nucleinsäuremolekül nach Anspruch 2 oder 3 enthält.

5. Nichtmenschlicher Wirt, der mit dem Plasmid nach Anspruch 4 transformiert ist.

6. In-vitro-Verfahren der Verwendung des Plasmids nach Anspruch 4, um ein NFP zu präparieren, wobei das Verfahren Folgendes aufweist:
(a) Einführen des Plasmids, das die Gensequenzcodierung für das NFP enthält, in eine Wirtszelle, um eine rekombinante Wirtszelle zu produzieren;
(b) Kultivieren der rekombinanten Wirtszelle; und
(c) Isolieren des NFP.

7. Verfahren nach Anspruch 6, wobei die Gensequenzcodierung für das NFP aus Himgewebe eines Patienten mit Alzheimer'scher Krankheit oder aus einer menschlichen Neuraltumorzelllinie gewonnen wird.

8. Verfahren nach Anspruch 6 oder Anspruch 7, wobei der Wirt *E.coli* ist.

9. Verfahren nach einem der Ansprüche 6 bis 8, wobei das Gen von einem Vektor beinhaltet wird.

10. Verfahren nach einem der Ansprüche 6 bis 9, wobei sich das Gen unter der Kontrolle eines induzierbaren Promotors befindet.

11. Verfahren nach Anspruch 10, wobei der Promotor ein Lambda-P₁-Promotor oder ein *tac*-Promotor ist.

12. Expressionsvektor, der das Nucleinsäuremolekül nach Anspruch 2 aufweist.

13. Virion, das den Expressionsvektor nach Anspruch 12 enthält.

14. 15- bis 30-mer-Antisense-Oligonucleotid, das aus der Gruppe gewählt ist, die SEQ ID NO:1, SEQ ID NO:2, SEQ ID NO:3, SEQ ID NO:4, SEQ ID NO:5 und SEQ ID NO:6 enthält, und das zu einer Nucleinsäuresequenzcodierung für das NFP nach Anspruch 1 komplementär ist und das zu der PTP-Nucleinsäuresequenz nichthomolog ist.

15. Antisense-Oligonucleotid nach Anspruch 14, das DNA ist.

16. Antisense-Oligonucleotid nach Anspruch 14 oder Anspruch 15, das ein o-Oligonucleotid ist.

17. Antisense-Oligonucleotid nach Anspruch 14 oder Anspruch 15, das ein S-Oligonucleotid ist.

18. Ribozym, das eine Zielsequenz enthält, die zu einer Nucleinsäuresequenzcodierung für das NFP nach Anspruch 1 komplementär und zu der PTP-Nucleinsäuresequenz nichthomolog ist.

19. DNA-Molekül, das für das Ribozym nach Anspruch 18 codiert.

20. Oligonucleotid, das die Sequenz 3'X5'-L-5'X3' enthält, wobei X eine Nucleinsäuresequenz aufweist, die aus der Gruppe, die aus SEQ ID Nr. 1 - 6 besteht, gewählt ist, und wobei L eine Oligonucleotid-Kopplung darstellt.

21. Oligonucleotid, das eine Sequenz 5'X3-L-3'X5' enthält, wobei X eine Nucleinsäuresequenz aufweist, die aus der Gruppe, die aus SEQ ID Nr. 1 - 6 besteht, gewählt ist, und wobei L eine Oligonucleotid-Kopplung darstellt.

22. Ribonucleotid-NFP-Extemal-Guide-Sequenz, welches enthält:
(a) eine 10-15-Nucleotidsequenz, die zu einer Nucleinsäuresequenz, die für das NFP nach Anspruch 1 codiert, komplementär ist und die zu der PTP-Nucleinsäuresequenz nichthomolog ist; und
(b) eine 3'-NCCA-Nucleotidsequenz, wobei N Purin ist.

23. NFP-External-Guide-Sequenz nach Anspruch 22, die DNA ist.

24. In-vitro-Verfahren zum Nachweisen der Anwesenheit von Neurofilamentprotein (NFP), das durch die Nucleinsäure nach Anspruch 2 codiert ist, wobei das Verfahren Folgendes enthält:
(a) Kontaktieren einer biologischen Probe von einem menschlichen Subjekt, von dem angenommen wird, ein durch die Nucleinsäure nach Anspruch 2 codiertes NFP zu enthalten, mit wenigstens einem Molekül, das zur Bindung an das NFP fähig ist;
(b) Ermitteln jeden Moleküls, das an das NFP gebunden ist.

25. Verfahren nach Anspruch 24, wobei das Molekül Folgendes ist:
(a) ein im Wesentlichen von natürlichen Verunreinigungen freier Antikörper;
(b) ein monoklonaler Antikörper; oder
(c) ein Fragment von (a) oder (b).

26. Verfahren nach Anspruch 24 oder Anspruch 25, wobei das Ermitteln jeden Moleküls, das an das NFP gebunden ist, durch In-situ-Bildgebung durchgeführt wird.

27. Verfahren nach einem der Ansprüche 24 bis 26, wobei das Nachweisen jeden Moleküls, das an das NFP gebunden ist, durch In-vitro-Bildgebung durchgeführt wird.

28. In-vitro-Verfahren des Diagnostizierens der Anwesenheit eines mit einem Neurofilamentprotein (NFP) zusammenhängenden Zustands bei einem menschlichen Subjekt, von dem angenommen wird, einen solchen Zustand aufzuweisen, das Folgendes aufweist:
(a) Inkubieren einer biologischen Probe, von der angenommen wird, ein NFP, das durch das Nucleinsäuremolekül nach Anspruch 2 codiert ist, zu enthalten, in Anwesenheit von wenigstens einem Bindemolekül, das zum Identifizieren des NFP fähig ist; und
(b) Ermitteln des Bindemoleküls, das in der Probe gebunden ist, wobei die Ermittlung anzeigt, dass das Subjekt einen mit NFP zusammenhängenden Zustand aufweist;
wobei der mit NFP zusammenhängende Zustand die Alzheimer'sche Krankheit, die Anwesenheit von Neuroektoderm-Tumoren, die Anwesenheit eines malignen Astrozytoms oder die Anwesenheit von Glioblastomen ist.

29. Diagnoseverfahren nach Anspruch 28, wobei die Ermittlung durch immunometrischen Test erfolgt.

30. Diagnoseverfahren nach Anspruch 29, wobei der immunometrische Test ein auf monoklonalem Antikörper basierender immunometrischer Test ist.

31. Diagnoseverfahren nach Anspruch 28, wobei das Verfahren Folgendes aufweist:
(a) Inkubieren der biologischen Probe mit zwei unterschiedlichen monoklonalen NFP-Antikörpem, die an einen Festphasenträger gebunden sind; und
(b) Ermitteln von NFP nach der Definition in Anspruch 1, das an die monoklonalen Antikörper gebunden ist, mit einer Lösung eines dritten unterschiedlichen nachweisbar markierten monoklonalen NFP-Antikörpers, wobei die monoklonalen NFP-Antikörper für das NFP-Molekül nach Anspruch 1 spezifisch sind.

32. Diagnoseverfahren nach einem der Ansprüche 28 bis 31, wobei der Inkubationsschritt weiters das Hinzufügen einer bekannten Menge von markiertem NFP nach Anspruch 1 umfasst, wodurch ein kompetitiver Immunotest erzielt wird.

33. Diagnoseverfahren nach Anspruch 32, wobei die Markierung zur Aussendung von Strahlung fähig ist.

34. Diagnoseverfahren nach Anspruch 33, wobei die Markierung ¹²⁵I ist

35. Diagnoseverfahren nach einem der Ansprüche 28 bis 34, wobei der Nachweis durch Immuno-Polymerase-Kettenreaktion erfolgt.

## Revendications

1. Protéine des neurofilaments (NTP) essentiellement exempte d'une quelconque impureté naturelle et codée par la molécule ADN AD10-7 présente dans les cellules de *E. coli* déposées à l'American Type Culture Collection, Manassass, Virginie, sous le numéro d'enregistrement 69262.

2. Molécule d'acide nucléique isolée codant pour une protéine des neurofilaments (NTP) comprenant la molécule ADN AD10-7 présente dans les cellules de *E. coli* déposées à l'American Type Culture Collection, Manassass, Virginie, sous le numéro d'enregistrement 69262.

3. Molécule d'acide nucléique selon la revendication 2 comprenant une séquence génétique codant pour une NTP, **caractérisée en ce que** ladite NTP est la NTP humaine.

4. Plasmide comprenant la molécule d'acide nucléique selon la revendication 2 ou la revendication 3.

5. Hôte non humain transformé par le plasmide selon la revendication 4.

6. Procédé in vitro d'utilisation du plasmide selon la revendication 4 pour préparer une NTP, ledit procédé comprenant :
(a) l'introduction dudit plasmide contenant ladite séquence génétique codant pour ladite NTP dans une cellule hôte en vue de produire une cellule hôte recombinée;
(b) la mise en culture de ladite cellule hôte recombinée; et
(c) l'isolement de ladite NTP.

7. Procédé selon la revendication 6, **caractérisé en ce que** ladite séquence génétique codant pour ladite NTP est obtenue à partir de tissu cérébral d'un patient atteint de la maladie d'Alzheimer ou à partir d'une lignée cellulaire de tumeur nerveuse humaine.

8. Procédé selon la revendication 6 ou la revendication 7, **caractérisé en ce que** ledit hôte est *E*. *coli*.

9. Procédé selon l'une quelconque des revendications 6 à 8, **caractérisé en ce que** ledit gène est contenu dans un vecteur.

10. Procédé selon l'une quelconque des revendications 6 à 9, **caractérisé en ce que** ledit gène est sous le contrôle d'un promoteur inductible.

11. Procédé selon la revendication 10, **caractérisé en ce que** ledit promoteur est un promoteur lambda P₁ ou un promoteur *tac*.

12. Vecteur d'expression comprenant la molécule d'acide nucléique selon la revendication 2.

13. Virion comprenant le vecteur d'expression selon la revendication 12.

14. Oligonucléotide antisens 15mère à 30mère choisi parmi le groupe constitué des SEQ ID NO:1, SEQ ID NO:2, SEQ ID NO:3, SEQ ID NO:4, SEQ ID NO:5 et SEQ ID NO:6, qui est complémentaire à une séquence d'acide nucléique codant pour la NTP selon la revendication 1 et qui n'est pas homologue à la séquence d'acide nucléique PTP.

15. Oligonucléotide antisens selon la revendication 14 qui est l'ADN.

16. Oligonucléotide antisens selon la revendication 14 ou la revendication 15 qui est un oligonucléotide O.

17. Oligonucléotide antisens selon la revendication 14 ou la revendication 15 qui est un oligonucléotide S.

18. Ribozyme comprenant une séquence cible qui est complémentaire à une séquence d'acide nucléique codant pour la NTP selon la revendication 1 et qui n'est pas homologue à la séquence d'acide nucléique PTP.

19. Molécule d'ADN qui code pour le ribozyme selon la revendication 18.

20. Oligonucléotide comprenant la séquence 3'X5'-L-5'X3', **caractérisé en ce que** X comprend une séquence d'acide nucléique choisie parmi le groupe constitué des SEQ ID NOs:1-6 et **en ce que** L représente une liaison oligonucléotide.

21. Oligonucléotide comprenant la séquence 5'X3'-L-3'X5', **caractérisé en ce que** X comprend une séquence d'acide nucléique choisie parmi le groupe constitué des SEQ ID NOs:1-6 et **en ce que** L représente une liaison oligonucléotide.

22. Séquence guide externe de NTP ribonucléotide comprenant :
(a) une séquence de 10-15 nucléotides qui est complémentaire à une séquence d'acide nucléique codant pour la NTP selon la revendication 1 et qui n'est pas homologue à la séquence d'acide nucléique PTP; et
(b) une séquence de nucléotides 3'-NCCA, N étant la purine.

23. Séquence selon la revendication 22 qui est un ADN.

24. Procédé in vitro pour détecter la présence d'une protéine des neurofilaments (NTP) codée par l'acide nucléique selon la revendication 2, ledit procédé comprenant :
(a) la mise en contact d'un échantillon biologique d'un sujet humain soupçonné de contenir une NTP codée par l'acide nucléique selon la revendication 2 avec au moins un molécule capable de se lier à ladite NTP;
(b) la détection d'une quelconque molécule liée à ladite NTP.

25. Procédé selon la revendication 24, **caractérisé en ce que** ladite molécule est:
(a) un anticorps essentiellement exempt d'impuretés naturelles;
(b) un anticorps monoclonal; ou
(c) un fragment de (a) ou de (b).

26. Procédé selon la revendication 24 ou la revendication 25, **caractérisé en ce que** la détection de l'une quelconque desdites molécules liées à ladite NTP est réalisée par imagerie in situ.

27. Procédé selon l'une quelconque des revendications 24 à 26, **caractérisé en ce que** la détection de l'une quelconque desdites molécules liées à ladite NTP est réalisée par imagerie in vitro.

28. Procédé in vitro pour diagnostiquer la présence d'un état pathologique lié à la protéine des neurofilaments (NTP) chez un sujet humain soupçonné de présenter un tel état pathologique, procédé qui comprend:
(a) l'incubation d'un échantillon biologique soupçonné de contenir une NTP codée par ladite molécule d'acide nucléique selon la revendication 2, en présence d'au moins une molécule de liaison capable d'identifier ladite NTP; et
(b) la détection de ladite molécule de liaison qui est liée audit échantillon, ladite détection indiquant que ledit sujet présente un état pathologique lié à la NTP;
**caractérisé en ce que** ledit état pathologique lié à la NTP est la maladie d'Alzheimer, la présence de tumeurs neuroectodermiques, la présence d'un astrocytome malin ou la présence de glioblastomes.

29. Procédé de diagnostic selon la revendication 28, **caractérisé en ce que** ladite détection est réalisée par test immunométrique.

30. Procédé de diagnostic selon la revendication 29, **caractérisé en ce que** ledit test immunométrique est un test immunométrique à base d'anticorps monoclonal.

31. Procédé de diagnostic selon la revendication 28, **caractérisé en ce que** ledit procédé comprend :
(a) l'incubation dudit échantillon biologique avec deux anticorps monoclonaux différents de NTP liés à un support en phase solide; et
(b) la détection de la NTP selon la revendication 1 liée auxdits anticorps monoclonaux avec une troisième solution d'anticorps monoclonaux de NTP marquée de manière détectable, lesdits anticorps monoclonaux de NTP étant spécifiques à la molécule NTP de la revendication 1.

32. Procédé de diagnostic selon l'une quelconque des revendications 28 à 31, **caractérisé en ce que** ladite étape d'incubation comprend en outre l'addition d'une quantité connue de NTP marquée selon la revendication 1, un essai immunologique compétitif étant ainsi établi.

33. Procédé de diagnostic selon la revendication 32, **caractérisé en ce que** ledit marqueur est capable d'émettre des radiations.

34. Procédé de diagnostic selon la revendication 33, **caractérisé en ce que** ledit marqueur est ¹²⁵I.

35. Procédé de diagnostic selon l'une quelconque des revendications 28 à 34, **caractérisé en ce que** ladite détection est réalisée par amplification en chaîne par immunopolymérase.
